# EUROPEAN PATENT APPLICATION

(11) **EP 2 821 504 A1**
(43) Date of publication of application: **07.01.2015**
(21) Application number: 13755605.6
(22) Date of filing: 28.02.2013
(51) Int. Cl.: C12Q 1/68, A61K 31/7088, A61P 21/04, A61P 25/16, A61P 25/28, C40B 30/06, G01N 33/15, G01N 33/50, G01N 33/566, G01N 33/68, C12N 15/09

(54) **METHOD FOR DESIGNATING DISEASE RELATING TO AMOUNT OF TDP-43 EXISTING IN CELLS**

(30) Priority: 28.02.2012 JP 2012042256
(71) Applicant: National University Corporation Tokyo University of Agriculture and Technology, Fuchu-shi, Tokyo 183-8538 (JP); Tokyo Metropolitan University, Shinjuku-ku Tokyo 163-0926 (JP)
(72) Inventor: TAKAHASHI, Nobuhiro, Fuchu-shi Tokyo 183-8538 (JP); IZUMIKAWA, Keiichi, Fuchu-shi Tokyo 183-8538 (JP); ISHIKAWA, Hideaki, Fuchu-shi Tokyo 183-8538 (JP); YOSHIKAWA, Harunori, Fuchu-shi Tokyo 183-8538 (JP); ISOBE, Toshiaki, Hachioji-shi, Tokyo 192-0916 (JP); TAOKA, Masato, Tokyo 152-0034 (JP); NAKAYAMA, Hiroshi, Wako-shi Saitama 351-0198 (JP)
(74) Representative: Cabinet Plasseraud
(86) International application number: PCT/JP2013/055497
(87) International publication number: WO 2013/129603

(57) **Abstract**

The present invention aims to develop and provide a method for identifying a disease associated with the abundance of TDP-43 in cells and a method for producing a TDP-43 binding inhibitor. By measuring the abundance of a measurement substance, the amount of binding between a measurement substance and TDP-43, etc. in cells obtained from a subject, it is identified whether or not the subject is suffering from a disease associated with the abundance of TDP-43 in cells. Also, a drug which can significantly reduce the binding between a measurement substance and TDP-43 is produced by adding a drug candidate substance.

## Description

### Technical Field

The present invention relates to a method for identifying a disease associated with the abundance of TDP-43 in cells, and a method for producing a drug which reduces the abundance of specific mt-tRNA in cells or a method for producing a binding inhibitor which inhibits the binding between TDP-43 and a nucleic acid or a polypeptide.

### Background Art

Amyotrophic Lateral Sclerosis (hereinbelow, often abbreviated as "ALS") is an intractable disease, which exhibits symptoms of upper limb dysfunction, gait disturbance, articulation disorder, difficulty in swallowing, respiratory disturbance, and so on caused by muscle atrophy and muscle weakness associated with progression of upper motor neuron disorder and lower motor neuron disorder due to pathological changes in the brain stem, spinal cord, and motor neurons. Although some cases of respirator-assisted relatively prolonged survival are known, disease progression is relatively rapid under normal circumstances, and in many cases, those who suffer from ALS die 3.5 years after the onset of the disease on average, primarily due to respiratory muscle paralysis.

Five to 10% of ALS is referred to as Familial Amyotrophic Lateral Sclerosis (familial ALS) for its association with family history. In approximately 20% of familial ALS, ALS1, which is a mutation in the gene for the free-radical scavenging enzyme Cu/Zn superoxide dismutase (superoxide dismutase 1: SOD1), is reported (Non Patent Literature 1), from which it is considered that the SOD1 gene is one of the causative genes of ALS. Also, as other causative genes of ALS, the genes for, for example, angiogenin (Non Patent Literature 2), vesicle-associated membrane protein (VAMP) / synaptobrevin-associated membrane protein B (VAPB) (Non Patent Literature 3), TAR DNA-binding protein 43 (TDP-43) (Non Patent Literature 4), and fused in sarcoma/translated in liposarcoma (FUS/TLS) (Non Patent Literature 5) are reported. However, specific mechanisms as to how ALS develops and how the disease progresses in association with abnormalities of these genes remain unverified.

Maruyama et al. (Non Patent Literature 6) disclose that three out of six patients with familial ALS have a mutation in the gene for optineurin, which inhibits NF-κB involved in intracellular signal transduction. Mutant optineurin has lost its NF-κB inhibitory activity, suggesting that NF-κB inhibitors may possibly have therapeutic effects on ALS. However, 90 to 95% of ALS is sporadic, and in many patients with ALS, mutations have been found not in the optineurin gene, but rather, in the TDP-43 gene. In view of this, even if NF-κB inhibitors are effective in the treatment of ALS, their therapeutic effects can be expected only in a very small subset of patients with ALS.

Rothstein et al. (Non Patent Literature 7) have confirmed approximately three times higher concentrations of glutamic acid (hereinbelow, often abbreviated as "Glu") in the cerebrospinal fluid of patients with ALS who have mutant SOD1 gene compared to healthy individuals having the wild-type SOD1 gene, and also, increased Glu in the cerebrospinal fluid of approximately 40% of patients with sporadic ALS also by collective investigations, suggesting that Glu toxicity associated with increased Glu plays an important role in the mechanism of development of ALS. An increase in Glu as observed in patients with ALS is assumed to be attributable to the loss of uptake of Glu by astrocytes, and from this assumption, a promising hypothesis has been advanced that sporadic ALS develops as a result of neurological disorder caused by Glu toxicity, particularly Glu toxicity due to increased Glu as mediated by the α-amino-3-hydroxy-5-methyl-4-isoxazolepropionic acid (AMPA) receptor, which is a subtype of the glutamic acid receptor. There is a report that the rate of RNA editing is low in the GluR2 Q/R site, which is an AMPA receptor subunit, in human ALS motor neurons, and this plays an important role in the pathological conditions of sporadic ALS (Non Patent Literatures 8 to 12), supporting the aforementioned hypothesis.

Lacomblez et al. (Non Patent Literature 13) disclose the outcome of a clinical trial that Riluzole is capable of significantly extending, but not to a great extent, the survival period of patients with ALS. Riluzole is a glutamic acid antagonist, which was developed based on the aforementioned hypothesis. As of now, only the aforementioned Riluzole is approved as an ALS treatment drug in Europe, the U.S., and Japan (trade name: Rilutek). However, Riluzole does not inhibit an increase in Glu, which is the cause of ALS, but serves no more than as a symptomatic treatment for alleviating the toxic effect of Glu brought about by increased Glu. Thus, Riluzole is effective neither for muscle weakness, which is the principal symptom of ALS, nor for other clinical manifestations.

Patent Literature 1 discloses that administration of a high dose of methylcobalamin produces a certain level of improvement effect on the clinical manifestations of ALS, and presently, a double-blind comparative study of methylcobalamin is undertaken. However, this drug is also based on the inhibitory effect on glutamic acid-induced cytotoxicity (Non Patent Literature 14), and thus, cannot be expected to improve the effect of Riluzole to a great extent. In the meantime, as to the effect of methylcobalamin on ALS, besides its inhibition of Glu-induced toxic effects, it is found that a high concentration of methylcobalamin competitively inhibits S-adenosylmethionine-mediated DNA methylation, thereby maintaining a high level of gene transcription (Non Patent Literature 15), and it is speculated that this might accelerate the synthesis of various kinds of proteins necessary for nerve regeneration, resulting in the protection of motor nerve cells in the spinal cord and cerebrum. However, there is no evidence actually indicating an association with the pathological conditions or mechanisms of development of ALS, rendering the above idea a mere general theoretical speculation.

Also, up until now, there has been no specific clinical test method which can make a positive diagnosis of ALS. Conventionally, ALS has been determined comprehensively based on the clinical history and neurologic findings, and prediction before the onset, diagnosis in the early phase of onset, and accurate diagnosis after the onset have been difficult.

As shown above, there has been no established high-accuracy diagnostic method or radical treatment, or progression-inhibiting or progression-delaying treatment for ALS, despite a demand for the development of them.

### Citation List

### Patent Literature

Patent Literature 1: JP Patent Application No. 9-41604 (1997)

### Non Patent Literature

Non Patent Literature 1: Rosen D. R., et al. 1993, Nature, 362 (6415): 59 to 62.
Non Patent Literature 2: Greenway M. J., et al. 2004, Neurology, 63 (10): 1936 to 1938.
Non Patent Literature 3: Ratnaparkhi A., et al. 2008, PLoS One, 3 (6): e2334.
Non Patent Literature 4: Arai T., et al. 2006, Biochem. Biophys. Res. Commun., 351 (3): 602 to 611.
Non Patent Literature 5: Kwiatkowski T. J. Jr, 2009, Science, 323 (5918): 1205 to 1208
Non Patent Literature 6: Maruyama H., et al., 2010, Nature, 465: 223 to 226
Non Patent Literature 7: Rothstein J. D., et al., 1990, Ann, Neurol., 28: 18 to 25.
Non Patent Literature 8: Trotti D., et al., 1999, Nat. Neurosci., 1999, 2: 427 to 433.
Non Patent Literature 9: Rothstein J. D., 1996, Neuron, 16: 675 to 686.
Non Patent Literature 10: Bruijn L. I., et al. 1997, Neuron, 18: 327 to 338.
Non Patent Literature 11: Howland D. S., et al. 2002, Proc. Natl. Acad. Sci. USA; 99: 1604 to 1609.
Non Patent Literature 12: Rothstein, J. D., 2009, Ann. Neurol., 65 (suppl): S3 to S9.
Non Patent Literature 13: Lacomblez L., 1996, Lancet, 347: 1425 to 1431.
Non Patent Literature 14: Akaike A., et al., 1993, Eur. J. Pharmacol., 241: 1 to 6.
Non Patent Literature 15: Pfohl-Leszkowicz A, et al., 1991, Biochemistry, 30: 8045 to 8051.

### Summary of Invention

### Technical Problem

An object of the present invention is to develop and provide a method for identifying a disease associated with the abundance of TAR DNA-binding protein-43 (TAR DNA-binding Protein 43kDa: in the present specification, hereinbelow, often abbreviated as "TDP-43") in cells as represented by ALS and frontotemporal lobar degeneration (hereinbelow, often referred to as "FTLD", which includes Pick's disease involving Pick bodies) and a method for producing a TDP-43 binding inhibitor.

### Solution to Problem

In order to achieve the aforementioned object, the present inventors studied the TDP-43 gene, which is frequently mutated in patients with ALS. Mutations in the TDP-43 gene are found in familial ALS as well as in sporadic ALS, suggesting that the TDP-43 gene is the major causative gene of ALS (Lagier-Tourenne, C., and Cleveland, D. W., 2009, Cell, 136; 1001 to 1004; Arai, et al., 2006, Biochem. Biophys. Res. Commun., 351: 602 to 611; Neumann, et al., 2006, Science, 314, 130 to 133; Chen-Plotkin, et al., 2010, Nat. Rev. Neurol., 6: 211 to 220; Lagier-Tourenne, et al., 2010, Hum. Mol. Genet. 19: R46 to R64; Kabashi, et al., 2008, Nat. Genet. 40: 572 to 574; Sreedharan, et al., 2008, Science, 319: 1668 to 1672; and Pesiridis, G.S., et al., 2009, Hum. Mol. Genet., 18: R156 to R162). Also, it is known that, in the patients with ALS, accumulation of depositions containing TDP-43 is observed in the cytoplasm of cells in the lesions (Arai T, et al., Biochem Biophys Res Commun., 2006, 351: 602 to 611). However, the function of TDP-43 in cells, the association between mutations in the TDP-43 gene and the development of ALS, and the mechanism of the development of ALS in association with mutations in the TDR-43 gene have been totally unknown so far. Although a genome-wide study to search for TDP-43 function in a large number of patients with sporadic ALS having mutant TDP-43 is also ongoing, results leading to the development of a new treatment method and screening method therefor have not been obtained to date (Sephton C.F., et al., 2011, J. Biol. Chem. 286: 1204 to 1215.; Polymenidou M, et al., 2011, Nat. Neurosci., 14 (4): 459 to 68.; and Tollervey J.R., et al., 2011, Nat. Neurosci., 14 (4): 452 to 458).

The present inventors conducted intensive studies with the focus on the presence of the RNA Recognition Motif (RRM) domain, which is the RNA-binding sequence, in the amino acid sequence of TDP-43. As a result, it was revealed that TDP-43 was involved in the biosynthesis or metabolism of four types of mitochondrial tRNAs (hereinbelow, referred to as "mt-tRNA"), which are mt-tRNA^{Asn}, mt-tRNA^{Gln}, mt-tRNA^{Glu}, and mt-tRNA^{Pro}, and in the amino acid metabolism of glutamic acid / glutamine and aspartic acid / asparagine, and was a scaffold protein serving as a scaffold in the biosynthesis or metabolism of the above mt-tRNAs and amino acids, and not only that, engaged in controlling the abundance of the above mt-tRNAs and amino acids in cells. Further, it was revealed that TDP-43 was also involved in the regulation of energy metabolism, and further, there was a correlation between the abundance of TDP-43 in cells and the amount of ATP or active oxygen in cells. Moreover, the present inventors found that apoptosis induced by an increased expression level of TDP-43 in cells was correlated with an increased amount of binding between TDP-43 and some of mt-tRNAs, and that apoptosis was suppressed by reducing the aforementioned binding to the normal level. The present invention was completed based on the above findings and specifically provides the followings.
(1) A method for identifying a disease associated with an abundance of TDP-43 in a cell, comprising:
   a measurement step of measuring at least one of
      (a) an abundance of at least one mt-tRNA selected from the group consisting of mt-tRNA^{Asn}, mt-tRNA^{Gln}, mt-tRNA^{Glu}, and mt-tRNA^{Pro},
      (b) an abundance of glutamic acid and/or aspartic acid,
      (c) an abundance of ATP or active oxygen,
      (d) an amount of binding between at least one polypeptide selected from the group consisting of Aralar 1, Aralar 2, glutamate dehydrogenase, and Musashi 2 and TDP-43, and
      (e) an amount of binding between at least one mt-tRNA selected from the group consisting of mt-tRNA^{Asn}, mt-tRNA^{Gln}, mt-tRNA^{Glu}, and mt-tRNA^{Pro} and TDP-43
         in a cell derived from a subject; and
         an identification step comprising comparing a measurement value obtained by the measurement step with a corresponding measurement value in a cell derived from a healthy individual, and when there is a statistically significant difference between these values, identifying the subject as suffering from the disease.
(2) The identification method according to (1), wherein the disease is a disease in which an abundance of TDP-43 in a cell derived from a subject is statistically significantly increased compared to an abundance of TDP-43 in a cell derived from a healthy individual.
(3) The identification method according to (1) or (2), wherein, when an initiation methionine in an amino acid sequence set forth in SEQ ID NO: 1 is designated as position 1, the TDP-43 has a mutation in which alanine at position 90 is substituted by valine (hereinbelow, often abbreviated as "A90V"), aspartic acid at position 169 is substituted by glycine (hereinbelow, often abbreviated as "D169G"), asparagine at position 267 is substituted by serine (hereinbelow, often abbreviated as "N267S"), glycine at position 287 is substituted by serine (hereinbelow, often abbreviated as "G287S"), glycine at position 290 is substituted by alanine (hereinbelow, often abbreviated as "G290A"), serine at position 292 is substituted by asparagine (hereinbelow, often abbreviated as "S292N"), glycine at position 294 is substituted by alanine (hereinbelow, often abbreviated as "G294A"), glycine at position 294 is substituted by valine (hereinbelow, often abbreviated as "G294V"), glycine at position 295 is substituted by arginine (hereinbelow, often abbreviated as "G295R"), glycine at position 295 is substituted by serine (hereinbelow, often abbreviated as "G295S"), glycine at position 298 is substituted by serine (hereinbelow, often abbreviated as "G298S"), methionine at position 311 is substituted by valine (hereinbelow, often abbreviated as "M311V"), alanine at position 315 is substituted by threonine (hereinbelow, often abbreviated as "A315T"), alanine at position 315 is substituted by glutamic acid (hereinbelow, often abbreviated as "A315E"), glutamine at position 331 is substituted by lysine (hereinbelow, often abbreviated as "Q331K"), serine at position 332 is substituted by asparagine (hereinbelow, often abbreviated as "S332N"), glycine at position 335 is substituted by aspartic acid (hereinbelow, often abbreviated as "G335D"), methionine at position 337 is substituted by valine (hereinbelow, often abbreviated as "M337V"), glutamine at position 343 is substituted by arginine (hereinbelow, often abbreviated as "Q343R"), asparagine at position 345 is substituted by lysine (hereinbelow, often abbreviated as "N345K"), glycine at position 348 is substituted by cysteine (hereinbelow, often abbreviated as "G348C"), asparagine at position 352 is substituted by serine (hereinbelow, often abbreviated as "N352S"), asparagine at position 352 is substituted by threonine (hereinbelow, often abbreviated as "N352T"), glycine at position 357 is substituted by serine (hereinbelow, often abbreviated as "G357S"), arginine at position 361 is substituted by serine (hereinbelow, often abbreviated as "R361S"), proline at position 363 is substituted by alanine (hereinbelow, often abbreviated as "P363A"), asparagine at position 378 is substituted by aspartic acid (hereinbelow, often abbreviated as "N378D"), serine at position 379 is substituted by cysteine (hereinbelow, often abbreviated as "S379C"), serine at position 379 is substituted by proline (hereinbelow, often abbreviated as "S379P"), alanine at position 382 is substituted by proline (hereinbelow, often abbreviated as "A382P"), alanine at position 382 is substituted by threonine (hereinbelow, often abbreviated as "A382T"), isoleucine at position 383 is substituted by valine (hereinbelow, often abbreviated as "I383V"), glycine at position 384 is substituted by arginine (hereinbelow, often abbreviated as "G384R"), asparagine at position 390 is substituted by aspartic acid (hereinbelow, often abbreviated as "N390D"), asparagine at position 390 is substituted by serine (hereinbelow, often abbreviated as "N390S"), or serine at position 393 is substituted by leucine (hereinbelow, often abbreviated as "S393L") or a mutation in which tyrosine at position 374 and subsequent amino acids are deleted.
(4) The identification method according to (3), wherein the mutation is D169G, G298S, or R361S.
(5) The identification method according to any of (1) to (4), wherein the disease is a nervous system disease.
(6) The identification method according to (5), wherein the nervous system disease is selected from the group consisting of amyotrophic lateral sclerosis, Alzheimer's disease, Parkinson's disease, and frontotemporal lobar degeneration.
(7) A method for producing a drug which reduces an abundance of aspartic acid, glutamic acid, ATP, or active oxygen in a cell, comprising: an introduction step of introducing a drug candidate substance into a cell, a measurement step of measuring the abundance in a cell comprising the drug candidate substance and in a cell not comprising the drug candidate substance, and a selection step, comprising comparing measurement values in two cells obtained by the measurement step and, when a measurement value in the cell comprising the drug candidate substance is statistically significantly lower, selecting the drug candidate substance as a drug of interest.
(8) A method for producing a TDP-43 binding inhibitor, comprising:
   a mixing step of mixing
      (a) at least one mt-tRNA selected from the group consisting of mt-tRNA^{Asn}, mt-tRNA^{Gln}, mt-tRNA^{Glu}, and mt-tRNA^{Pro}, and/or
      (b) at least one protein selected from the group consisting of Aralar1, Aralar2, Glutamate DeHydrogenase (hereinbelow, referred to as "GDH"), and Musashi 2, and

         TDP-43 with a drug candidate substance, a detection step of detecting an amount of binding between the mt-tRNA and/or the protein and the TDP-43, and a selection step of selecting, when the binding is not detected or a comparison between the amount of binding detected and an amount of binding between a corresponding mt-tRNA and/or protein and TDP-43 in an absence of the drug candidate substance shows a statistically significant difference, the drug candidate substance as a TDP-43 binding inhibitor.
(9) The production method according to (8), wherein the drug candidate substance is a substance competing for binding to TDP-43 with the mt-tRNA specified by (a) or the protein specified by (b).
(10) The production method according to (8), wherein the drug candidate substance is a substance which reduces an abundance of the mt-tRNA specified by (a) or the protein specified by (b) in an active form in a cell.
(11) The production method according to (9) or (10), wherein the drug candidate substance is a nucleic acid molecule, a peptide, or a low molecular weight compound.
(12) The production method according to (11), wherein the drug candidate substance is:
   (a) a polypeptide of 100 amino acids or less, comprising a polypeptide consisting of an amino acid sequence set forth in SEQ ID NO: 2, 4, or 5,
   (b) a polypeptide of 130 amino acids or less, comprising a polypeptide resulting from addition, deletion, or substitution of 1 to 3 amino acids in an amino acid sequence set forth in SEQ ID NO: 2, 4, or 5,
   (c) an amino acid sequence having a 90% or more identity with an amino acid sequence set forth in SEQ ID NO: 2, 4, or 5, or
   (d) a moiety of a polypeptide consisting of an amino acid sequence set forth in SEQ ID NO: 2, 4, or 5.
(13) The production method according to (11), wherein the drug candidate substance is a peptide of 9 to 20 amino acids comprising:
   (a) a moiety of a peptide consisting of an amino acid sequence set forth in SEQ ID NO: 2,
   (b) a moiety of a peptide comprising addition, deletion, or substitution of 1 to 3 amino acids in an amino acid sequence set forth in SEQ ID NO: 2, or
   (c) a moiety of a peptide consisting of an amino acid sequence having a 90% or more identity with an amino acid sequence set forth in SEQ ID NO: 2,
      wherein the peptide of 9 to 20 amino acids binds to a nucleic acid molecule of 5 to 50 nucleotides comprising a nucleotide sequence consisting of TGG or UGG and/or GTT or GUU.
(14) The production method according to any of (7) to (11), wherein, when an initiation methionine in an amino acid sequence set forth in SEQ ID NO: 1 is designated as position 1, the TDP-43 comprises a mutation of A90V, D169G, N267S, G287S, G290A, S292N, G294A, G294V, G295R, G295S, G298S, M311V, A315T, A315E, Q331K, S332N, G335D, M337V, Q343R, N345K, G348C, N352S, N352T, G357S, R361S, P363A, N378D, S379C, S379P, A382P, A382T, I383V, G384R, N390D, N390S, or S393L, or a mutation in which tyrosine at position 374 and subsequent amino acids are deleted.
(15) The production method according to (14), wherein the mutation is D169G, G298S, or R361S.
(16) The production method according to any of (7) to (15), wherein the drug which reduces an abundance of amino acid, ATP, or active oxygen in a cell according to (7) or the TDP-43 binding inhibitor according to any of (8) to (15) is an active ingredient of a therapeutic agent for a disease associated with an abundance of TDP-43 in a cell.
(17) The production method according to (16), wherein the disease associated with an abundance of TDP-43 in a cell is a disease in which an abundance of TDP-43 in a cell derived from a subject is statistically significantly increased compared to an abundance of TDP-43 in a cell derived from a healthy individual.
(18) The production method according to (16) or (17), wherein the disease is a nervous system disease.
(19) The production method according to (18), wherein the disease is selected from the group consisting of amyotrophic lateral sclerosis, Alzheimer's disease, Parkinson's disease, and frontotemporal lobar degeneration.
(20) A TDP-43 binding inhibitor comprising a nucleic acid molecule of 5 to 50 nucleotides, wherein the nucleic acid molecule comprises two or more of a nucleotide sequence consisting of TG or UG and/or GT or GU, and inhibits binding between mt-tRNA^{Asn}, mt-tRNA^{Gln}, mt-tRNA^{Glu}, or mt-tRNA^{Pro} and TDP-43.
(21) The TDP-43 binding inhibitor according to (20), comprising a nucleic acid molecule of 6 to 50 nucleotides, wherein the nucleic acid molecule comprises two or more of a nucleotide sequence consisting of TGG or UGG and/or GTT or GUU.
(22) The TDP-43 binding inhibitor according to (20) or (21), comprising a sequence comprising a successive repetition of the nucleotide sequence.
(23) The TDP-43 binding inhibitor according to (22), wherein the number of the repetition is 3 to 15.
(24) The TDP-43 binding inhibitor according to (23), comprising a nucleotide sequence set forth in SEQ ID NO: 43, 45, or 46.
The present specification encompasses the contents described in the specification and/or drawings of JP Patent Application No. 2012-042256, based on which the present application claims priority.

### Advantageous Effects of Invention

According to the identification method of the present invention, it is made possible to identify, with high accuracy, whether or not a subject is suffering from a disease associated with the abundance of TDP-43 in cells as represented by ALS and FTLD.

According to the production method of the present invention, it is made possible to efficiently select a substance which possibly serves as an active ingredient in the treatment of a disease associated with the abundance of TDP-43 in cells as represented by ALS and FTLD.

The TDP-43 binding inhibitor of the present invention can be provided as an active ingredient in the treatment of a disease associated with the abundance of TDP-43 in cells.

### Brief Description of Drawings

[Figure 1] Figure 1 shows a result of urea denaturation PAGE showing the RNA that was co-immunoprecipitated by pull-down of DAP-TDP-43. Bands 1 to 3 in the frame show the RNA that binds specifically to DAP-TDP-43. The right panel shows the two right-side lanes of the left panel that were overexposed.
[Figure 2] Figure 2 shows a result of nano LC-MS/MS.
[Figure 3] Figure 3A shows a result of SYBR Gold staining following urea denaturation PAGE of the RNA co-immunoprecipitated by pull-down of DAP-TDP-43. Figure 3B shows a result of Northern blot of mt-tRNA^{Asn}, mt-tRNA^{Gln}, mt-tRNA^{Pro} and mt-tRNA^{Glu} co-immunoprecipitated with DAP-TDP-43.
[Figure 4] Figure 4A shows a result of detection by Western blot of the TDP-43 protein in HeLa cells transfected with TDP-43-siRNA or Cont (nonspecific siRNA). Figure 4B shows a cell growth rate in HeLa cells transfected with TDP-43-siRNA or Cont.
[Figure 5] Figure 5 shows a change in the abundance of various types of RNAs in the HeLa cells upon treatment with TDP-43-siRNA. 5.8S represents 5.8S rRNA, U1 represents U1 snRNA, Asn represents mt-tRNA^{Asn}, Gln represents mt-tRNA^{Gln} and Leu represents mt-tRNA^{Leu(UUR)}. The results are based on results from at least 3 independent experiments.
[Figure 6] Figure 6A shows a result of Western blot analysis of DAP-TDP-43 and endogenous TDP-43, when DAP-TDP-43 was overexpressed. Figure 6B shows a result of Northern blot analysis of mt-tRNA^{Asn} and mt-tRNA^{Gln}, and 5.8S rRNA, when DAP-TDP-43 was overexpressed.
[Figure 7] Figure 7A shows the degradation of mt-tRNA^{Asn} or mt-tRNA^{Gln} over time. Figure 7B shows a result of the co-immunoprecipitation with DAP-TDP-43 over time.

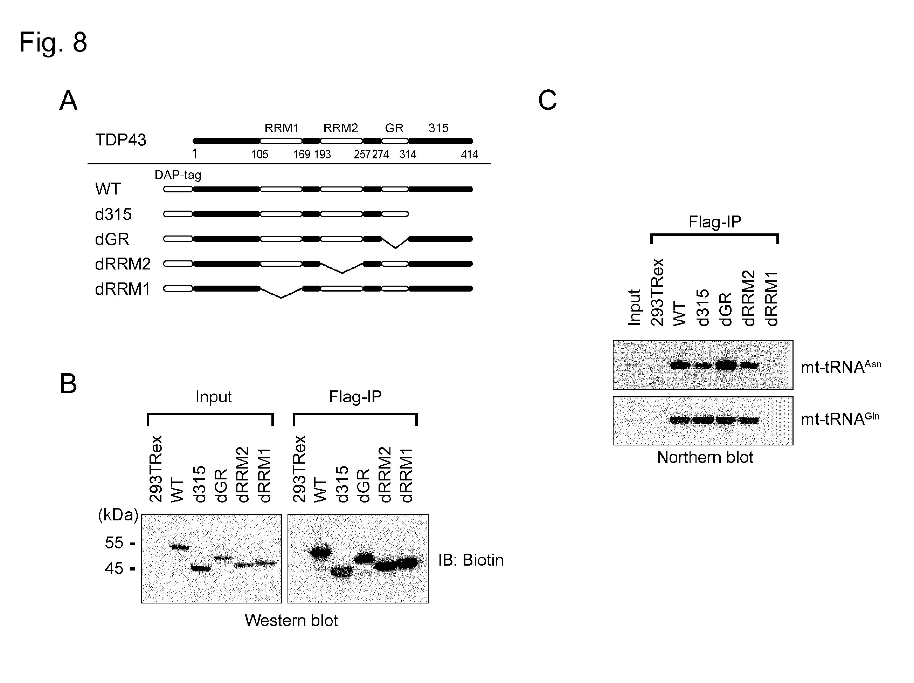

[Figure 8] Figure 8A is a conceptual diagram showing the structure of the wild type TDP-43 and each domain-deficient type of TDP-43. The black bar and the white bar represent domains in TDP-43. The numerical values under the bar show positions in the amino acid sequence of TDP-43 when the initiation methionine is designated as position 1. Figure 8B shows a result of Western blot showing intracellular proteins expressed by doxycycline induction in the wild type TDP-43 and each domain-deficient type of TDP-43 expressing strains. Figure 8C shows a result of Northern blot showing mt-tRNA^{Asn} and mt-tRNA^{Gln} bound to the wild type TDP-43 and each domain-deficient type of TDP-43.
[Figure 9] Figure 9 shows the cytotoxicity induced by domain-deficient type of TDP -43. The results are based on results from at least 3 independent experiments.

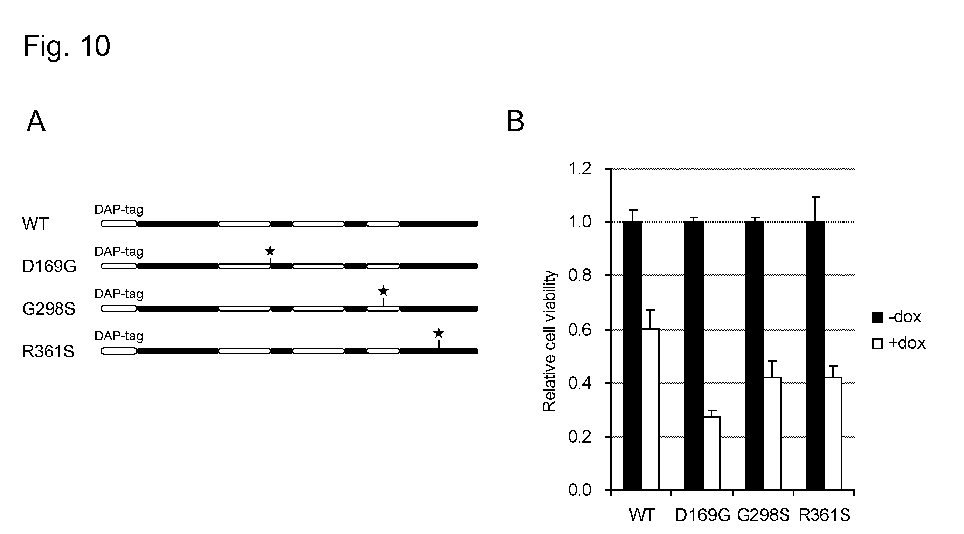

[Figure 10] Figure 10A is a conceptual diagram showing the wild type TDP-43 and each mutant TDP-43 used in the experiment. In the figure, the asterisk shows the location of mutation (point mutation) in each mutant TDP-43. D169G represents a mutation in TDP-43 in which D at the amino acid sequence position 169 is substituted by G, G298S represents a mutation in TDP-43 in which G at the amino acid sequence position 298 is substituted by S, and R361S represents a mutation in TDP-43 in which R at the amino acid sequence position 361 is substituted by S. Figure 10B shows the viability in the wild type and each mutant TDP-43 expressing strains.
[Figure 11] Figure 11 shows the abundance of each type of mt-tRNA in cells when the wild type and each mutant TDP-43 were expressed by doxycycline induction. Each value represents a value upon induction of expression with doxycycline (+dox) relative to a value of 1 in the absence of doxycycline (-dox).
[Figure 12] Figure 12 shows a result of Western blot showing a novel TDP-43 binding protein co-immunoprecipitated by pull-down of DAP-TDP-43.
[Figure 13] Figure 13A shows a result of interchange immunoprecipitation of Aralar 1 and Aralar 2. Figure 13B shows a result of mt-tRNA^{Asn} co-immunoprecipitated with Aralar 1 or Aralar 2.
[Figure 14] Figure 14 shows a result of Northern blot showing mt-tRNA co-immunoprecipitated by pull-down of Musashi 2.
[Figure 15] Figure 15 shows binding of Aralar 1 or Aralar 2 to the domain-deficient type of TDP-43.
[Figure 16] Figure 16A shows the ATP abundance per cell when TDP-43 was silenced with TDP-43-siRNA. Figure 16B shows the ATP abundance per cell when DAP-TDP-43 was overexpressed by doxycycline induction.
[Figure 17-1] Figure 17-1 shows the abundance of mt-tRNA^{Asn}, mt-tRNA^{Gln} and tRNA^{Leu(UUR)} in cells when each domain-deficient type of TDP-43 was expressed. Each value represents a value upon induction of expression with doxycycline (+dox) relative to a value of 1 in the absence of doxycycline (-dox).
[Figure 17-2] Figure 17-2 shows a result of measurement of the ATP when each domain-deficient type of TDP-43 was expressed.
[Figure 18] Figure 18 shows the abundance of ATP in cells after the induction of mutant TDP-43 in expression-induced type of cell lines having ALS-like mutant TDP-43 (D169G, G298S, R361S).
[Figure 19] Figure 19 is a sequence alignment for four types of mt-tRNAs bound to TDP-43. The asterisks in the figure indicate the bases conserved in all of the four types of mt-tRNAs. Also shown under the sequence are, the bases corresponding to the D-loop, anticodon loop, variable region, T-loop and the acceptor arm when each mt-tRNA formed a conformation.
[Figure 20] Figure 20 shows the secondary structure of the mt-tRNA^{Asn} bound to TDP-43. The base with an asterisk shown in the figure represents the base that is conserved in all of the four types of the mt-tRNAs bound to TDP-43 shown in Figure 19. The arrow in the figure shows the separative site of the mt-tRNA corresponding to the TDP-43 binding inhibitor candidate oligonucleotides (D-loop, anticodon, T-loop) used in a competitive assay of Example 13 (2). In the figure, U is substituted by T because the mt-tRNA is represented by DNA.
[Figure 21] Figure 21 shows inhibition of binding between TDP-43 and mt-tRNA by the TDP-43 binding inhibitor candidate oligonucleotide. Figure 21A shows a result of urea denaturation PAGE showing the nucleic acids that were co-immunoprecipitated with DAP-TDP-43 by Flag-IP. The mt-tRNA in the figure represents four types of mt-tRNAs bound to TDP-43. Figure 21B shows a result of Northern blot of the mt-tRNA^{Asn} co-immunoprecipitated with DAP-TDP-43 in competitive reaction.
[Figure 22] Figure 22 shows inhibition of binding between TDP-43 and mt-tRNA by various TDP-43 binding inhibitor candidate oligonucleotides having sequence repeats. Figure 22A shows a result of urea denaturation PAGE showing the oligonucleotides that were co-immunoprecipitated with DAP-TDP-43 by Flag-IP. The mt-tRNA in the figure represents four types of mt-tRNAs bound to TDP-43. Figure 22B shows a result of Northern blot of the mt-tRNA^{Asn} co-immunoprecipitated with DAP-TDP-43 in competitive reaction.
[Figure 23] Figure 23 shows inhibition of binding between TDP-43 and mt-tRNA when (TG)₁₂ was added at each concentration. Figure 23A shows a result of urea denaturation PAGE showing the mt-tRNA that was co-immunoprecipitated with DAP-TDP-43 by Flag-IP.
Figure 23B shows a result of Northern blot of the mt-tRNA^{Asn} co-immunoprecipitated with DAP-TDP-43 in competitive reaction. Figure 23C is a graph representing the results from B.
[Figure 24] Figure 24 shows the relationship over time between TDP-43 and the amount of active oxygen in cells.

### Description of Embodiments

### 1. Method for identifying a disease associated with the abundance of TDP-43 in cells

### 1-1. Summary

The first aspect of the present invention is a method for identifying a disease associated with the abundance of TDP-43 in cells. The method of the present aspect comprises identifying, by measuring the abundance of a test substance, the amount of the test substance bound to TDP-43 in cells collected from a subject, or the like, whether the subject suffers from a disease associated with the abundance of TDP-43 in the cell.

### 1-2. Target Disease

The target disease of the present invention is a "disease associated with the abundance of TDP-43 in cells." Here, "TAR DNA-binding protein 43kDa (TDP-43)" is a kind of heterogeneous nuclear ribonucleoprotein (hnRNP). TDP-43 is a protein which has two RNA binding domains in its molecule and can move between the nucleoplasm and cytoplasm in cells. In the present specification, TDP-43 encompasses the human wild type TDP-43 consisting of the amino acid sequence set forth in SEQ ID NO: 1, as well as the human mutant TDP-43 consisting of an amino acid sequence including deletion, substitution and/or addition of one or several amino acids in the amino acid sequence set forth in SEQ ID NO: 1, and the human mutant TDP-43 which consists of an amino acid sequence having 85% or more, preferably 90% or more, more preferably 95% or more identity with the amino acid sequence set forth in SEQ ID NO: 1 and has a function equivalent to the human wild type TDP-43, or TDP-43 orthologs from other biological species. In the present specification, the term "several" refers to integers of 2 to 10, e.g., integers of 2 to 7, 2 to 5, 2 to 4, 2 to 3. In the present specification, the "identity" refers, when a gap is introduced into one or both of two amino acid sequences as needed so as to maximize the number of matching amino acids and the sequences are aligned, to the ratio (%) of the number of matching amino acids of one amino acid sequence to the total number of amino acids of the other amino acid sequence (which is the amino acid sequence set forth in SEQ ID NO: 1, herein).

Specific examples of human mutant TDP-43 consisting of an amino acid sequence including substitution or deletion of one or several amino acids in the amino acid sequence set forth in SEQ ID NO: 1 include mutations wherein alanine at position 90 is substituted by valine (A90V), aspartic acid at position 169 is substituted by glycine (D169G), asparagine at position 267 is substituted by serine (N267S), glycine at position 287 is substituted by serine (G287S), glycine at position 290 is substituted by alanine (G290A), serine at position 292 is substituted by asparagine (S292N), glycine at position 294 is substituted by alanine (G294A), glycine at position 294 is substituted by valine (G294V), glycine at position 295 is substituted by arginine (G295R), glycine at position 295 is substituted by serine (G295S), glycine at position 298 is substituted by serine (G298S), methionine at position 311 is substituted by valine (M311V), alanine at position 315 is substituted by threonine (A315T), alanine at position 315 is substituted by glutamic acid (A315E), glutamine at position 331 is substituted by lysine (Q331K), serine at position 332 is substituted by asparagine (S332N), glycine at position 335 is substituted by aspartic acid (G335D), methionine at position 337 is substituted by valine (M337V), glutamine at position 343 is substituted by arginine (Q343R), asparagine at position 345 is substituted by lysine (N345K), glycine at position 348 is substituted by cysteine (G348C), asparagine at position 352 is substituted by serine (N352S), asparagine at position 352 is substituted by threonine (N352T), glycine at position 357 is substituted by serine (G357S), arginine at position 361 is substituted by serine (R361S), proline at position 363 is substituted by alanine (P363A), asparagine at position 378 is substituted by aspartic acid (N378D), serine at position 379 is substituted by cysteine (S379C), serine at position 379 is substituted by proline (S379P), alanine at position 382 is substituted by proline (A382P), alanine at position 382 is substituted by threonine (A382T), isoleucine at position 383 is substituted by valine (I383V), glycine at position 384 is substituted by arginine (G384R), asparagine at position 390 is substituted by aspartic acid (N390D), asparagine at position 390 is substituted by serine (N390S), or serine at position 393 is substituted by leucine (S393L), or human mutant TDP-43 having a mutation in which tyrosine at position 374 and subsequent amino acids are deleted, when the initiation methionine in the amino acid sequence set forth in SEQ ID NO: 1 is designated as position 1 (the same for the specification below). Human mutant TDP-43 having mutation D169G, G298S or R361S is preferred.

In the present specification, a "disease associated with the abundance of TDP-43 in cells" refers to a disease known to have the abundance of TDP-43 per cell that is statistically significantly increased or reduced compared to that in healthy individuals. The abundance of TDP-43 per cell is extremely strictly controlled in cells of healthy individuals (Ayala, Y.M., et al., 2011, EMBO J. 30 (2) 277-288). Therefore, an individual will experience some kind of physical abnormality if its abundance in cells varies beyond a normal range. The disease associated with the abundance of TDP-43 in cells may be any type of disease in which the abundance of TDP-43 in cells varies beyond a normal range. The disease may also include a disease in which the relation between change in the abundance of TDP-43 in cells and the disease has not been elucidated. Preferred is a disease known to have the abundance of TDP-43 per cell that is statistically significantly increased compared to that in healthy individuals.

The "healthy individual" in the present specification refers to an animal at least not suffering from a disease associated with the abundance of TDP-43 in cells, preferably a healthy animal not suffering from any disease. A healthy individual in principle is homologous to a subject described below, and preferably refers to an individual having similar biologic conditions to the subject, e.g., individuals with the same or similar conditions regarding subspecies (including races, breeds), sex, age (including age in month, week), body weight, predisposition (allergy etc.), and medical history.

In the present specification, "statistically significant" means that, when a quantitative difference in TDP-43 between in cells derived from the subject and in an equivalent number of cells derived from a healthy individual was statistically processed, there is a significant difference between the two. Specifically, an exemplary case is where p (significance level) is 5%, 1% or lower than 0.1%. Testing methods for statistical procedure may include without particular limitation any known testing methods that can verify significance. For example, Student's t-test, multiple comparison tests may be used.

As a specific example of the disease associated with the abundance of TDP-43 in cells, most diseases known to have the abundance of TDP-43 that is statistically significantly increased compared to that in healthy individuals are nervous system diseases. Thus, nervous system diseases are suitable as a target disease for the present invention. Examples of such nervous diseases include ALS, FTLD, Alzheimer's disease (AD) or Parkinson's disease (PD). ALS or FTLD is preferable as the target disease for the present invention.

### 1-3. Identification method

A method for identifying a disease associated with the abundance of TDP-43 in cells of the present invention comprises a measurement step and an identification step. Each step is specifically described below.

### (1) Measurement step

A "measurement step" in this aspect refers to the step of measuring the intracellular abundance of or the amount of binding to TDP-43 of the test substance in cells derived from the subject.

A "subject" in the present specification refers to an individual subjected to the test, i.e. an animal providing cells described below. An animal as referred to herein is a vertebrate, preferably a mammal, more preferably a human.

"Cells derived from a subject" in the present specification mean cells collected from the subject. Cells may be derived from any organ or tissue. Cerebral nervous system cells, cerebrospinal fluid cells, blood cells or the like may be suitably used in the present invention. Alternatively, nerve cells (motor neurons) or the like differentiated/induced from induced pluripotent stem cells (iPS cells) created based on cells such as skin cells collected from a subject may also be suitably used in the present invention. These cells may be obtained by any known collection method. Alternatively, they may be obtained by cultivating/proliferating cells derived from a subject. For example, blood cells may be obtained by collecting peripheral blood via injection into peripheral veins or the like. Alternatively, cerebrospinal fluid cells may be obtained by collecting encephalon liquid via known lumbar puncture. The cells may be used immediately after being collected from the subject, or may be stored by freezing or refrigeration for a certain period of time, followed, as needed by treatment such as thawing, before being used. The amount of cells used in this step may be at least 2 × 10⁶, preferably at least 1 × 10⁶, more preferably at least 5 × 10⁵ cells, to be capable enough of detecting the test substance described below.

A "test substance" in this aspect is a substance to be measured in this step, and specifically includes,
(i) four types of mt-tRNAs, i.e. mt-tRNA^{Asn}, mt-tRNA^{Gln}, mt-tRNA^{Glu} and mt-tRNA^{Pro},
(ii) glutamic acid (Glu) and aspartic acid (Asp),
(iii) ATP or active oxygen, or
(iv) Aralar 1, Aralar 2, GDH and Musashi 2.

Values to be measured in this measurement step are,
(a) abundance of at least one mt-tRNA selected from the mt-tRNA group described in (i) above in cells,
(b) abundance of one or both of the amino acids described in (ii) above in cells, preferably amino acid concentration,
(c) abundance of ATP or active oxygen described in (iii) above in cells, preferably the concentration of ATP or active oxygen,
(d) amount of binding between at least one polypeptide selected from the polypeptide group described in (iv) above and TDP-43, or
(e) amount of binding between at least one mt-tRNA selected from the mt-tRNA group described in (i) above and TDP-43.

In the present specification, "abundance (in a cell)" refers to the amount of a test substance contained in a cell. Abundance may be a relative amount such as concentration, fluorescence intensity, ionic strength or radiation intensity, or may be an absolute amount of a test substance per predetermined cell number, such as capacity.

In the present specification, the "amount of binding to TDP-43" refers to the relative or absolute amount of a test substance directly or indirectly bound to a predetermined amount of TDP-43. Thus, to "measure the intracellular abundance or the amount of binding to TDP-43, of a test substance" refers to quantitating the intracellular abundance or the amount of binding to TDP-43, of a test substance.

In the amount of binding of a test substance described in (d) above, the amount of Aralar 1 and Aralar 2 specifically refers to the amount of binding to the GR domain and/or the 315 domain in TDP-43. In addition, the amount of binding of the test substance described above in (e) more specifically refers to the amount of binding between at least one mt-tRNA selected from the mt-tRNA group described above in (i) and RRM1 (RNA Recognition Motif-1) domain of TDP-43. For (a) to (e) described above, measurement of any one of them will suffice, although two or more of them may be measured. Measuring two or more of them may avoid incorrect identification due to measurement error or the like and ensure higher identification accuracy.

In healthy individuals, the abundance or the amount of binding per cell of the test substance described above in (a) to (e) is extremely strictly controlled so as not to exceed the predetermined amount by 10%. The present inventors have revealed that a change in the abundance or the amount of binding of the test substance described above in (a) to (e) is closely related to a disease associated with the abundance of TDP-43 in cells. Thus, the presence or absence of the disease associated with the abundance of TDP-43 in cells can be identified by measuring the abundance or amount of binding described in (a) to (e).

Specific measurement methods in this step vary depending on which of the test substances described above in (a) to (e) is measured.

The method for measuring the abundance of mt-tRNA described in (a) in cells may be any method known in the art for detecting RNA. For example, a detection method using a nucleic acid probe which has a nucleotide sequence complementary to all or part of the nucleotide sequence of the target mt-tRNA, and is capable of specifically detecting the target mt-tRNA, or quantitative RT-PCR may be used. Examples of detection methods using a nucleic acid probe include Northern blotting, surface plasmon resonance (SPR) or quarts crystal microbalance (QCM). All the methods mentioned above are techniques known in the art and may be performed in the present invention according to a known procedure. Specifically, reference may be made to the methods described, for example in Sambrook, J. et al. (2001) Molecular Cloning: A Laboratory Manual Third Ed., Cold Spring Harbor Laboratory Press, Cold Spring Harbor, New York, and Toyosaka Moriizumi, Takamichi Nakamoto (1997), Sensor Engineering, Shokodo, Co., Ltd., Toshifumi Inada, Haruhiko Shiomi, 2008, Yodosha Co., Ltd., Notebook for RNA Experiment (1997). Two or more of the methods described above may be used in combination to obtain more accurate measurement results.

The methods for measuring the abundance of amino acids described in (b) and the abundance of ATP or active oxygen described in (c) can employ, for example, mass spectrometry, NMR or luciferase color reaction methods. Mass spectrometry includes liquid chromatography-mass spectrometry (abbreviated hereinafter as "LC-MS"), high performance liquid chromatography tandem mass spectrometry (abbreviated hereinafter as "LC-MS/MS"), gas chromatography-mass spectrometry, gas chromatography tandem mass spectrometry (GC-MS/MS), capillary electrophoresis mass spectrometry (CE-MS) and ICP mass spectrometry (ICP-MS). These analytical methods are techniques known in the art and may be performed accordingly. For example, reference may be made to Iijima et al., The Plant Journal (2008) 54, 949-962, Hirai et al. Proc Natl Acad Sci USA (2004) 101 (27) 10205-10210, Sato et al., The Plant Journal (2004) 40 (1) 151-163 or Shimizu et al., Proteomics (2005) 5, 3919-3931. A preferable measurement method is LC-MS or LC-MS/MS.

Furthermore, the method of measuring the amount of binding between polypeptide and TDP-43 described in (d) may be any method known in the art for detecting interaction between proteins. Examples of the method include an immunological detection method using an antibody that specifically recognizes each polypeptide, i.e. anti-Aralar 1 antibody, anti-Aralar 2 antibody, anti-GDH antibody or anti-Musashi 2 antibody, and anti-TDP-43 antibody. The antibody that specifically recognizes each polypeptide in the present specification may be any of a polyclonal antibody, a monoclonal antibody and a recombinant antibody. The recombinant antibody includes a chimeric antibody and a synthetic antibody. A "synthetic antibody" herein refers to antibodies synthesized by a chemical method or a recombinant DNA method. Examples thereof include a single chain fragment of variable region (scFv), diabody, triabody or tetrabody. Examples of the immunological detection method include co-immunoprecipitation, far-western blotting, ELISA (enzyme-linked immunosorbent assay), enzyme antibody technique and radioimmunoassay. All these methods are known in the art and may in principle be performed in the present invention according to a known procedure. Specifically, reference may be made to the method described, for example in Sambrook, J. et al., (2001) Molecular Cloning: A Laboratory Manual Third Ed., Cold Spring Harbor Laboratory Press, Cold Spring Harbor, New York.

Furthermore, the method for measuring the amount of binding between mt-tRNA and TDP-43 described in (e) may be any method known in the art for detecting the interaction between RNA and proteins. Examples of the method include an immunological nucleic acid detection method using a nucleic acid probe which has a nucleotide sequence complementary to all or part of the nucleotide sequence of the target mt-tRNA, and is capable of specifically detecting each target mt-tRNA, and an anti-TDP-43 antibody. Specifically, the method includes, for example, co-immunoprecipitation, far-western blotting, Northern blotting, ELISA and RT-PCR.

### (2) Identification step

In this aspect, the "identification step" refers to the step comprising comparing a measurement value obtained by the measurement step with a corresponding measurement value in a cell derived from a healthy individual, and when there is a statistically significant difference between these values, identifying the subject as suffering from the disease.

The "corresponding measurement values in the cells derived from a healthy individual" refer to the measurement values of (a) to (e) above in the cells derived from a healthy individual that correspond to the measurement values of (a) to (e) above in the cells derived from a subject, which are obtained by the measurement step above. For example, when the abundance of predetermined mt-tRNA in the cells derived from a subject is measured in the measurement step, the measurement values of the abundance of the predetermined mt-tRNA in the cells derived from a healthy individual will represent the corresponding measurement values in the cells derived from a healthy individual.

The corresponding measurement values in the cells derived from a healthy individual are values measured by the same method and under the same conditions as in the measurement of the test substance in the cells derived from a subject. Thus, in the measurement step, the measurement of the test substance in the cells derived from a subject as well as the measurement of the corresponding test substance in the cells derived from a healthy individual may be performed in the same method and under the same conditions. Also, the measurement values of (a) to (e) are obtained beforehand in healthy individuals with various biologic conditions and compiled into a database, then the data of the healthy individual having biologic conditions closest to those of a subject is retrieved in the database, when measuring the test substance in the cells derived from the subject, then the test substance in the cells derived from the subject may be measured by the measurement method and under the conditions for the test substance in the data. In this case, the presence or absence of the disease associated with the abundance of TDP-43 in cells can be conveniently identified without the need for a control sample derived from a healthy individual.

The statistically significant difference is regardless of whether the abundance or amount of binding of the test substance derived from a subject in cells is higher or lower than its counterpart derived from a healthy individual. This is because the test substance is usually regulated to be within a predetermined range, and any deviation from the range, whatever the degree, may indicate relationship with the disease associated with the abundance of TDP-43 in cells.

Thus, for example, when the abundance of mt-tRNA described in (a) is significantly higher or lower in the cells derived from a subject than in the cells derived from a healthy individual, the subject is identified to be more likely to be suffering from the disease associated with the abundance of TDP-43 in cells. Similarly, when the abundance of the amino acids described in (b) or the abundance of ATP or active oxygen described in (c) is significantly higher or lower in the cells derived from a subject than in the cells derived from a healthy individual, the subject is identified to be likely to be suffering from the disease associated with the abundance of TDP-43 in cells. In addition, when the amount of binding between the polypeptide and TDP-43 described in (d) or the amount of binding between the mt-tRNA and TDP-43 described in (e) is significantly higher or lower in the cells derived from a subject than in the cells derived from a healthy individual, the subject is identified to be more likely to be suffering from the disease associated with the abundance of TDP-43 in cells. More specifically, for example, if in cells derived from a subject with symptoms or signs similar to ALS, the abundance of mt-tRNA described in (a) is significantly higher than in the cells derived from a healthy individual, the abundance of the amino acids described in (b) or the abundance of ATP or active oxygen described in (c) is significantly higher than in the cells derived from a healthy individual, or the amount of binding between the polypeptide and TDP-43 described in (d) or the amount of binding between the mt-tRNA and TDP-43 described in (e) is significantly higher than in the cells derived from a healthy individual, the subject is identified to be more likely to be suffering from ALS. By comparing the measurement values of two or more test substances between a subject and a healthy individual, more accurate identification may be made with lower pseudo-positive and false-negative rates than when a single test substance is measured.

When the corresponding measurement values are compared between a subject and a healthy individual, a known protein or nucleic acid expected to be contained in cells in equal amounts may be used as an internal control to compare and correct for the amount of cells provided for the measurement from the subject and healthy individual. Examples of proteins as an internal control include glyceraldehyde 3-phosphate dehydrogenase (GAPDH), β-actin and albumin, and examples of nucleic acids as an internal control include 5S rRNA, 5.8S rRNA, U1 snRNA. The use of such internal control may lead to more correct measurement values because it allows correction for the quantification results from a subject and a healthy individual.

### 1-4. Effects

According to the identification method of the present invention for the disease associated with the abundance of TDP-43 in cells, the disease associated with the abundance of TDP-43 in cells including thus far refractory ALS can be identified correctly in the precritical or early stage of episodes.

Also, the improvement or aggravation status of the disease in a patient can be monitored by performing the identification method of the present invention for the disease associated with the abundance of TDP-43 in cells for the same patient with the disease associated with the abundance of TDP-43 in cells over time.

### 2. Drug production method

### 2-1. Overview

According to a second aspect of the present invention, a method for producing a drug which reduces the abundance of a measurement substance in cells is provided. A drug which possibly serves as an active ingredient of a therapeutic agent for the treatment of a disease associated with the abundance of TDP-43 in cells can be obtained by the present production method.

### 2-2. Production method

The production method according to the present aspect includes an introduction step, a measurement step, and a selection step. Hereinbelow, each step will be specifically explained.

### (1) Introduction step

According to the present aspect, the "introduction step" refers to a step of introducing a drug candidate substance into cells.

According to the present aspect, the "drug candidate substance" refers to a candidate substance which possibly serves as a drug which reduces the abundance of a measurement substance, which will be explained later, in cells in the production method of the present invention. The type of the substance is not particularly limited as long as the substance can be a candidate for a drug having the aforementioned reduction effect. Examples of the drug candidate substance include a peptide, an oligonucleotide, or a low molecular weight compound. Specific examples of the peptide include an enzyme such as RNAse, ATPase, superoxide dismutase, peroxidase, catalase, and GDH.

According to the present aspect, the "measurement substance" includes some of the measurement substances given in the aforementioned first aspect, which are,
(i) two types of amino acids, which are glutamic acid (Glu) and aspartic acid (Asp),
(ii) ATP or active oxygen, or
(iii) four types of mt-tRNAs, which are mt-tRNA^{Asn}, mt-tRNA^{Gln}, mt-tRNA^{Glu}, and mt-tRNA^{Pro}.

The "cells" used in the present aspect are animal-derived cells, preferably vertebrate-derived cells, more preferably mammal-derived cells, and even more preferably human-derived cells, and particularly, cells derived from a patient with a disease associated with the abundance of TDP-43 in cells, which is the therapeutic target. It does not matter from which organ or tissue the cells are derived. As mentioned earlier, because the drug produced by the production method of the present invention possibly serves as the active ingredient of a therapeutic agent for a disease associated with the abundance of TDP-43 in cells, the cells to be used in the present aspect are preferably cells (including iPS cells) derived from an organ or a tissue at the site where the target disease, which is a disease associated with the abundance of TDP-43 in cells, developed, or cultured cells with over-expression of TDP-43. The cells are preferably cultured cells. Any of primary culture cells, subcultured cells, and cells of an established cell line may be used.

The amount of the drug candidate substance to be introduced into cells may be appropriately determined according to the type of the drug candidate substance used. Also, according to the present aspect, it is sufficient that the drug candidate substance merely have a reduction effect on the abundance of a measurement substance in cells, and the drug candidate substance need not completely remove the measurement substance. This is so because, as will be described later, complete removal of the measurement substance according to the present aspect from inside the cell could cause serious side effects in the cell, and ultimately in the organism, since any of the measurement substances is vital for the survival of the organism.

As a method for introducing a drug candidate substance into cells, a method publicly known in the art may be used. For example, for introducing a nucleic acid molecule or a peptide into cells, a well-known method such as electroporation, a calcium phosphate method, a liposome method, a DEAE dextran method, microinjection, viral infection, lipofection, a method employing a cell membrane-permeable peptide can be used. Further, a commercially available nucleic acid-introducing agent such as Lipofectamin 2000 (Invitrogen) may also be used. When a low molecular weight compound is to be introduced into cells, it normally suffices to add the compound to the medium.

### (2) Measurement step

According to the present aspect, the "measurement step" is different from the measurement step given in the aforementioned first aspect, and is a step of measuring the abundance of the measurement substance in a cell containing the drug candidate substance and in a cell not containing the drug candidate substance. As the measurement method, a similar method to that descried in the aforementioned first aspect may be carried out.

With regard to "a cell containing the drug candidate substance and a cell not containing the drug candidate substance", it is desirable that these cells be put under the same conditions, except the condition of containing or not containing the drug candidate substance. That is, it is desirable that these cells have the same organism of origin, the same tissue of origin, and the same culture conditions (including medium composition (except the presence or absence of the drug candidate substance), culture time, culture temperature, and the like).

### (3) Selection step

According to the present aspect, the "selection step" refers to a selection step, including comparing a measurement value in a cell containing the drug candidate substance and a measurement value in a cell not containing the drug candidate substance obtained by the measurement step, in other words, comparing the abundance of the measurement substances, and when the measurement value in the cell containing the drug candidate substance is statistically significantly lower than the measurement value in the cell not containing the drug candidate substance, selecting the drug candidate substance as the drug of interest. No particular consideration is given to the difference in the measurement values as long as the difference has the significance. This is so because differences in the efficacy of the drug obtained may be accommodated by increasing or decreasing the amount of the drug to be incorporated in one dosage form as appropriate in drug preparation.

### 2-3. Effect

According to the method for producing a drug which reduces the abundance of a measurement substance in cells of the present invention, a hitherto unknown drug which possibly serves as an active ingredient of a therapeutic agent for a disease associated with the abundance of TDP-43 in cells can be obtained.

3. Method for producing a TAR DNA-binding Protein-43 binding inhibitor

### 3-1. Overview

According to a third aspect of the present invention, a method for producing a TDP-43 binding inhibitor is provided. The production method according to the present aspect is characterized by producing a drug capable of significantly reducing the binding between a measurement substance and TDP-43 by the addition of a drug candidate substance.

### 3-2. Production method

The production method according to the present aspect includes a mixing step, a detection step, and a selection step. Hereinbelow, each step will be specifically explained.

### (1) Mixing step

According to the present aspect, the "mixing step" refers to a step of mixing a measurement substance and TDP-43 with a drug candidate substance.

According to the present aspect, the "measurement substance" is different from the measurement substance given in the aforementioned first aspect, and includes at least one of the following substances.
(i) four types of mt-tRNAs, which are mt-tRNA^{Asn}, mt-tRNA^{Gln}, mt-tRNA^{Glu}, and mt-tRNA^{Pro}, and
(ii) four types of proteins, which are Aralar1, Aralar2, GDH, and Musashi 2.

### (A) Drug candidate substance

According to the present aspect, the "drug candidate substance" refers to a candidate substance for a drug capable of significantly reducing the binding between the aforementioned measurement substance and TDP-43, i.e., a candidate substance for a TDP-43 binding inhibitor.

Examples of the aforementioned drug candidate substance include a nucleic acid molecule, a peptide, or a low molecular weight compound.

### (A-1) Nucleic acid molecule

The nucleic acid molecule as the drug candidate substance includes mutant mt-tRNA, a nucleic acid fragment with a binding domain, a nucleic acid with RNAi function, a nucleic acid aptamer, a ribozyme, an antisense nucleic acid, and a U1 adaptor.

According to the present aspect, the "mutant mt-tRNA" refers to a mutant of the wild-type mt-tRNA selected from the group consisting of mt-tRNA^{Asn}, mt-tRNA^{Gln}, mt-tRNA^{Glu}, and mt-tRNA^{Pro}, wherein the mutant has lost its original function (loss-of-function mt-tRNA) due to mutation, while retaining its binding activity to TDP-43. The aforementioned mutation encompasses deletion, substitution, and/or addition of one or several nucleotides in the aforementioned wild-type mt-tRNA.

According to the present aspect, the "nucleic acid fragment with a binding domain" refers to a moiety of the nucleotide sequence of mt-tRNA of the aforementioned measurement substance, and is a partial fragment of mt-tRNA containing the active region, which mediates direct binding to TDP-43. The "nucleic acid fragment with a binding domain" also encompasses a partial fragment of the aforementioned mutant mt-tRNA. Examples of the aforementioned partial fragment of mt-tRNA directly binding to TDP-43 include a common region found in four types of mt-tRNAs (mt-tRNA^{Asn}, mt-tRNA^{Gln}, mt-tRNA^{Glu}, and mt-tRNA^{Pro}) binding to the RRM1 of TDP-43. Specifically, such a common region is a consensus sequence of GUU in the D stem & loop and UGG in the T stem & loop. Thus, a partial fragment of mt-tRNA containing a nucleotide sequence consisting of GTT or GUU and/or TGG or UGG or a DNA fragment thereof can be a drug candidate substance since it can compete with the aforementioned four types of mt-tRNAs for binding to the RRM1 of TDP-43. The above partial fragment of mt-tRNA may be 5 to 50-nucleotide long, preferably 5 to 30-nucleotide long.

According to the present aspect, the "nucleic acid with RNAi function" refers to a substance capable of inducing RNA interference (RNAi) in the body of an organism to thereby degrade the transcription product of the target gene, thereby silencing the expression of the gene. Examples of the nucleic acid with RNAi function include small interfering RNA (siRNA), short hairpin RNA (shRNA), or micro RNA (miRNA). Also, for RNAi, see, for example, Bass B. L., 2000, Cell, 101, 235 to 238; Sharp P. A., 2001, Genes Dev., 15, 485 to 490; Zamore P. D., 2002, Science, 296, 1265 to 1269; and Dernburg, A. F. & Karpen, G. H., 2002, Cell, 111, 159 to 162). According to the present aspect, examples of a gene encoding a transcription product which can be the target of the nucleic acid with RNAi function include the gene encoding each of mt-tRNA^{Asn}, mt-tRNA^{Gln}, mt-tRNA^{Glu}, mt-tRNA^{Pro}, Aralar1, Aralar2, GDH, and Musashi 2. The nucleic acid with RNAi function directed to these targets may be designed using a technique publicly known in the art. Giving one specific example, for designing siRNA, for example, first of all, from the nucleotide sequence of the Aralar1 gene, select a nucleotide sequence which is specific for the Aralar1 gene and which is a consecutive nucleotide sequence region of 15 nucleotides or more and 35 nucleotides or less, preferably 15 nucleotides or more and 30 nucleotides or less, and more preferably 18 nucleotides or more and 25 nucleotides or less as the nucleotide sequence of the sense strand of siRNA. Subsequently, find a nucleotide sequence complementary to the aforementioned nucleotide sequence of the RNA sense strand thus selected as the nucleotide sequence of the antisense strand. Further, in preparing siRNA, convert thymine (T) nucleotides in the selected region to uracil (U) nucleotides in both the sense and antisense strands. Further, the guanine-cytosine (GC) content in the RNA sense strand of the selected region is preferably 20 to 80%, more preferably 30 to 70%, and even more preferably 40 to 60%. Also, it is preferable to add thymine-thymine (TT) or uracil-uracil (UU), preferably TT, to the 3'-terminal end of the RNA sense and antisense strands of the selected region.

According to the present aspect, the "nucleic acid aptamer" refers to a ligand nucleic acid capable of specifically binding to the target substance (here, a measurement substance or TDP-43) by virtue of its own three-dimensional structure. As the nucleic acid aptamer, DNA aptamer, RNA aptamer, and a mixed aptamer thereof are known according to its type. In the present aspect, any of those aptamers may be used. With regard to aptamers, see, for example, Janasena, Clin. Chem., 1999, 45: 1628 to 1650.

According to the present aspect, the "ribozyme" refers to an RNA molecule having a catalytic activity, which is alternatively called ribozyme. The ribozyme specifically binds to any of the four types of mt-tRNA molecules given in the aforementioned (i) of the present aspect, which are not only substrates, but also target substances (measurement substances), thereby functioning to cleave or connect the target RNA molecule or catalyze chemical reactions such as oxidation and reduction.

According to the present aspect, the "antisense nucleic acid" refers to, when the measurement substance is a protein, an antisense nucleic acid targeting the transcription product of the gene of the protein. A nucleic acid as used herein encompasses not only DNA or RNA, but also, for example, nucleic acid analogs such as PNA and LNA.

According to the present aspect, the "U1 adaptor" refers to a bifunctional oligonucleotide consisting of approximately 25 nucleotides, which contains the 5'-side "target domain", which is complementary to the 3'-terminal exon of the pre-mRNA of the target gene, and the 3'-side "U1 domain", which has a sequence complementary to the 5' region of U1 snRNA (Goraczniak R., et al., 2009, Nat Biotechnol., Vol 27, pp. 257 to 263,). When a U1 adaptor is introduced, a U1 small nuclear ribonucleoprotein (U1 snRNP) including U1 snRNP binds near the poly(A) signal of the pre-mRNA of the target gene, thereby specifically inhibiting polyadenylation of this mRNA. As a result, the pre-mRNA of the target gene is rendered unstable and eventually degraded in the nucleus, whereby gene silencing is accomplished. In light of the above, a U1 adaptor targeting the gene of at least one protein selected from the group consisting of Aralar1, Aralar2, GDH, and Musashi 2 may be designed.

### (A-2) Peptide

The peptide as the drug candidate substance includes a mutant protein, a peptide fragment with a binding domain, an antibody, a peptide aptamer, an enzyme, and the like. The peptide may be either an oligopeptide or a polypeptide. The size of the peptide is not particularly limited as long as the peptide can function as a TDP-43 binding inhibitor. Normally, the peptide consists of 20 amino acids or less, preferably 15 amino acids or less, and more preferably 10 amino acids or less. Further, the peptide may also be modified. The peptide modification includes modification for functional purposes or modification for labeling purposes. Examples of the modification for functional purposes include glycosylation, acetylation, formylation, amidation, phosphorylation, methylation, circularization, or PEGylation. Examples of the modification for labeling purposes include labeling with a fluorescent dye (fluorescein, FITC, rhodamine, Texas Red, Cy3, and Cy5), a fluorescent protein (such as PE, APC, and GFP), an enzyme (such as horseradish peroxidase, alkaline phosphatase, and glucose oxidase), a radioactive isotope (such as ³H, ¹⁴C, and ³⁵S) or biotin or (strept)avidin.

According to the present aspect, the "mutant protein" refers to a mutant of the wild-type protein selected from the group consisting of Aralar1, Aralar2, GDH, Musashi 2, and TDP-43, wherein the mutant has lost its original function (loss-of-function-type protein) due to mutation or has its original function enhanced or has gained a new function (gain-of function-type protein) by mutation, and wherein, when the mutant is a mutant of Aralar1, Aralar2, GDH, or Musashi 2, it retains its binding activity to TDP-43, and when the mutant is a mutant of TDP-43, it retains its binding activity to one protein selected from the group consisting of Aralar1, Aralar2, GDH, and Musashi 2 or to mt-tRNA selected from the group consisting of mt-tRNA^{Asn}, mt-tRNA^{Gln}, mt-tRNA^{Glu}, and mt-tRNA^{Pro}. The aforementioned mutation encompasses deletion, substitution, and/or addition of one to several amino acids in the aforementioned wild-type protein.

According to the present aspect, the "peptide fragment with a binding domain" refers to a moiety of the amino acid sequence of TDP-43 or the measurement substance protein given in the aforementioned (ii) of the present aspect, and is a partial fragment of the protein containing the active region, which mediates direct binding to TDP-43 or the measurement substance protein given in the aforementioned (ii) of the present aspect. The "peptide fragment with a binding domain" also encompasses a partial fragment of the aforementioned mutant protein. For example, when the peptide fragment with a binding domain is a peptide fragment with a TDP-43 binding domain, the peptides listed in the following (a) to (d) fall under the peptide fragment with a binding domain:
(a) a polypeptide of 100 amino acids or less, preferably 80 amino acids or less, comprising a polypeptide consisting of an amino acid sequence set forth in SEQ ID NO: 2, 4, or 5,
(b) a polypeptide of 130 amino acids or less, preferably 100 amino acids or less, more preferably 80 amino acids or less, comprising a polypeptide comprising addition, deletion, or substitution of 1 to 3 amino acids, preferably 1 or 2 amino acids in an amino acid sequence set forth in SEQ ID NO: 2, 4, or 5,
(c) an amino acid sequence having a 90% or more, preferably 95% or more, more preferably 97% or more identity with an amino acid sequence set forth in SEQ ID NO: 2, 4, or 5, and
(d) a moiety of a polypeptide consisting of an amino acid sequence set forth in SEQ ID NO; 2, 4, or 5.

Here, SEQ ID NO: 2 corresponds to the RRM1 domain of human TDP-43 set forth in SEQ ID NO: 1, which is located from position 105 to 169. Also, SEQ ID NO: 4 corresponds to the GR domain of human TDP-43 set forth in SEQ ID NO: 1, which is located from position 274 to 314. Further, SEQ ID NO: 5 corresponds to the 315 domain of human TDP-43 set forth in SEQ ID NO: 1, which is located from position 315 to 414. Also, SEQ ID NO: 3 corresponds to the RNA Recognition Motif-2 (RRM2) domain of human TDP-43 set forth in SEQ ID NO: 1, which is located from position 193 to 257.

The research results of the present inventors have revealed that RRM1 of TDP-43 is the binding domain for mt-tRNA^{Asn}, mt-tRNA^{Gln}, mt-tRNA^{Glu}, and mt-tRNA^{Pro}, which are the aforementioned measurement substances, and the GR domain and 315 domain are the binding domains for Aralar1 and Aralar2. Thus, a peptide fragment containing the RRM1 domain of TDP-43 or a peptide having a 90% or more, preferably 95% or more, more preferably 98% or more identity with an amino acid sequence of the above peptide fragment can be a drug candidate substance since it can compete with TDP-43 for binding to the aforementioned four types of mt-tRNAs. The above peptide fragment may be 5 to 60-amino acid long, preferably 9 to 20-amino acid long.

According to the present aspect, the "antibody" refers to a substance functioning as a neutralization antibody, and examples thereof include a monoclonal antibody, a polyclonal antibody, a recombinant antibody, and an antibody fragment.

When the antibody is a polyclonal antibody or a monoclonal antibody, the immunoglobulin molecule may be any class (such as IgG, IgE, IgM, IgA, IgD and IgY) or any subclass (such as IgG1, IgG2, IgG3, IgG4, IgA1, and IgA2).

The "recombinant antibody" refers to a chimeric antibody, a humanized antibody, a synthetic antibody, or a multispecific antibody.

The "chimeric antibody" refers to an antibody produced by combining the amino acid sequences of antibodies derived from different animals, and is obtained by replacing the constant region (C region) of an antibody by the C region of another antibody. For example, an antibody resulting from replacing the C region of a mouse monoclonal antibody by the C region of a human antibody falls under the chimeric antibody. By doing so, immune reactions against the above antibody in the human body can be reduced.

The "humanized antibody" refers to a mosaic antibody resulting from replacing the complementarity-determining region (CDR) in the V region of an antibody derived from a mammal other than a human, for example, a mouse, by CDR of a human antibody.

The "synthetic antibody" refers to a chemically synthesized antibody or an antibody synthesized by using the recombinant DNA method. Examples thereof include an antibody newly synthesized using the recombinant DNA method. Specific examples thereof include a single chain Fragment of variable region (scFv), a diabody, a triabody, or a tetrabody.

The "multispecific antibody" refers to a polyvalent antibody, that is, an antibody having a plurality of antigen-binding sites within a molecule, in which the antigen-binding sites bind to respective different epitopes. Examples of the multispecific antibody include a bispecific antibody, which is an antibody having two antigen-binding sites as IgG, in which the antigen-binding sites bind to respective different epitopes.

The "antibody fragment" includes, for example, Fab, F(ab')2, and Fv.

Any of the aforementioned antibodies may be produced by using a method publicly known in the art.

According to the present aspect, the "peptide aptamer" refers to, similarly to the aforementioned nucleic acid aptamer, a peptide capable of specifically binding to the target substance (here, the measurement substance or TDP-43) by virtue of its own three-dimensional structure.

According to the present aspect, the "enzyme" refers to an RNAse, preferably, an RNAse capable of specifically recognizing and degrading each of mt-tRNA^{Asn}, mt-tRNA^{Gln}, mt-tRNA^{Glu}, and mt-tRNA^{Pro}, and a protease, preferably a protease capable of specifically recognizing and degrading each of Aralar1, Aralar2, GDH, and Musashi 2.

### (A-3) Low molecular weight compound

In the present specification, the "low molecular weight compound" refers to a compound having a molecular weight of 5000 or less, preferably 2000 or less, more preferably 1000 or less, and includes a substance other than the aforementioned nucleic acids and peptides. Examples of the low molecular weight compound include various pharmaceutical compounds such as hormones (such as steroid hormone), neurotransmitters (such as adrenaline, epinephrine, noradrenaline, and dopamine), histone deacetylase inhibitors, and immunosuppressants. Examples of the low molecular weight compound also include a derivative of a low molecular weight compound having an equivalent pharmacological activity to a specific low molecular weight compound, or a salt thereof.

### (B) Specific examples of a drug candidate substance

Examples of a drug candidate substance include a competitive substance or an indirect binding inhibitor.

### (B-1) Competitive substance

According to the present aspect, the "competitive substance" refers to a substance inhibiting the binding between a measurement substance and TDP-43 by competing with the measurement substance for binding to TDP-43 or by competing with TDP-43 for binding to the measurement substance. According to the present aspect, for example, when TDP-43 is deposited in the site when TDP-43 is isolated from an intracellular site where it is supposed to exert its function by forming aggregates through incorporation of a measurement substance such as mt-tRNA, a substance capable of dissociating such aggregates is also encompassed by the competitive substance.

As the competitive substance which competes with a measurement substance, mutant mt-tRNA, a nucleic acid fragment with a binding domain, a nucleic acid aptamer, a mutant protein, a peptide fragment with a binding domain, or a low molecular weight compound can be preferably used as a drug candidate substance. Also, as the competitive substance which competes with TDP-43, a mutant protein, a peptide fragment with a binding domain, or a low molecular weight compound can be preferably used. Also, as described earlier, a nucleic acid molecule containing a common region found in four types of mt-tRNAs, which mediates binding to RRM1 of TDP-43, specifically, a consensus sequence of GUU in the D stem & loop and UGG in the T stem & loop, or a part of the sequence, or a peptide containing RRM1 of TDP-43, to which four types of mt-tRNAs bind, or a peptide containing the GR domain and 315 domain, to which Aralar1 and Aralar2 bind, can also be preferably used.

### (B-2) Indirect binding inhibitor

According to the present aspect, the "indirect binding inhibitor" refers to a substance which secondarily inhibits the binding between a measurement substance and TDP-43 by reducing the absolute abundance of a measurement substance or the abundance of an activated measurement substance in cells.

As the indirect binding inhibitor which reduces the absolute abundance of a measurement substance in cells, a nucleic acid with RNAi function, a ribozyme, an antisense nucleic acid, a U1 adaptor, an enzyme, or a low molecular weight compound can be preferably used. Also, as the indirect binding inhibitor which reduces the abundance of an activated measurement substance in cells, a mutant mt-tRNA, a nucleic acid fragment with a binding domain, a nucleic acid aptamer, a mutant protein, a peptide fragment with a binding domain, an antibody, or a peptide aptamer can be preferably used.

### (C) Method

The mixing step may be performed either *in vivo* using cultured cells and the like or *in vitro.*

When the present step is performed *in vivo,* the "cells" used in the present aspect refer to animal-derived cells, preferably vertebrate-derived cells, more preferably mammal-derived cells, even more preferably human-derived cells, and particularly, cells derived from a patient with a disease associated with the abundance of TDP-43 in cells, which is the therapeutic target. The cells are preferably cultured cells, more preferably cultured cells with over-expression of TDP-43 (including iPS cells). Any of primary culture cells, subcultured cells, and cells of an established cell line may be used.

The method for determining the amount of the drug candidate substance introduced into cells and introducing the drug candidate substance into cells may be carried out in accordance with the aforementioned second aspect.

While endogenous TDP-43 and measurement substances may be used, exogenous TDP-43 and measurement substances may be introduced into cells together with or separately from the drug candidate substance. While TDP-43 or a measurement substance itself may be introduced into cells, it is also possible to introduce a nucleic acid encoding these substances into cells and allow them to be expressed inside the transformed cells. The cells after introduction are cultured for several days under the conditions of appropriate time, temperature, and CO₂ concentration. The cell culture method may be carried out by a method publicly known in the art.

Also, when the present step is carried out *in vivo,* it is necessary, before the detection step, to prepare a cell extract containing complexes formed by binding between the aforementioned TDP-43 and the aforementioned mt-tRNA and/or proteins by lysing the cells into which the drug candidate substance and the like are introduced. For the method for preparing a cell extract, a publicly known cell extract preparation method using a lysis buffer and the like may be used.

When the present step is carried out *in vitro*, TDP-43, a measurement substance, and a drug candidate substance may be each mixed based on a method publicly known in the art in which protein-protein interaction or protein-nucleic acid interaction can take place. For example, the method described in Sambrook, J. et. al., (2001) Molecular Cloning: A Laboratory Manual Third Ed., Cold Spring Harbor Laboratory Press, Cold Spring Harbor, New York may be referred to. Further, it is also possible to immobilize any of TDP-43, a measurement substance, and a drug candidate substance to a solid support in advance. The above operation is convenient because, by doing so, detection of the complexes in the subsequent detection step is made easy. As the solid support, an insoluble support in the form of a bead, a microplate, a test tube, a stick, a test piece, or the like made of a material such as polystyrene, polycarbonate, poly(vinyl toluene), polypropylene, polyethylene, poly(vinyl chloride), nylon, polymethacrylate, latex, gelatin, agarose, cellulose, sepharose, glass, metal, ceramic, or a magnetic substance can be used. Immobilization is carried out by directly binding any of TDP-43, a measurement substance, and a drug candidate substance to a solid support by a publicly known method such as physical adsorption, chemical binding, or a combination of these methods. It is also possible to perform a method which includes immobilizing an antibody such as an anti-TDP-43 antibody to a solid support in advance, and then indirectly binding TDR-43, which is the antigen, to the solid support by antigen-antibody reaction.

### (2) Detection step

According to the present aspect, the "detection method" refers to a method of detecting the binding between the aforementioned TDP-43 and the aforementioned mt-tRNA and/or protein. In the present step, when the aforementioned binding is detected, the amount of binding is desirably quantitated before proceeding to the subsequent step.

The detection step can be accomplished basically by applying a similar technique to that used in the measurement step described in the aforementioned first aspect. For detection of the binding between TDP-43 and the aforementioned mt-tRNA, a method publicly known in the art for detecting protein-nucleic acid complex may be used. For example, after collecting TDP-43 containing complexes by immunoprecipitation using anti-TDP-43 antibody, TDP-43 is removed from the complexes by a denaturing agent such as phenol or by a protease, and the remaining nucleic acid molecules may be detected by Northern blotting using a nucleic acid probe capable of specifically detecting the target mt-tRNA, or by quantitative RT-PCR. Further, also, for detection of the binding between TDP-43 and the aforementioned protein, a method publicly known in the art for detecting the target protein-protein complex may be used. For example, after collecting TDP-43 containing complexes by coimmunoprecipitation using anti-TDP-43 antibody, the protein may be detected by Western blotting using an antibody specific for the target protein. Explaining with a specific example, for example, cell-derived total RNA, mt-tRNA^{Asn}, or mt-tRNA^{Gln} is prepared and mixed with tag-fused TDP-43, which is synthesized in *E. coli* and the like and then purified *in vitro*, and after collecting an antibody or binding substance against the tag (for example, a DAP tag, a FLAG tag, a histidine tag, a HA tag, a GFP tag, and the same will apply hereinbelow) by a solid support, mt-tRNA^{Asn} and mt-tRNA^{Gln} bound to the tag-fused TDP-43 are separated by urea-modified acrylamide gel, and then detected and quantitated by staining with SYBR GOLD and the like. When a more sensitive detection method is necessary, detection and quantitation are performed by Northern blotting. Also, when purified mt-tRNA^{Asn} or mt-tRNA^{Gln} is used, it can be labeled with a fluorescent reagent or other color developing reagents, an enzyme, biotin, or the like, and these molecules which have bound to the immobilized tag-fused TDP-43 can be quantitated based on the fluorescence, intensity of color development, and the like. Conversely, it is possible to immobilize purified mt-tRNA^{Asn} or mt-tRNA^{Gln} and quantitate the binding of TDP-43 fused with a fluorescent reagent or other color developing reagents or an enzyme to the immobilized purified mt-tRNA^{Asn} or mt-tRNA^{Gln} by measuring fluorescence or intensity of color development. In this case, instead of using the full-length mt-tRNA^{Asn}, mt-tRNA^{Gln}, or TDP-43 molecule, it is also possible to use an RNA fragment or a peptide fragment containing the binding sequence of each of the above molecules. As a more specific example of interaction detection, there is a method which includes synthesizing trigger factor (TG)-GST-fused TDP-43 in *E. coli,* purifying the TDP-43 by affinity chromatography using a glutathione-immobilized support, mixing the TDP-43 with total RNA extracted from cells, and then detecting the RNA by Northern blotting.

Further, it is also possible to prepare a cell line expressing tag-fused TDP-43, collect the tag-fused TDP-43 expressed using a solid support to which an antibody (such as an anti-FLAG antibody) or a binding substance (such as streptavidin and avidin) against the tag is immobilized, extract mt-tRNA^{Asn} and/or mt-tRNA^{Gln} bound to the tag-fused TDP-43, separate the mt-tRNA^{Asn} and/or mt-tRNA^{Gln} by urea-modified acrylamide gel, and then detect and quantitate the mt-tRNA^{Asn} and/or mt-tRNA^{Gln} by SYBR GOLD staining or by Northern blotting. Quantitative changes in mt-tRNA^{Asn} and/or mt-tRNA^{Gln} bound to the tag-fused TDP-43 in cells can be detected and quantitated by allowing a peptide, a nucleic acid, and a compound to coexist while collecting the tag-fused TDP-43.

Alternatively, it is also possible to quantitate the binding by immobilizing TDP-43 to a solid support in advance, passing a measurement substance therethrough to thereby form complexes of TDP-43 and the measurement substance, passing a solution containing a drug candidate substance therethrough so as to create competition between the measurement substance and the drug candidate substance, and then calculating the amount of dissociated complexes by surface plasmon resonance spectroscopy or ELISA.

### (3) Selection step

According to the present aspect, the "selection step" is different from the "selection step" described in the aforementioned second aspect, and is a step of selecting, when the binding between TDP-43 and mt-tRNA and/or protein, which is the measurement substance, is not detected in the detection step, or a comparison between the amount of binding detected and the amount of binding between a TAR DNA-binding protein-43 and mt-tRNA and/or protein in the absence of the drug candidate substance shows a statistically significant difference, the drug candidate substance as a TAR DNA-binding protein-43 binding inhibitor.

The case "when the binding between TDP-43 and mt-tRNA and/or protein, which is the measurement substance, is not detected" in the aforementioned detection step indicates that the drug candidate substance completely inhibits the binding between TDP-43 and mt-tRNA and/or protein, which is the measurement substance. In that case, the drug candidate substance is selected as the TDP-43 binding inhibitor of interest as it can be a potent TDP-43 binding inhibitor. However, such a TDP-43 binding inhibitor may also adversely block the interaction between TDP-43 and the measurement substance vital for the survival of the organism, causing serious side effects in cells, and ultimately in the organism. Therefore, in use of the TDP-43 binding inhibitor, it is necessary to make sure that the TDP-43 binding inhibitor is a drug having a temporary effect, and also, to optimize, in advance, the dose at which only beneficial efficacy can be obtained. However, a drug candidate substance which specifically and completely inhibits the binding between a gain-of-function-type mutant TDP-43 and a measurement substance is most preferable as the TDP-43 binding inhibitor.

Also, the case when "a comparison between the amount of binding detected and an amount of binding between a corresponding mt-tRNA and/or protein and TDP-43 in an absence of the drug candidate substance shows a statistically significant difference" in the aforementioned detection step indicates that a comparison between the amounts of binding between TDP-43 and the measurement substance under the conditions in which TDP-43 and the measurement substance are the same and only difference is the presence or absence of the addition of the drug candidate substance shows a statistically significant difference. This means that, in other words, the drug candidate substance has partially inhibited the binding between TDP-43 and the measurement substance. The above partially-inhibiting drug candidate substance is preferable as a TDP-43 binding inhibitor, and thus is selected as the TDP-43 binding inhibitor of interest.

It should be noted that the detection of the binding between mt-tRNA and/or protein and TDP-43 in the absence of a drug candidate substance is entirely carried out by the same method under the same conditions as in the case of detection of the binding between mt-tRNA and/or protein and TDP-43 in the presence of a drug candidate substance, except differing in the presence or absence of the drug candidate substance. Accordingly, in the aforementioned detection step, the above operation may also be carried out at the same time with detection of the binding between mt-tRNA and/or protein and TDP-43 in the presence of a drug candidate substance. Further, it is also possible to quantitate the amount of binding between mt-tRNA and/or protein and TDP-43 in the absence of a drug candidate substance in advance, compile the results in a database, and quantitate the amount of binding in the case in which only a drug candidate substance is newly added by the same method under the same conditions.

The TDP-43 binding inhibitor obtained by the production method of the present aspect possibly serves as an active ingredient of a therapeutic agent for a disease associated with the abundance of TDP-43 in cells.

It should be noted that the inhibitory effect of the drug which reduces the abundance of a measurement substance according to the aforementioned second aspect or the TDP-43 binding inhibitor according to the present aspect on cytotoxicity caused by overexpression of TDP-43 can be confirmed by the changes in the cell proliferation rate caused by treatment with these drugs. When the cell proliferation rate is statistically significantly increased compared to cells not treated with the drug, then the drug can be determined as having an inhibitory effect on cytotoxicity. It is to be noted that the cell proliferation rate can be obtained by various methods publicly known in the art, such as calculating from direct counting of the number of cells under a microscope or measuring the amount of bromouridine. Alternatively, it is also possible to measure the amount of ATP or active oxygen instead of the cell proliferation rate. In this case, using an increased amount of ATP or active oxygen in cells caused by an increased expression level of TDP-43 as an index, when the amount of ATP or active oxygen in cells treated with the drug is statistically significantly decreased, then the drug can be determined as having an inhibitory effect on cytotoxicity. Further, when an increase in the amount of mt-tRNA^{Asn} and mt-tRNA^{Gln} in cells has been successfully decreased by the aforementioned drug treatment, then the drug can be determined as having an inhibitory effect on cytotoxicity.

### 4. TAR DNA binding protein-43 binding inhibitor

### 4-1. Overview

According to a fourth aspect of the present invention, a TDP-43 binding inhibitor is provided. The TDP-43 binding inhibitor of the present invention possibly serves as the active ingredient of a therapeutic agent for a disease associated with the abundance of TDP-43 in cells.

### 4-2. Configuration

The TDP-43 binding inhibitor according to the present aspect is a competitive substance consisting of a nucleic acid molecule inhibiting the binding between TDP-43 and four types of mt-tRNAs (mt-tRNA^{Asn}, mt-tRNA^{Gln}, mt-tRNA^{Glu}, and mt-tRNA^{Pro}).

In the present specification, as a general rule, the "nucleic acid molecule" refers to a biopolymer in which constituent units, the constituent unit being a nucleotide, are connected by a phosphodiester linkage. Accordingly, the nucleic acid molecule normally includes a natural-type nucleic acid in which naturally occurring natural-type nucleotides are connected, such as DNA, in which deoxyribonucleotides, each having one of the nucleotides adenine, guanine, cytosine, and thymine, are connected, and/or RNA, in which ribonucleotides, each having one of the nucleotides adenine, guanine, cytosine, and uracil, are connected. Besides DNA and RNA, the nucleic acid molecule of the present invention can also encompass a non-natural type nucleotide or a non-natural type nucleic acid.

In the present specification, the "non-natural type nucleotide" refers to a nucleotide which does not exist in nature. For example, an artificially-constructed or chemically-modified nucleotide having similar properties and/or configurations to those of the aforementioned natural-type nucleotide falls under the non-natural type nucleotide.

In the present specification, the "non-natural type nucleic acid" refers to an artificially-constructed nucleic acid analog having similar configurations and/or properties to those of a natural-type nucleic acid. Examples thereof include a Peptide Nucleic Acid (PNA), a phosphate group-bearing peptide nucleic acid (PHONA), a Bridged Nucleic Acid/Locked Nucleic Acid (BNA/LNA), and a morpholino nucleic acid. Further, examples thereof include a chemically-modified nucleic acid and a nucleic acid analog such as methylphosphonate-type DNA/RNA, phosphorothioate-type DNA/RNA, phosphoramidate-type DNA/RNA, and 2'-O-methyl-type DNA/RNA.

The nucleic acid molecule inhibiting the binding between TDP-43 and four types of mt-tRNAs (mt-tRNA^{Asn}, mt-tRNA^{Gln}, mt-tRNA^{Glu}, and mt-tRNA^{Pro}) contains a moiety of the consensus sequence found in the aforementioned four types, which corresponds to the TDP-43 binding domain. Specifically, the above nucleic acid molecule has a nucleotide sequence consisting of TG or UG and/or GT or GU, for example, a nucleotide sequence consisting of TGG or UGG and/or GTT or GUU. The nucleic acid molecule of the present invention contains two or more of such a nucleotide sequence, preferably three or more, more preferably five or more, even more preferably six or more, seven or more, eight or more, nine or more, and 10 or more of such a nucleotide. One nucleic acid molecule may contain the same above nucleotide sequence (for example, only TG or GT) or a combination of the above nucleotide sequences (for example, TG and GT). Each nucleotide sequence may be discontinuous or successively repeated. In the case when the sequence is successively repeated, the number of repeats is, for example, 3 to 20, preferably 4 to 18, more preferably 5 to 15. The nucleotide length of the nucleic acid molecule is 5 to 50-nucleotide-long, preferably 10 to 40-nucleotide-long, more preferably 15 to 30-nucleotide-long. Specific examples of the aforementioned nucleic acid molecule include the DNA oligonucleotide (TG)₁₂ set forth in SEQ ID NO: 43, which corresponds to a sequence having 12 repeats of UG, which is a moiety of the consensus sequence UUG contained in the T stem & loop, the DNA oligonucleotide (TGG)₈ set forth in SEQ ID NO: 45, which consists of a sequence having eight repeats of the consensus sequence UGG contained in the T stem & loop, and the DNA oligonucleotide TT(GTT)₇G set forth in SEQ ID NO: 46, which consists of a sequence having seven repeats of the consensus sequence TTG contained in the D stem & loop.

### Examples

### <Example 1: Identification of RNA bound to TDP-43>

### (Object)

RNA bound to TDP-43 is identified.

### (Method)

### (1) Preparation of DAP-tagged TDP-43 expression vectors

A DAP (doubly affinity-purification) tag is a tag in which a 6 × His tag, biotinylated tag and a Flag tag are connected in series in this order from the amino terminal. A gene segment of Flag tag-fused TDP-43 was first prepared with pEGFP-TDP-43 (Arai et al., Biochem Biophys Res Commun, 2006, 351:602-611) as a template by PCR using KOD-Plus DNA polymerase (TOYOBO) to construct a DAP-tagged TDP-43 (DAP-TDP-43) expression vector. Oligonucleotides with a nucleotide sequence set forth in SEQ ID NOs: 6 and 7 were used as primers. For cloning, the PCR product was inserted into the KpnI/XhoI site in the pcDNA5/FRT/TO (lifetechnologies) vector. The cDNA encoding the 6 × His and biotinylated sequences was prepared by PCR amplification with pcDNA3.1(+)-bio as a template according to the method of Hayano et al. (Interaction Analysis. J. Proteome Res., 2008, 7: 4183-4190). Oligonucleotides with a nucleotide sequence set forth in SEQ ID NOs: 8 and 9 were used as primers. The resulting vector was designated as DAP-TDP-43 pcDNA5/FRT/TO.

### (2) Construction of a doxycycline-induced cell line

Flp-In-T-REx Expression System (lifetechnologies) was used to prepare a cell line expressing DAP-TDP-43 induced by doxycycline. Flp-In-T-REx 293 cells (293 TRex cells) were cultured in a 24 well plate at 37°C, 5% CO₂, using DMEM culture media (Sigma-Aldrich) supplemented with 10% immobilized bovine serum (Biowest LLC), 0.1 mg/mL streptomycin (Wako Pure Chemicals) and 100 U/mL penicillin G (Wako Pure Chemicals). At about 80% confluence, cells were transfected with 0.25 µg of pOG44 (lifetechnologies) and 0.25 µg of DAP-TDP-43 pcDNA5/FRT/TO in 2 µL of Lipofectamine 2000. After 48 hours, cell strains having TDP-43 monoclones inserted into the common genome site (DAP-TDP-43 expressing strains) were selected in culture media containing 200 µg/mL hygromycin B (lifetechnologies).

100 ng/mL doxycycline was added and cultured for 24 hours to induce DAP-TDP-43 expression.

### (3) Preparation of cell extract

Cell extracts were prepared according to the method of Izumikawa et al. (Biochem J., 2008, 413 (3): 505-516). That is, after rinsing in PBS, the cells were suspended for 30 minutes in ice in a dissolution buffer (50 mM Tris/HCl (pH 7.4), 150 mM NaCl, 0.5% (w/v) IgepalCA-630) 5 times the cell mass in volume containing 2 mM ribonucleoside-vanadyl complex, 1 mM PMSF, 2 µg/mL aprotinin, 2 µg/mL pepstatin A and 2 µg/mL leupeptin. The cells were centrifuged at 20000 g for 30 minutes at 4°C and the supernatant was collected as the cell extract. The protein concentration in the cell extract was measured by Protein Assay (Bio-Rad).

### (4) Immunoprecipitation and RNA collection

To immunoprecipitate DAP-TDP-43 containing the Flag tag, 6 mg of protein fractions were prepared from the cell extract at 2 × 10⁷ cells, and 15 µL of ANTI-Flag-M2 affinity gel (Sigma-Aldrich) was added followed by incubation at 4°C for two hours. The affinity gel bound to DAP-TDP-43 was washed five times in 1 mL of the dissolution buffer described above and eluted at 4°C for 5 minutes, using 150 µL of protein-RNA extraction buffer (7 M urea, 350 mM NaCl, 1% SDS, 10 mM Tris-HCl (pH 8.0), 10 mM EDTA, 2% 2-mercaptoethanol). The RNA co-immunoprecipitated with the protein eluted was separated from the gel by centrifugation at 1000 g for 5 minutes at 4°C, and extracted with phenol-chloroform to separate the protein from RNA. The protein was precipitated by mixing the organic layer after phenol-chloroform extraction with 3 volumes of isopropanol and centrifuging at 20000 g for 10 minutes at 4°C, and washed once immediately with 75% ethanol and dried. The RNA was collected from the aqueous layer by isopropanol precipitation.

### (5) Urea denaturation PAGE

The separation of the RNA collected from the cell extract essentially followed the method described in Sambrook, J. et al. (Molecular Cloning: A Laboratory Manual Third Ed., 2001, Cold Spring Harbor Laboratory Press, Cold Spring Harbor, New York). The RNA obtained by the pull-down method using beads with immobilized anti-Flag antibodies was dissolved in formamide, denatured by heat-treating at 65°C for 10 minutes, and quenched in ice. After adjusting the RNA to achieve a final concentration before electrophoresis of 80% formamide, 10 mM EDTA (pH 8.0), 1/16 volumes of 10 × Loading buffer (TaKaRa), the RNA was separated with 8% polyacrylamide gel containing 8 M urea and 0.5 × TBE buffer. After electrophoresis, the gel was stained with SYBR Gold (lifetechnologies) for 5 minutes, and the band specifically bound to TDP-43 was cut out.

### (6) In-gel digestion

The in-gel digestion of the RNA essentially followed the method of Taoka et al. (Anal. Chem., 2010, 82 (18): 7795-7803). The cut out gel portion was cut into small pieces and dried under vacuum. The pieces were then treated for 1 hour with 15 µL of 2 µg/µL RNase T1 or RNase A at 37°C. After dissolving in 100 µL of RNase-free water, nucleic acid fragments were extracted from the gel by passing through the centrifugal filter tube (Ultrafree-MC, Millipore) containing a polyvinylidene fluoride filter, to which 5 µL of 2 M triethylammonium acetate (pH 7.0) was added.

### (7) Nano LC-MS/MS analysis

The LC-MS/MS analysis essentially used the LC system (LC Assist, Japan) consisting of a nanoflow pump disclosed in Natsume et al. (2002, Anal. Chem., 74, 4725-4733). The column was prepared by a laser puller (Sutter Instruments Co., CA) using a fused silica capillary (150 µm i.d. × 375 µm o.d.) and filled with reverse phase slurry (Develosil C30-UG-3, particle size 3 µm, Nomura Chemical, Japan) up to 50 mm in length.

### (A) Liquid chromatography (LC) conditions

- Equipment used: RENCON-P (LC Assist)
- Eluent A: 10 mM triethylamine acetic acid (Glen Research)
- Eluent B: methanol (Wako Pure Chemical Industries, Ltd.)
- Column: Develosil C30-UG-3, 150 µm × 50 mmL, particle size 3 µm (Nomura Chemical)
- Trap column Monocap for Trap, 200 µm × 45 µm (GL Science)
- Column temperature: 25°C
- Flow rate: 100 nL/min
- Time continuous concentration gradient (time schedule gradient) 0 min: 10% B - 40 min: 40% B

### (B) MS condition

- Equipment used: LTQ Orbitap XL (Thermo scientific)
- Mass range: 500 - 1950 Da
- Scan mode: FT normal
- Ionization method: ESI-negative
- Scan speed: normal
- Analysis software: Excalibur Qual Browser

### (C) MSMS condition

- Equipment used: same as above
- Mass range: normal
- Scan mode: normal
- Scan speed: enhanced
- Analysis software: Excalibur Qual Browser

The human genome database containing the mitochondrial genome was analyzed with the spectrum obtained from the in-gel digested RNA as a query using the search engine Ariadne (Taoka, et al., 2010, Anal. Chem. 82 (18): 7795-7803; Nakayama, et al., 2009, Nucleic Acids Res. 37: e47).

### (Results)

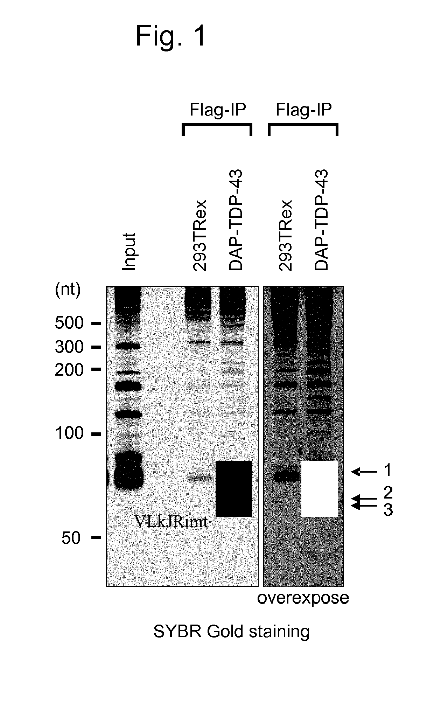

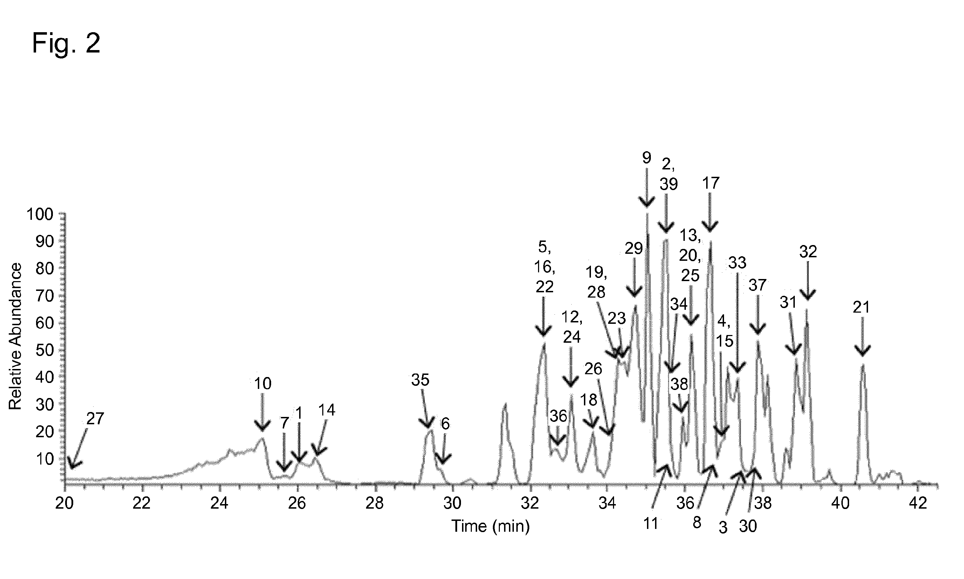

Results are shown in Figures 1 and 2. Figure 1 shows results from PAGE. The three bands (bands 1 to 3 shown with arrows) of about 70 nucleotides within the black frame represent the RNA specific for DAP-TDP-43. Figure 2 shows a nano LC-MS/MS chromatogram of band 1. The chromatogram shows a series of product ions resulting from the RNA fragment in-gel digested in the collision-induced dissociation-MS/MS analysis. The product ions included large quantities of c/y and a/w ions and a trace amount of derivatives (hydrated or dehydrated ions and ions which lost their nucleotide bases).

Each band was identified as follows from the results of the Ariadne analysis.
Band 1: mt-tRNA^{Asn} gene, mt-tRNA^{Gln} gene and a sequence on chromosome 1 that is completely identical with mt-tRNA^{Asn} gene
Band 2: mt-tRNA^{Glu} and mt-tRNA^{Pro} genes
Band 3: mt-tRNA^{Pro} gene

Thus, four types of mt-tRNAs (mt-tRNA^{Asn}, mt-tRNA^{Gln}, mt-tRNA^{Glu} and mt-tRNA^{Pro}) have been confirmed to bind to TDP-43.

### <Example 2: Identification of mt-tRNA bound to TDP-43 by Northern blotting>

### (Object)

Results from Example 1 are confirmed by Northern blotting.

### (Method)

The RNA obtained in "(4) Immunoprecipitation and collection" of Example 1 was separated by 8% polyacrylamide gel electrophoresis involving 8 M urea and 0.5 × TBE buffer, and transcribed onto a nylon membrane (Hybond-N+: Bio-Rad) using Trans-blot SD Cell (Bio-Rad) under conditions of 5 V, 60 minutes. The RNA was crosslinked with the membrane by UV, and then hybridized in a pre-hybridization solution (0.6 M NaCl, 120 mM Tris-HCl (pH 8.0), 4.8 mM EDTA, 0.1% SDS, 1 × Denhardt's Solution) at 42°C overnight using biotinylated probes. The membrane was washed 3 times for 30 minutes using a wash solution (90 mM Tris-HCl (pH 8.0), 0.45 M NaCl, 3.6 mM EDTA, 0.1% SDS) at 25°C, and the RNA was detected using a Chemiluminescent Nucleic Acid Detection Module kit (Thermo Fisher Scientific) according to the attached instructions. Each oligonucleotide probe used for detection has the following sequences complementary to the nucleotide sequence specific for respective mt-tRNA etc. That is, the mt-tRNA^{Asn} probe has a sequence (SEQ ID NO: 10) complementary to positions 7 to 34 of the nucleotide sequence of the mt-tRNA^{Asn} gene, the mt-tRNA^{Gln} probe has positions 36 to 65 (SEQ ID NO: 11) of the nucleotide sequence of the mt-tRNA^{Gln} gene, the mt-tRNA^{Pro} probe has positions 28 to 57 (SEQ ID NO: 12) of the nucleotide sequence of the mt-tRNA^{Pro} gene, the mt-tRNA^{Glu} probe has positions 33 to 62 (SEQ ID NO: 13) of the nucleotide sequence of the mt-tRNA^{Glu} gene, and as controls, the mt-tRNA^{Leu(UUR)} probe has positions 1 to 30 (SEQ ID NO: 14) of the nucleotide sequence of the mt-tRNA^{Leu(UUR)} gene, and the tRNA^{Met} probe has positions 46 to 72 (SEQ ID NO: 15) of the nucleotide sequence of the tRNA^{Met} gene. Each probe was labelled at 3' end using the Biotin 3' End DNA Labeling kit (Thermo Fisher Scientific). The signal intensity of each RNA band was quantitated using the LAS4000 image analyzer and MultiGauge software (Fujifilm). Each value represents an average for at least 3 independent experiments (± 1 SD).

### (Results)

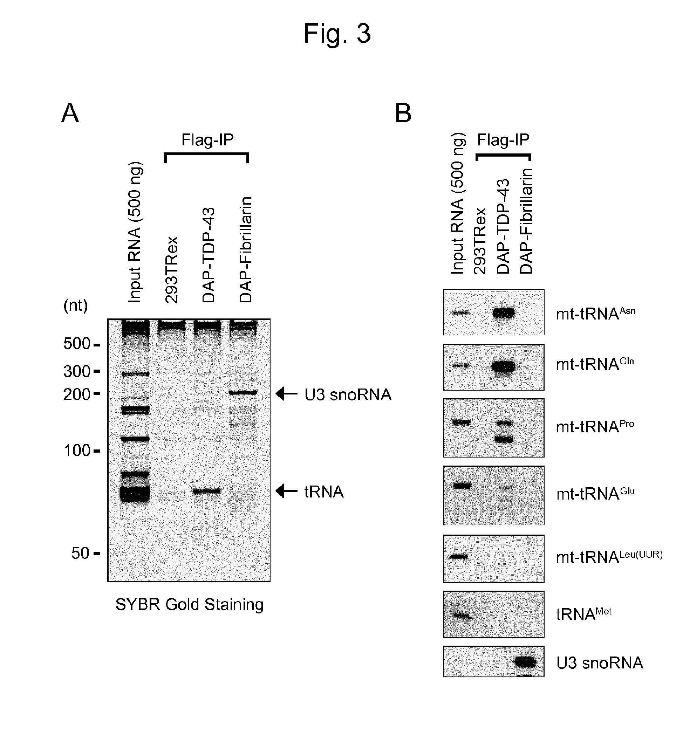

Results are shown in Figure 3. mt-tRNA^{Asn} and mt-tRNA^{Gln} could be detected as a single band from the RNA co-immunoprecipitated with the DAP-TDP-43 obtained in "(4) Immunoprecipitation and collection" of Example 1 (A, B). In contrast, the other mt-tRNAs as controls, mt-tRNA^{Leu(UUR)} and tRNA^{Met}, which is a cytoplasm-tRNA, could not be detected (A). Also, mt-tRNA^{Pro} and mt-tRNA^{Glu} could be detected from the RNA co-immunoprecipitated with DAP-TDP-43 (B). This has demonstrated that mt-tRNA^{Asn}, mt-tRNA^{Gln}, mt-tRNA^{Pro} and mt-tRNA^{Glu} bind to TDP-43.

### <Example 3: Physiologic function in binding between mt-tRNA and TDP-43>

### (Object)

Physiologic function in binding between mt-tRNA and TDP-43 is clarified.

### (a) Suppression of the abundance of TDP-43 in cells by TDP-43 siRNA

### (Method)

The abundance of the mt-tRNA when TDP-43 was silenced using small interference RNA to TDP-43 (TDP-43 siRNA) was examined.

siRNA consisting of the oligonucleotides set forth in SEQ ID NO: 16 and SEQ ID NO: 17 was constructed as TDP-43 siRNA. Also, siRNA (hereinafter referred to as "Cont" in this example) consisting of the oligonucleotides set forth in SEQ ID NO: 18 and SEQ ID NO: 19 was constructed as a negative control for TDP-43 siRNA (stealth RNA: lifetechnologies). HeLa cells were then cultured in 35 mm Petri dish and, at about 70% confluence, transfected with 50 pmol of siRNA targeted for TDP-43 using 2.5 µL of Lipofectamine 2000 according to the attached instructions.

At 0, 24, 48, 72 and 96 hours after transfection, a part of the culture medium was collected and subjected to a cell proliferation assay. The cell proliferation assay was calculated by visually counting the number of cells in a Burker-Turk chamber (Hirschmann; Laborgerate Hilgenberg).

In addition, after 24, 48, 72 and 96 hours, a cell extract was prepared by the same method as that described above in "(3) Preparation of cell extract" of Example 1. The amount of TDP-43 protein in the cell extract was detected by Western blot analysis to confirm the silencing of the target gene by siRNA. Glyceraldehyde-3-phosphate dehydrogenase (hereinafter "GAPDH") was used as an internal control. An anti-TDP-43 antibody was used to confirm the silencing of TDP-43 and an anti-GAPDH antibody (ambion) was used to detect

### GAPDH.

Furthermore, to obtain the total RNA in the cells when TDP-43 was silenced, the cells washed in PBS were dissolved in RNAgent Denaturing Solution (Promega), the aqueous layer containing the RNA was separated by acid phenol chloroform extraction, and the RNA was collected by isopropanol precipitation and washed in 75% ethanol. This, as the total cellular RNA, was subjected to co-immunoprecipitation-Northern blot analysis by the same method as that described above in "(5) Urea denaturation PAGE" of Example 1.

The oligonucleotide probes used for detection included, in addition to the probes described above in Example 2, a U1 snRNA probe (positions 1 to 24 (SEQ ID NO: 20) of the nucleotide sequence of the U1 snRNA gene) and a 5.8S rRNA probe (positions 123 to 146 (SEQ ID NO: 21) of the nucleotide sequence of the 5.8S rRNA gene).

### (Results)

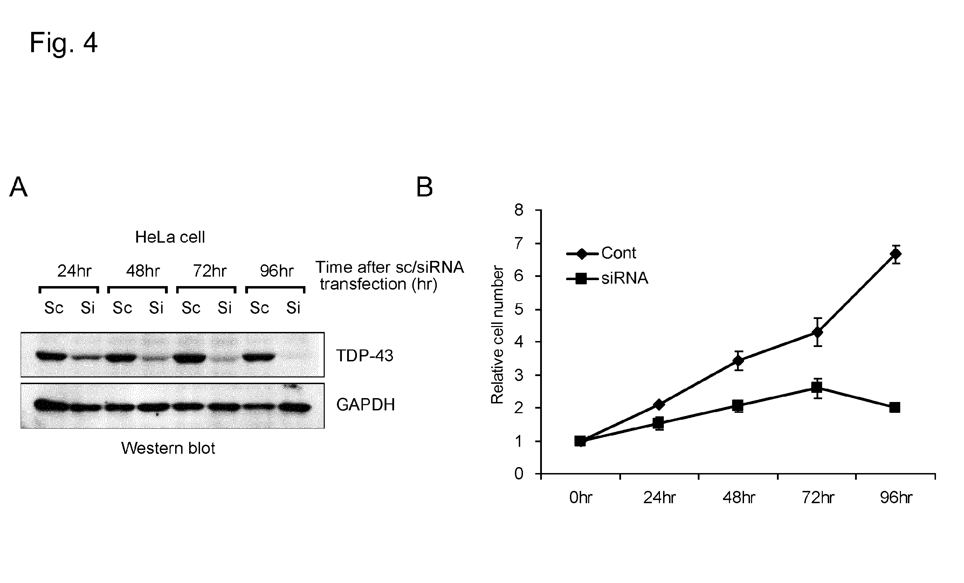

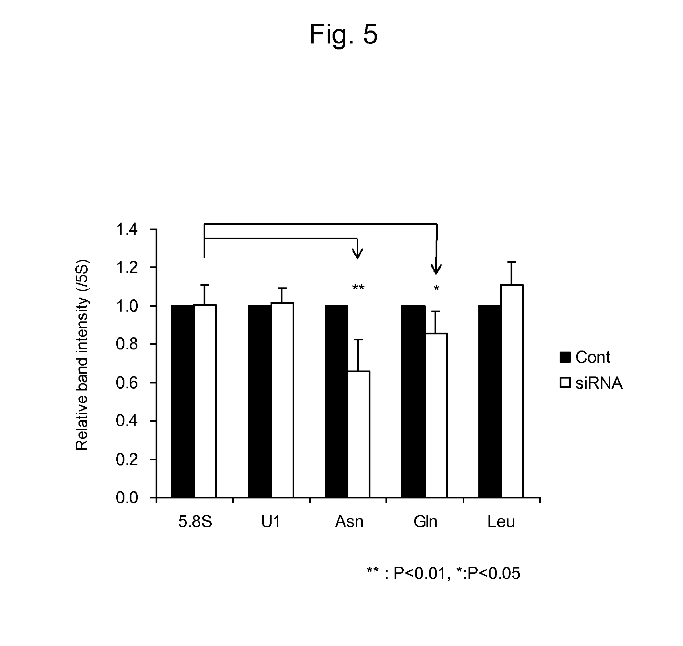

Results are shown in Figure 4 and Figure 5. Figure 4A shows a result from the detection of TDP-43 protein mass in HeLa cells transfected with TDP-43-siRNA or Cont. When the cells were transfected with TDP-43-siRNA, the expression level of the TDP-43 protein decreased by about 90% at 24 or more hours after transfection in comparison with Cont. These results confirmed the silencing of TDP-43 by TDP-43-siRNA. In addition, Figure 4B shows a cell growth rate in HeLa cells transfected with TDP-43-siRNA or Cont. In the figure, a relative value is shown relative to the assumed cell number of 1 at transfection. According to the figure, growth was clearly suppressed in the HeLa cells treated with TDP-43-siRNA as compared to Cont. These results have demonstrated that the suppression of TDP-43-siRNA expression suppresses cell growth.

Figure 5 shows a change in the abundance of various types of RNAs in HeLa cells upon treatment with TDP-43-siRNA. In the figure, 5.8S, U1, Asn, Gln and Leu represent 5.8S rRNA, U1 snRNA, mt-tRNA^{Asn}, mt-tRNA^{Gln} and mt-tRNA^{Leu(UUR)}, respectively. The HeLa cells transfected with TDP-43-siRNA had significantly decreased abundance of mt-tRNA^{Asn} and mt-tRNA^{Gln} (p<0.01, p<0.05, respectively) in comparison with the HeLa cells transfected with Cont. On the other hand, such a decrease could not be detected in 5.8S RNA, U1 snRNA and mt-tRNA^{Leu(UUR)}.

### (b) Overexpression of DAP-TDP-43 with doxycycline

### (Method)

100 ng/mL of doxycycline was added to the DAP-TDP-43 expressing strain prepared in "(2) Construction of the doxycycline-induced cell line" of Example 1 to overexpress DAP-TDP-43 in the cells. At 0, 8, 24, and 48 hours after adding doxycycline, a part of the culture medium was collected to prepare a cell extract according to the same method as that described in "(3) Preparation of cell extract" of Example 1, followed by Western blot analysis. In addition, total RNA was extracted according to the same methods as those described in "(4) Immunoprecipitation and RNA collection" and "(5) Urea denaturation PAGE" of Example 1, followed by Northern blot analysis. The probes described above were used as oligonucleotide probes for detection. As an internal control, GADPH was used for western blot analysis and 5.8S rRNA for Northern blot analysis.

### (Results)

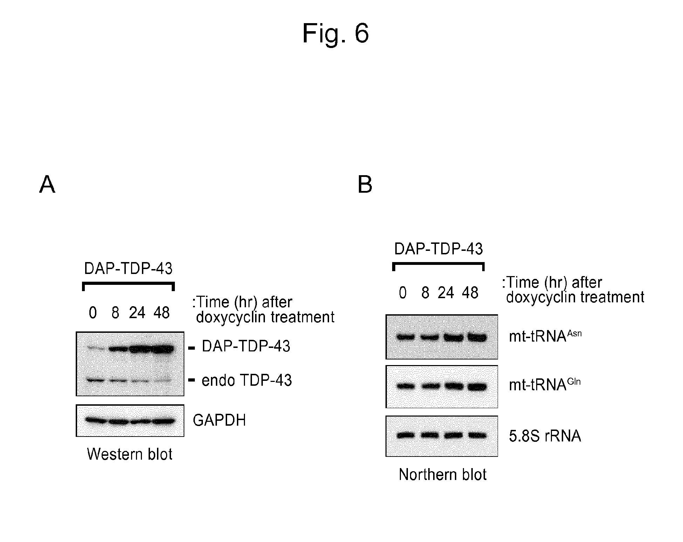

Results are shown in Figure 6. Figure 6A shows a result of the western blot analysis for DAP-TDP-43 and endogenous TDP-43 when DAP-TDP-43 was overexpressed. Figure 6B shows a result of Northern blot analysis for the mRNA with mt-tRNA^{Asn} and mt-tRNA^{Gln} found to bind to TDP-43.

When DAP-TDP-43 was overexpressed, the abundance of mt-tRNA^{Asn} and mt-tRNA^{Gln} in cells increased in proportion to its expression level.

Results from (a) and (b) above have demonstrated that TDP-43 is involved at least in the biosynthesis or metabolism of mt-tRNA^{Asn} and mt-tRNA^{Gln}.

### <Example 4: Control of abundance of mt-tRNA^{Asn} and mt-tRNA^{Gln} in cells by TDP-43>

### (Object)

To confirm the abundance of mt-tRNA^{Asn} and mt-tRNA^{Gln} in cells is controlled by TDP-43.

### (Method)

(A) Degradation of mt-tRNA^{Asn} or mt-tRNA^{Gln} over time was measured using DAP-TDP-43 expressing strains, when DAP-TDP-43 was overexpressed. After adding doxycycline, the culture medium of DAP-TDP-43 expressing strains was cultured for 24 hours to overexpress DAP-TDP-43, ethidium bromide (EtBr) was added to inhibit mt-tRNA synthesis, and a part of the culture medium was collected at 6, 12, and 24 hours after the addition of EtBr at 0 hour, the total cellular RNA was prepared by the same method as that described above in Example 3, and mt-tRNA was detected according to the same method as the Northern blotting described above in Example 2. The 5S rRNA stained with SYBR Gold was used for an internal control.
(B) A change in the amount of binding of mt-tRNA^{Asn} and mt-tRNA^{Gln} associated with an increase in the expression level of TDP-43 was observed. The amount of binding between DAP-TDP-43 and mt-tRNA^{Asn} or mt-tRNA^{Gln} over time when EtBr was not added after DAP-TDP-43 overexpression was examined. Doxycycline was added to the culture medium of the DAP-TDP-43 expressing strain, and a part of the culture medium was collected after 4, 8, and 24 hours and subjected for confirmation to co-immunoprecipitation-Northern blot analysis according to the methods as those described above in "(4) Immunoprecipitation and RNA collection" and "(5) Urea denaturation PAGE" of Example 1.

### (Results)

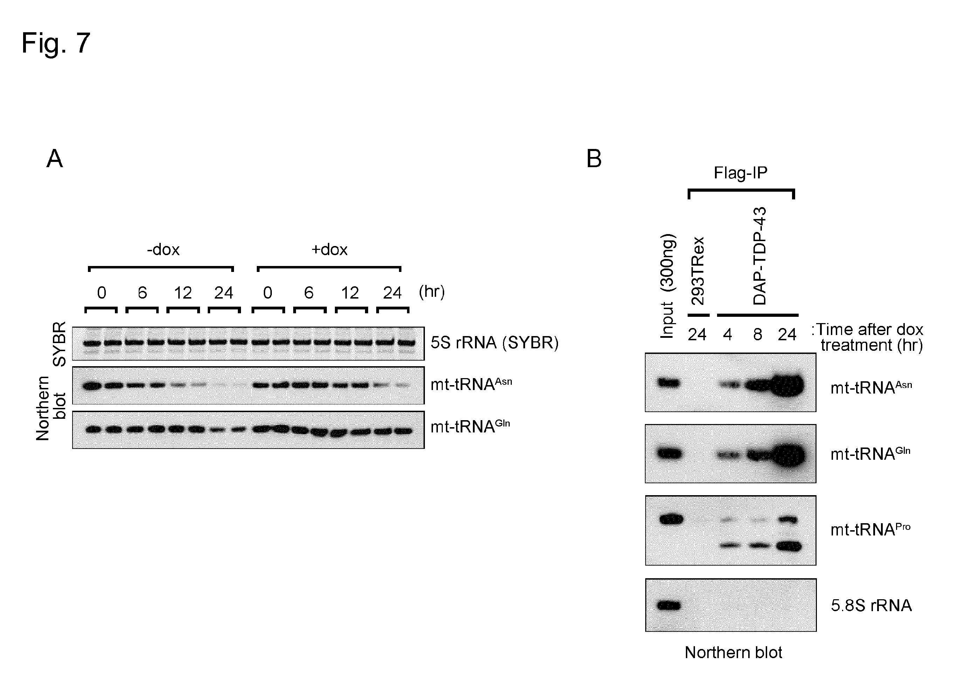

Results are shown in Figure 7. Figure 7A shows the degradation of mt-tRNA^{Asn} or mt-tRNA^{Gln} over time, and Figure 7B shows results of their co-immunoprecipitation with DAP-TDP-43.
(A) It was found that the degradation rate of mt-tRNA^{Gln} decreased gradually and the degradation rate of mt-tRNA^{Asn} slowed down dramatically in the strain in which TDP-43 was induced with doxycycline (+dox) as compared to the strain without induction (-dox). These results show that TDP-43 is involved in the intracellular stability of mt-tRNA^{Asn} and mt-tRNA^{Gln}.
(B) It was found that mt-tRNA^{Asn} and mt-tRNA^{Gln} bound to overexpressed TDP-43 increased, and the abundance of these mt-tRNAs in cells increased correlatively. In a mutant TDP-43 in ALS, its intracellular stability has been reported to increase in comparison with the wild type TDP-43 (Ling, et al., 2010, Proc. Natl. Acad. Sci. U.S.A. 2107: 13318-13323). Thus, results suggest that the increase in the abundance of TDP-43 in cells is accompanied by the increase in the abundance of mt-tRNA^{Asn} and mt-tRNA^{Gln} in cells.

Results of (A) and (B) described above have demonstrated that TDP-43, as a scaffold protein, controls the intracellular stabilization of mt-tRNA^{Asn} or mt-tRNA^{Gln} by means of binding.

### <Example 5: Identification of the mt-tRNA binding site in TDP-43>

### (Object)

Identifying to which domain of TDP-43 mt-tRNA binds.

### (Method)

### (1) Preparation of the DAP-tagged domain-deficient TDP-43 expression vector

Expression vectors for the domain-deficient type of TDP-43 deficient in RRM1 domain, RRM2 domain, GR domain or 315 domain (designated dRRM1, dRRM2, dGR and d315, respectively, see Figure 8A) were prepared.

Fragments of each domain-deficient mutant TDP-43 were amplified from the DAP-TDP-43 pcDNA5/FRT/TO constructed in Example 1 by PCR using KOD plus DNA polymerase and prepared. Primer sets used were DNAs with the nucleotide sequences set forth in SEQ ID NOs: 22 and 23 for dRRM1, the nucleotide sequences set forth in SEQ ID NOs: 24 and 25 for dRRM2, the nucleotide sequences set forth in SEQ ID NOs: 26 and 27 for dGR and the nucleotide sequences set forth in SEQ ID NOs: 28 and 29 for d315. For dRRM1, dRRM2 and dGR, the amplified DNA fragments were cut with ClaI, self-ligated, and after confirmation of mutation by sequencing, cut with HindIII/XhoI and inserted into the HindIII/XhoI site of pcDNA5/FRT/TO. For d315, the amplified DNA fragment was cut with BamHI/XhoI and inserted into the vector site created by removing the TDP-43 site at the BamHI/XhoI site from DAP-TDP-43 pcDNA5/FRT/TO. Thus, expression vectors, DAP-dRRM1, DAP-dRRM2, DAP-dGR and DAP-d315, were obtained.

(2) To detect the mt-tRNA^{Asn} and mt-tRNA^{Gln} bound to TDP-43, a cell line was constructed according to "(2) Construction of a doxycycline-induced cell line" described in Example 1, and then subjected to induction of expression with doxycycline, cells were collected after 24 hours, and mt-tRNA^{Asn} and mt-tRNA^{Gln} were detected according to the methods described in "(3) Preparation of cell extract," "(4) Immunoprecipitation and RNA collection" and "(5) Urea denaturation PAGE."

### (Results)

Results are shown in Figure 8. Figure 8A is a conceptual diagram showing the structure of wild type TDP-43 (WT) and each domain-deficient type of TDP-43. Figure 8B shows results of Western blot indicating proteins in the cells expressed by doxycycline induction in strains expressing wild type TDP-43 and each domain-deficient type of TDP-43. Figure 8C shows results of Northern blot indicating mt-tRNA^{Asn} and mt-tRNA^{Gln} bound to wild type TDP-43 and each domain-deficient type of TDP-43.

Figure 8C indicates that binding of mt-tRNA^{Asn} and mt-tRNA^{Gln} was maintained in dRRM2, dGR and d315, while binding of mt-tRNA^{Asn} and mt-tRNA^{Gln} was not observed in dRRM1. These results have demonstrated that these mt-tRNAs bind to the RRM1 domain in TDP-43.

### <Example 6: Cytotoxicity>

### (Object)

Cytotoxicity induced by the domain-deficient type of TDP-43 is examined.

### (Method)

Cytotoxicity in human cells was examined using wild type and the overexpressing line for the domain-deficient type of TDP-43 described above, prepared in Example 5. Doxycycline was added to each cell culture medium of DAP-TDP43 (WT), DAP-dRRM1, DAP-dRRM2, DAP-dGR and DAP-d315, and the number of cells after 48 hours was calculated by visually counting the number of cells in a Burker-Turk chamber (Hirschmann; Laborgerate Hilgenberg).

### (Results)

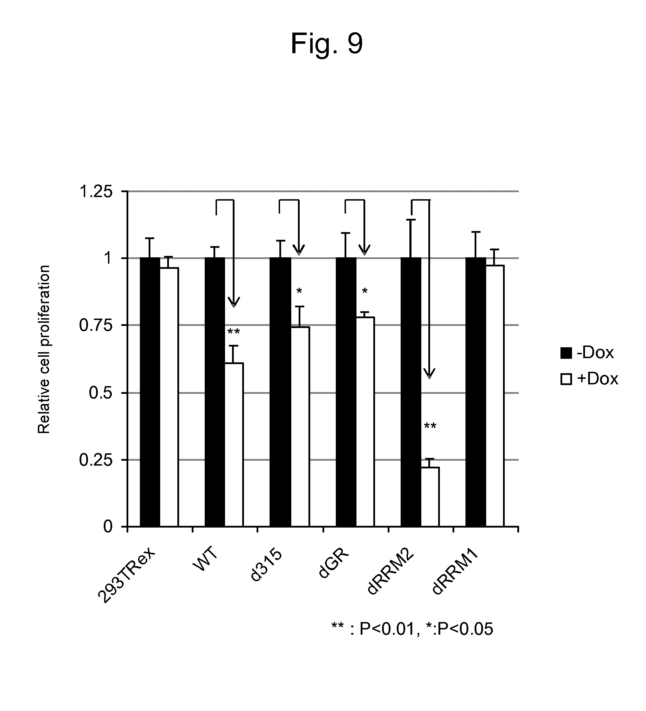

Results are shown in Figure 9. In the figure, the cell growth rate is expressed as relative values compared to a value of 1 representing the number of cells when cultured under the same conditions except that doxycycline was not added. In the figure, * represents P<0.05, and ** P<0.01.

Cytotoxicity of the similar level to that of wild type DAP-TDP43 was observed in DAP-dGR and DAP-d315 with overexpression (+dox). In addition, cytotoxicity stronger than that of wild type DAP-TDP43 was observed in DAP-dRRM2. On the other hand, the DAP-dRRM1 which is deficient in RRM1, a binding domain for mt-tRNA^{Asn} and mt-tRNA^{Gln}, hardly exhibited cytotoxicity after overexpression. These results suggest that binding to RRM1 of mt-tRNA^{Asn} and mt-tRNA^{Gln} is involved in the cytotoxicity of TDP-43. In other words, cytotoxicity can be suppressed by inhibiting binding of mt-tRNA to TDP-43.

### <Example 7: Correlation between the cytotoxicity of mutant TDP-43 and binding of mt-tRNA^{Asn} and mt-tRNA^{Gln}>

### (Object)

Correlation between TDP-43 and mt-tRNA in ALS is examined.

### (Method)

Cell lines (DAP-D169G, DAP-G298S, DAP-R361S) involving the induction of expression of mutant TDP-43 (D169G, G298S, R361S; see Figure 10A) into which mutations identified in ALS had been introduced were created. D169G, G298S and R361S were constructed essentially according to the method described in Example 5. Primer sets used are DNA sequences with the nucleotide sequences set forth in SEQ ID NOs: 30 and 31 for D169G, the nucleotide sequences set forth in SEQ ID NOs: 32 and 33 for G298S, and the nucleotide sequences set forth in SEQ ID NOs: 34 and 35 for R361S. In addition, the amplified DNA fragment was cut with DpnI to remove template DNA, and transformed into *Escherichia coli,* resulting in a vector with a sequence of interest. After confirmation of mutation by sequencing, this was cut with HindIII/XhoI and inserted into the HindIII/XhoI site of pcDNA5/FRT/TO. Viability of the mutant strain at 48 hours after doxycycline induction was measured.

In addition, the ability of mt-tRNA^{Asn} and mt-tRNA^{Gln} to bind mutant TDP-43 in these mutant TDP-43 expressing strains was confirmed. The detection method followed the method described in Example 2.

### (Results)

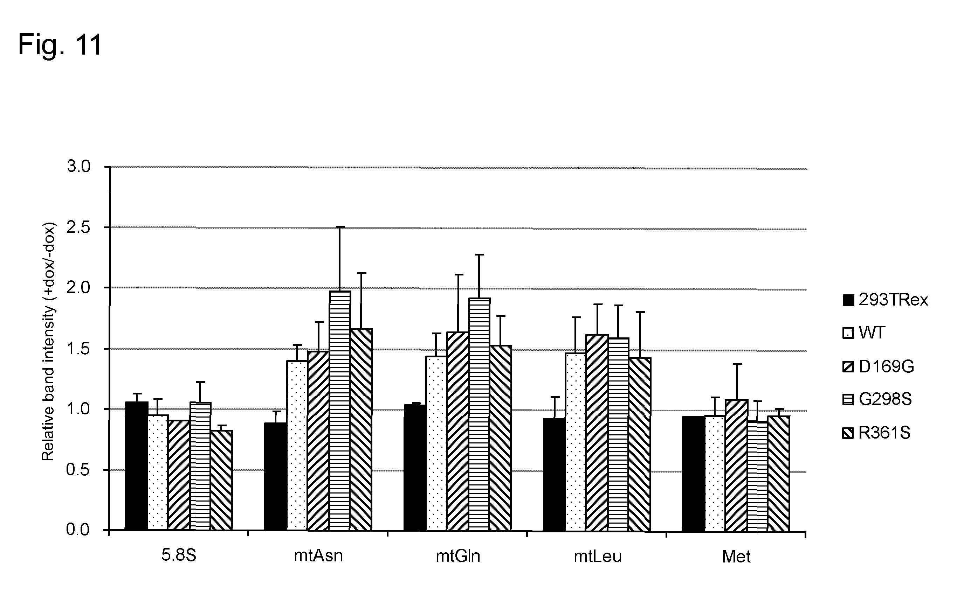

Results are shown in Figure 10 and Figure 11.

Figure 10A is a conceptual diagram showing wild type TDP-43 and each mutant TDP-43 used for the experiment. Figure 10B is a graph indicating the viability in wild type and each mutant TDP-43 expressing strain. +dox and -dox represent the cells from the culture media with added doxycycline and the cells without addition, respectively. Figure 10B has shown that all the cells expressing mutant TDP-43 having ALS-like mutation have lower cell viability and stronger cytotoxicity in comparison with the cells expressing wild type TDP-43.

Figure 11 is a graph indicating the abundance of each mt-tRNA in cells when wild type and each mutant TDP-43 were overexpressed. In the figure, the abundance is presented in relative intensity of the detection band when doxycycline was added as compared to a value of 1 when doxycycline was not added. The graph shows that the abundance of mt-tRNA^{Asn} and mt-tRNA^{Gln} in cells increased due to overexpression of mutant TDP-43 as in wild type TDP-43. From the report that the mutant TDP-43 observed in ALS has increased expression levels in cells due to stabilization, these results show that, in ALS-like mutant TDP-43, the abundance in cells increases, thereby mt-tRNA^{Asn} and mt-tRNA^{Gln} are stabilized, and the abundance of these in cells increases. As a result, cytotoxicity increases in the cells by also inducing the effect described below in Examples 10 and 11, leading to cell death.

### <Example 8: Novel TDP-43 binding protein>

### (Object)

TDP-43 binding proteins include conventionally reported FUS/TLS and ataxin2 (Kwiatkowski, et al., 2009, Science, 323: 1205-1208, Vance, et al., 2009, Science 323, 1208-1211, Elden, et al., 2010, Nature. 466: 1069-1075). Thus, other proteins bound to TDP-43 are analyzed.

### (1) Isolation and identification of TDP-43 binding protein

### (Method)

Using the DAP-TDP-43 having two different tags, proteins bound to the DAP-TDP-43 were collected by a two-step purification method involving performing a pull-down process twice using the different tags, separated by SDS-PAGE, and digested in gel with protease. These methods followed the methods described in Fujiyama-Nakamura et al. (Mol Cell Proteomics, 2009, 8, 1552-1565) and Hayano et al. (J. Proteome Res. 7: 4183-4190). This was followed by LC-MS/MS analysis and shotgun analysis by use of the mascot search engine. LC-MS/MS was carried out under the following conditions.

### (A) Liquid chromatography (LC) conditions

- Equipment used: RENCON (LC Assist)
- Eluent A: Aqueous 0.1% formic acid (Milli-Q water)
- Eluent B: Acetonitrile containing 0.1% formic acid (Wako Pure Chemical Industries, Ltd.)
- Column: 45 mm × 0.150 mm i.d. particle size 3 µm (Kanto Chemical)
- Column temperature: 25°C
- Flow rate: 100 nL/min
- Time continuous concentration gradient (time schedule gradient) 0% B 0 min: 0% - 60 min: 35% B
- Trap column Monocap for Trap 200 µm × 45 µm (GL Science)

### (B) MS conditions

- Equipment used: LTQ-Orbitrap XL (Thermo Fisher Scientific)
- Mass range: 450-1500 Da
- Scan mode: FT normal
- Ionization method: ESI-Positive
- Scan speed: normal
- Analysis software: Excalibur Qual Browser

### (C) MSMS conditions

- Equipment used: Same as above
- Mass range: normal
- Scan mode: Ion-trap normal
- Scan speed: normal
- Analysis software: Excalibur Qual Browser

### (Results)

In addition to previously reported TDP-45 binding protein candidates; hnRNP, interleukin-enhancer binding factor, insulin-like growth factor 2 mRNA binding protein, Matrin-3 and DHX9 (Ling et al., 2010, Proc. Natl. Acad. Sci. U.S.A. 2107: 13318-13323), Aralar 1 (SLC25A12), Aralar 2 (SLC25A13; citrin), Musashi 2 and GDH were isolated as novel TDP-43 binding protein candidates (data not shown). Aralar 1 and Aralar 2 are mitochondrial aspartate-glutamate transporter proteins, and involved in the exchange between aspartic acid and glutamic acid via mitochondrial inner membranes (Gellerich FN, et al., 2009, PLoS One. 9; 4 (12): e8181). Musashi 2 is a protein that has an RNA binding domain, and is believed to be necessary for the growth of myeloid leukemia cells (Jaenisch & Daley, Nat Med. 2010 16 (8): 903-908.) and the maintenance and growth of neurologic stem cells (Seigel GM, et al., Mol Vis. 2007 Jun 8, 13: 823-832). GDH is an enzyme that converts glutamic acid into alpha-ketoglutaric acid (oxoglutaric acid), which is an intermediate product of the tricarboxylic acid cycle, and it is known to be involved in energy metabolism and ammonia detoxification (http://www.uniprot.org/uniprot/P00367).

### (2) Effects on binding of novel TDP-43 binding proteins due to overexpression of TDP-43

### (Method)

Doxycycline was added to the culture media of DAP-TDP-43 cell line to induce the expression of DAP-TDP-43, and cells were collected after 4, 8 and 24 hours and subjected to immunoprecipitation with DAP-TDP-43. The method used followed Examples 1 and 2 described above. An anti-Aralar 1 antibody (Santacruz), anti-Aralar 2 antibody (abeam), anti-Musashi 2 antibody (abeam) and anti-GDH antibody (abeam) were used for the detection of Aralar 1, Aralar 2, Musashi 2 and GDH, respectively. In addition, β-actin was used for an internal control. The β-actin used was anti-β-actin antibody (Santacruz).

### (Results)

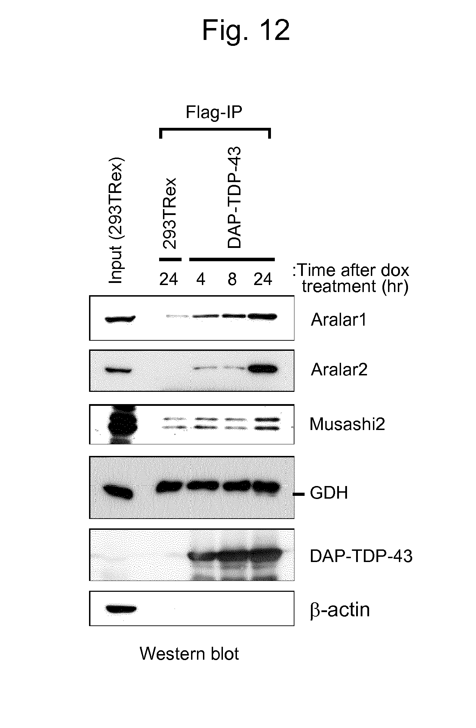

Results are shown in Figure 12. The abundance of DAP-TDP-43 in cells increased with time after the induction of expression. This was accompanied by the increase in the amount of Aralar 1, Aralar 2, Musashi 2 and GDH that co-immunoprecipitated with DAP-TDP-43. Thus, TDP-43 was found to bind to mt-tRNA such as mt-tRNA^{Asn} as well as the proteins of Aralar 1, Aralar 2, Musashi 2 and GDH.

### (3) Binding affinity with Aralar 1, Aralar 2 and mt-tRNA

### (Method)

Co-immunoprecipitation that involved interchange of immunoprecipitating proteins was performed to confirm binding of Aralar 1 and Aralar 2. In addition, detection using mt-tRNA^{Asn} probe was performed after pull-down with the antibody described above to confirm binding between Aralar 1 or Aralar 2 and mt-tRNA. The co-immunoprecipitation method essentially followed the methods described in Sambrook, J. et al. (Molecular Cloning: A Laboratory Manual Third Ed., 2001, Cold Spring Harbor Laboratory Press, Cold Spring Harbor, New York) and Examples 1 and 2 above.

### (Results)

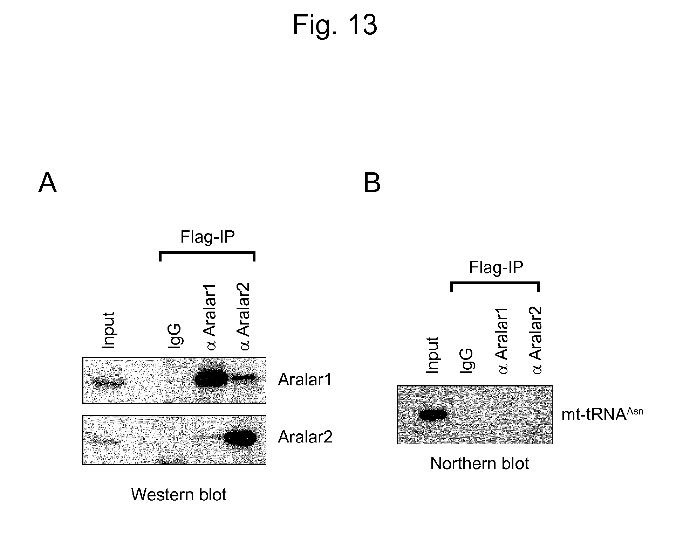

Results are shown in Figure 13. Figure 13A shows results of immunoprecipitation involving the interchange of Aralar 1 and Aralar 2. In addition, Figure 13B shows results of mt-tRNA^{Asn} that co-immunoprecipitates with Aralar 1 or Aralar 2. Figure 13B has demonstrated that Aralar 1 and Aralar 2 not only bind to TDP-43, but they also bind to each other *in vivo.* On the other hand, mt-tRNA^{Asn} was proved to bind to TDP-43 but not to Aralar 1 or Aralar 2, because it did not co-immunoprecipitate with Aralar 1 or Aralar 2. These results show that a TDP-43 complex containing mt-tRNA is distinct from a TDP-43 complex containing Aralar 1 and Aralar 2.

### (4) Binding affinity with Musashi 2 and mt-tRNA

### (Method)

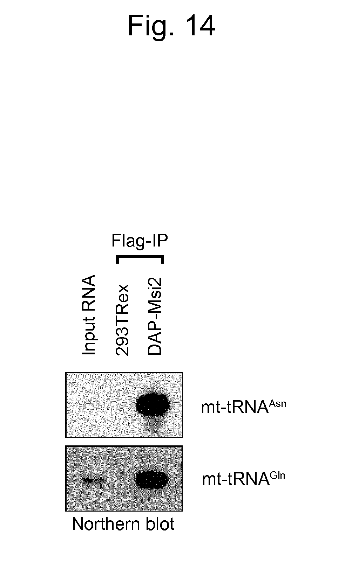

A cell line involving the induction of expression of DAP-Musashi2 (described in Figure 14 as DAP-Msi2) was constructed to confirm binding between Musashi2 and mt-tRNA. The method essentially followed the method described in Example 5. After pull-down with DAP-Musashi2, mt-tRNA^{Asn} and mt-tRNA^{Gln} bound to DAP-Musashi2 were detected using respective probes. The co-immunoprecipitation method essentially followed the methods described in Sambrook, J. et al. (Molecular Cloning: A Laboratory Manual Third Ed., 2001, Cold Spring Harbor Laboratory Press, Cold Spring Harbor, New York) and Examples 1 and 2 above.

### (Results)

Results are shown in Figure 14. Upon pull-down with DAP-Musashi-2 (DAP-Msi2), co-immunoprecipitation of mt-tRNA^{Asn} and mt-tRNA^{Gln} was confirmed.

### <Example 9: Identification of the TDP-43 binding site of TDP-43 binding proteins>

### (Object)

Which domain of TDP-43 Aralar 1 and Aralar 2, TDP-43-binding proteins identified in Example 8, bind to is examined.

### (Method)

Using expression strains, DAP-dRRM1, DAP-dRRM2, DAP-dGR and DAP-d315 prepared in Example 5, the co-immunoprecipitation method essentially followed the methods described in Sambrook, J. et al. (Molecular Cloning: A Laboratory Manual Third Ed., 2001, Cold Spring Harbor Laboratory Press, Cold Spring Harbor, New York) and Examples 1 and 2 above. Protein consumption by the wild type and each domain-deficient type of TDP-43 was determined by detecting biotin using a Chemiluminescent Nucleic Acid Detection Module kit (Thermo Fisher Scientific).

### (Results)

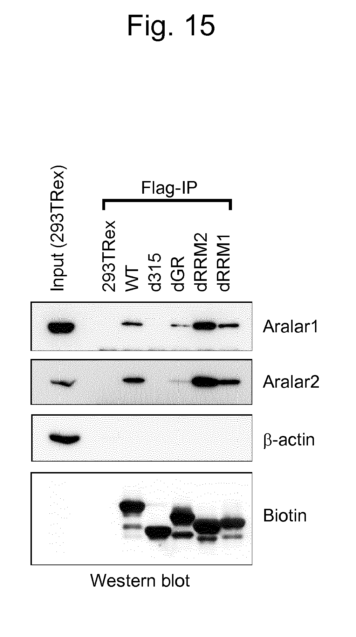

Results are shown in Figure 15. The figure shows that both Aralar 1 and Aralar 2 could be detected in the RRM 1 and RRM 2 domain-deficient mutants but were poor or undetectable in the GR domain-deficient mutant and the 315 domain-deficient mutant. These results have demonstrated that Aralar 1 and Aralar 2 bind to the GR and 315 domains on the C-terminal side of TDP-43.

### <Example 10: Relationship between TDP-43 and ATP>

### (Object)

Relationship between TDP-43 and ATP is examined.

### (Method)

ATP assays were performed to measure the amount of ATP in cells when TDP-43 was silenced using TDP-43 siRNA prepared in Example 3 and when DAP-TDP-43 was overexpressed using DAP-TDP-43 cells. The amount of ATP was measured using Celltiterglo (Promega) according to the attached instructions. As a control for the amount of cells, the number of cells was counted according to the method described above in Example 6. The amount of ATP in the DAP-TDP-43 overexpressing cells was measured on cells at 8, 24, and 48 hours after the induction of expression (at 0 hour) with added doxycycline.

### (Results)

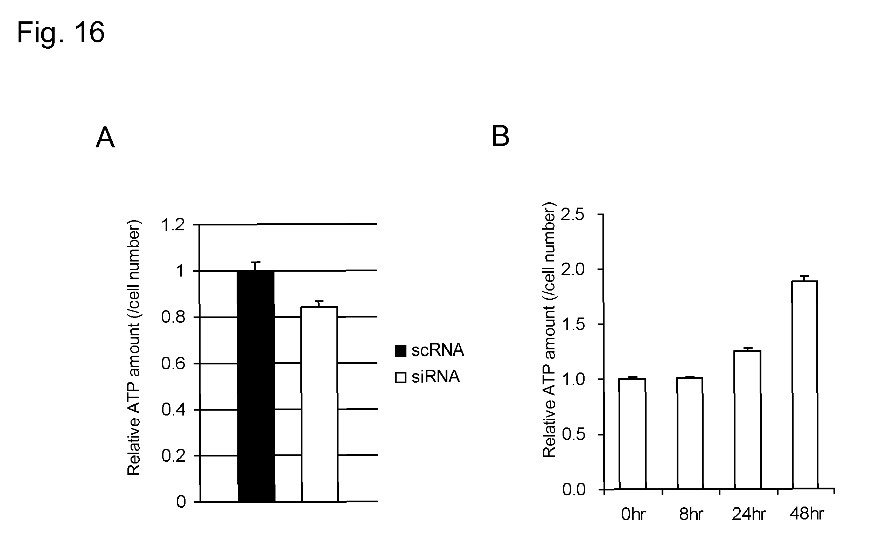

Results are shown in Figure 16. When TDP-43 was suppressed, the abundance of ATP in TDP-43 cells decreased to 82% of that in Cont cells (Figure 16A). When DAP-TDP-43 was overexpressed, the abundance of ATP per cell increased by 1.8 times at 48 hours after doxycycline induction (Figure 16B). These results have shown that TDP-43 is involved in the control of oxidative phosphorylation reaction.

### <Example 11: Abundance of mt-tRNA and ATP in cells in domain-deficient type of TDP-43>

### (Object)

Abundance of mt-tRNA (mt-tRNA^{Asn} and mt-tRNA^{Gln}) or ATP in cells in the domain-deficient type of TDP-43 is examined.

### (Method)

Strains expressing the domain-deficient type of TDP-43 included expression-induced cell lines for DAP-dRRM1, DAP-dRRM2, DAP-dGR and DAP-d315, prepared in Example 5. The induction of expression was achieved by adding doxycycline to the culture media (+dox), total RNA was extracted from the cells 48 hours after addition, and abundance in cells was detected and determined using respective probes described above for each RNA. Culture media with no added doxycycline (-dox) was used as a control for expression induction, and syngeneic cell lines cultured under the same conditions for the other. In addition, each domain-deficient type of TDP-43 was induced by the same method described above in Example 10, and then the amount of ATP in cells was measured.

### (Results)

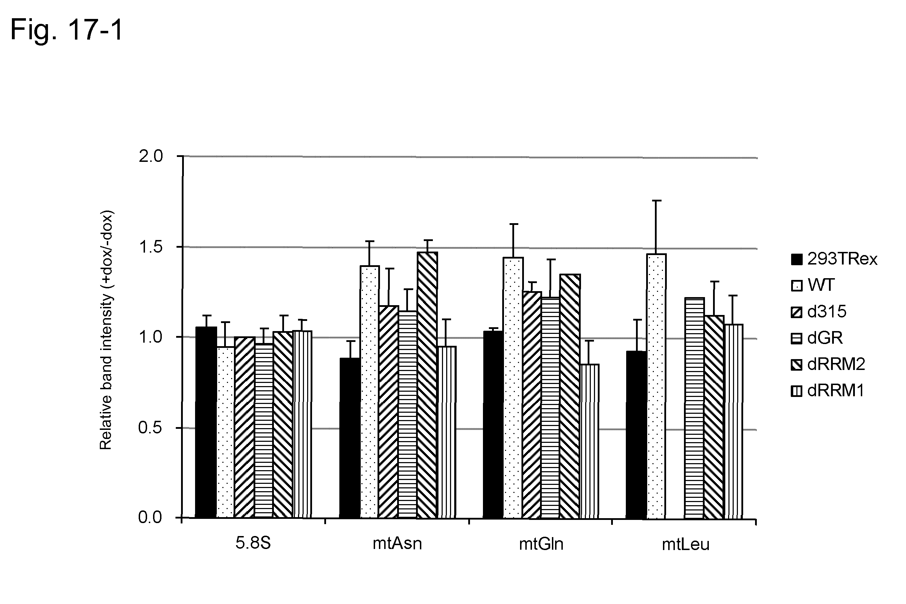

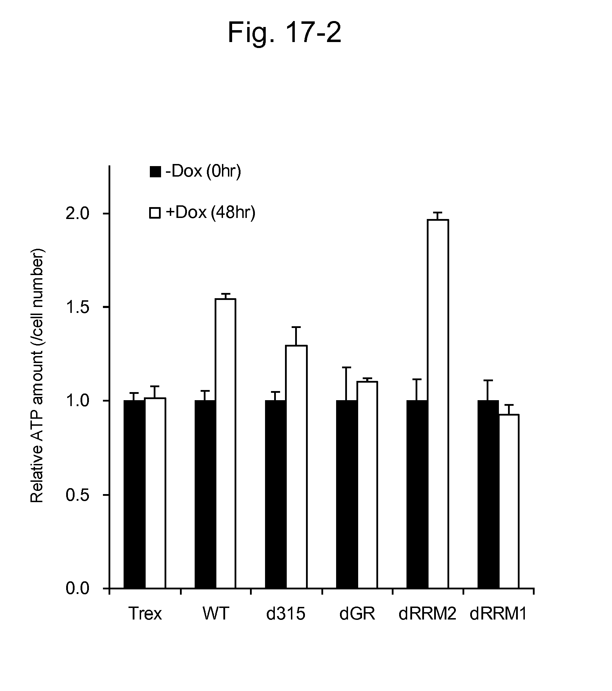

Results are shown in Figure 17-1 and Figure 17-2. Figure 17-1 presents the abundance of mt-tRNA^{Asn}, mt-tRNA^{Gln} and mt-tRNA^{Leu(UUR)} in cells in each domain-deficient type of TDP-43 as the relative intensity (a relative value when expression was induced (+dox) as compared to a value of 1 without doxycycline addition (-dox)) of the band detected. Figure 17-1 shows that the overexpression of DAP-dRRM 2 resulted in a significant increase in the amount of mt-tRNA^{Asn} and mt-tRNA^{Gln}.

Figure 17-2 shows a result of measurement of ATP in each domain-deficient type of TDP-43. As in the results in Figure 17-1, the overexpression of DAP-dRRM 2 resulted in a significant increase in the amount of ATP in cells. On the other hand, the overexpression of dRRM1 did not result in an increase in the amount of ATP in cells.

These results show that the domain-deficient type of TDP-43 increases cytotoxicity in proportion to the increase in the abundance of mt-tRNA^{Asn}, mt-tRNA^{Gln} and ATP in cells.

### <Example 12: Correlation between mutant TDP-43 and the abundance of ATP in cells>

### (Object)

Correlation between TDP-43 and the abundance of ATP in cells in ALS is examined.

### (Method)

Cell lines (DAP-D169G, DAP-G298S, DAP-R361S) involving the induction of expression of mutant TDP-43 (D169G, G298S, R361S; see Figure 10A) into which mutations identified in ALS had been introduced, which were prepared in Example 7, were induced with doxycycline and the abundance of ATP in cells was measured after 48 hours. The measurement method followed the method described in Example 2.

### (Results)

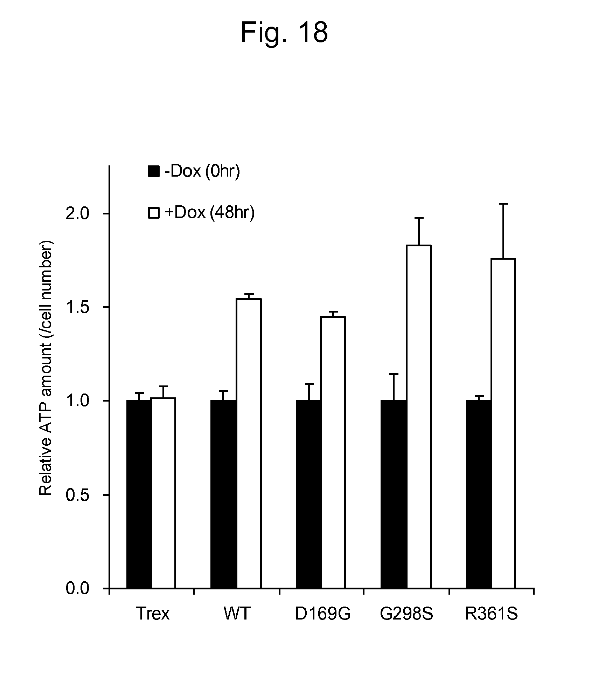

Results are shown in Figure 18. Any of the TDP-43 mutants increased the abundance of ATP in cells due to overexpression. This shows that the mutant TDP-43 has the ability to increase the abundance of mt-tRNA^{Asn} and mt-tRNA^{Gln} (Example 7) as well as ATP in cells in ALS, and this ability to increase is consistent with cytotoxicity.

The mechanism of pathogenesis of a disease condition due to TDP-43 mutations in ALS is still not well understood. However, a hypothesis as follows can be derived from the results in Examples 1 to 12 described above.

In other words, an assumption is that stabilization of TDP-43 in cells, or a change in its binding ability with mt-tRNA^{Asn}, mt-tRNA^{Gln}, mt-tRNA^{Glu} and/or mt-tRNA^{Pro}, Aralar 1 or Aralar 2, GDH or Musashi 2, due to mutations, may trigger ALS episodes. When TDP-43 is stabilized by mutation, a TDP-43-mt-tRNA complex combining mutant TDP-43 and mt-tRNA described above, and/or a TDP-43-protein complex combining the protein increases. Upon formation of the TDP-43-mt-tRNA complex combining mutant TDP-43 and the mt-tRNA, the abundance of mt-tRNA^{Asn} and mt-tRNA^{Gln} increases and protein synthesis in the mitochondria is promoted, resulting in activated mitochondria. At the same time, formation of the TDP-43-protein complex combining the protein will promote conversion from Glu to alpha-ketoglutaric acid, leading to acceleration in the tricarboxylic acid cycle. These effects are believed to result in an abnormal acceleration in mitochondrial oxidative phosphorylation, and an increase in ATP synthesis. We presume that the acceleration of this abnormal oxidative phosphorylation may cause abnormal urea metabolism due to increased active oxygen and increased ammonia associated with Glu metabolism, leading to cell injury and apoptosis. On the other hand, the cells exert a protective system to feed back this phenomenon. The cells trap the TDP-43-mt-tRNA complex and/or the TDP-43-protein complex which increased as a result of TDP-43 mutation within a particular location of the cytoplasm as an aggregate, and isolate the TDP-43 from the intracellular site where TDP-43 should exert its original function, but in this instance, the mt-tRNA^{Asn} and mt-tRNA^{Gln} included in the TDP-43-mt-tRNA complex, and the TDP-43-protein complex are expected as well to be simultaneously deposited as an aggregate. A change in the ability of TDP-43 to bind to Aralar 1 or Aralar 2, GDH or Musashi 2 due to mutation is also believed to result in the same phenomenon as when TDP-43 is stabilized. This results in the mt-tRNA^{Asn} and mt-tRNA^{Gln} being trapped within an aggregate without charge with Asp and Glu, and also, at the same time, the trapping of Musashi 2 which binds to Aralar 1, Aralar 2, GDH and/or mt-tRNA^{Asn}, mt-tRNA^{Gln} which are involved in the conversion from Glu to alpha-ketoglutaric acid. As a result, this eliminates a charge to mt-tRNA^{Asn} and mt-tRNA^{Gln} and the conversion to alpha-ketoglutaric acid, resulting in accumulation of Asp and/or Glu in cells. Because Asp is converted into Glu in astrocytes, in particular, more excessive accumulation of Glu occurs (Pardo et al., 2011, J. Cereb. Blood Flow Metab. 31 (1): 90-101, Hertz L., 2011, J. Cereb. Blood Flow Metab. 31 (1): 384-387). Excessively accumulated Glu toxin may be expected to induce cell death of motor neurons which are particularly sensitive. One hypothesis is that such mechanism of pathogenesis may explain how mutations of TDP-43 observed in ALS, and a disease condition of ALS, particularly an elevation in Glu concentration occur. Thus, it is believed that substances that have effects on all proteins and/or RNAs involved in this mechanism of pathogenesis and reduce intracellular concentration of Asp and/or Glu can represent an active ingredient of a therapeutic agent for ALS.

### <Example 13: Production of TDP-43 binding inhibitors>

TDP-43 binding inhibitors which inhibit binding of TDP-43 to its binding partner mt-tRNA (mt-tRNA^{Asn}, mt-tRNA^{Gln}, mt-tRNA^{Glu} and mt-tRNA^{Pro}) in a competitive manner are produced using the TDP-43 binding inhibitor production method of the present invention.

### (1) Search for candidate TDP-43 binding regions for four types of mt-tRNAs

### (Object)

Supposing TDP-43 binds to the consensus region of the four types of mt-tRNAs, a region in the four types of mt-tRNAs that binds to TDP-43 was searched for.

### (Method)

First, each mt-tRNA sequence underwent multiple alignment by lustalW, a consensus sequence and a motif were extracted by WebLogo and MEME, respectively, and the consensus sequence was analyzed by overlaying it on a three-dimensional structure.

### (Results)

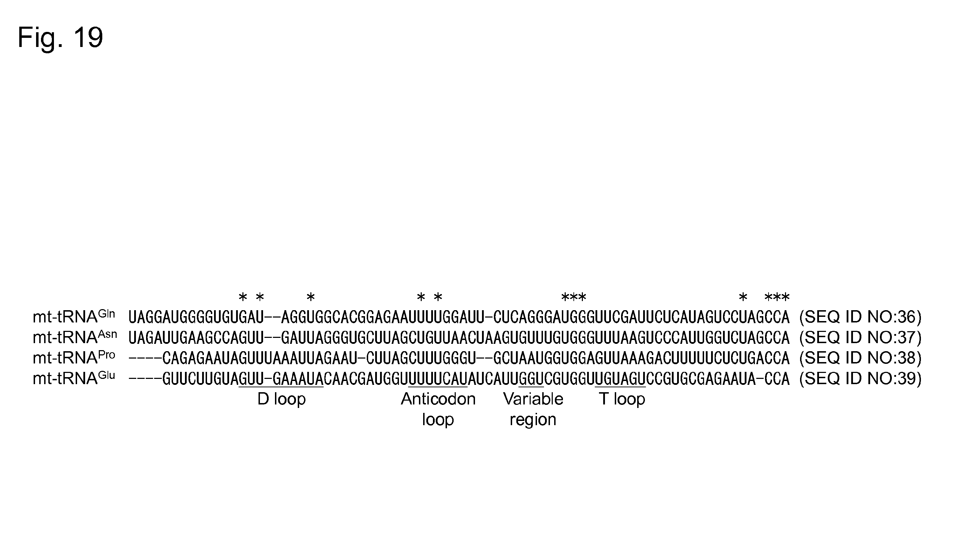

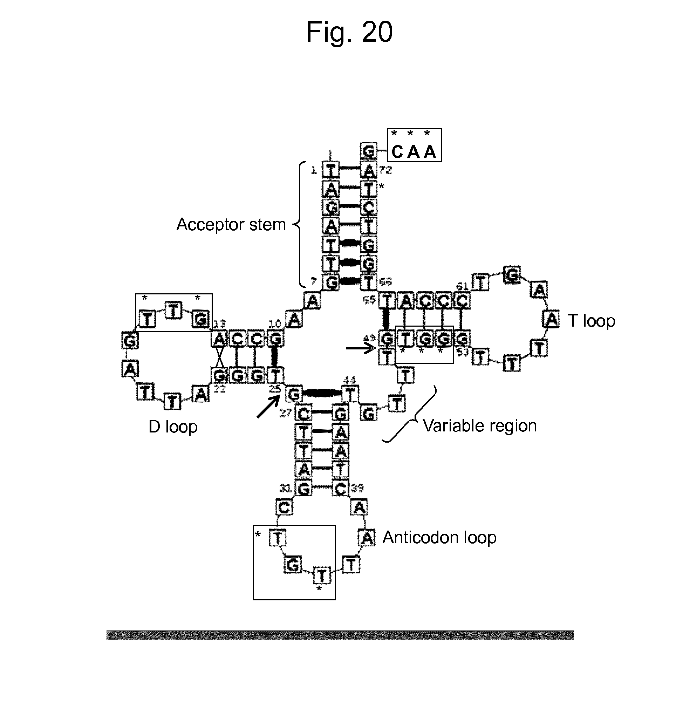

Figure 19 shows results from the alignment of the nucleotide sequences of the four types of mt-tRNAs, and Figure 20 shows the secondary structure of mt-tRNA^{Asn} and the location for the consensus sequence in the four types of mt-tRNAs.

Results from the alignment and the secondary structure revealed that consensus sequences existed in four regions including the GUU (GAU for mt-tRNA^{Gln} only) in the D stem & loop, UXU in the anticodon loop, UGG in the T stem & loop and CCA in the acceptor arm (aminoacyl arm). Of these, the consensus sequence UXU in the anticodon loop is commonly found in many other mt-tRNAs, and the consensus sequence CCA of the acceptor arm is commonly found in all mt-tRNAs, and they are thus not specific for the four types of mt-tRNAs. Thus, these two consensus sequences were excluded from candidate TDP-43 binding regions. On the other hand, the mt-tRNA having the consensus sequences GUU and UGG in the D stem & loop and T stem & loop, respectively, included only the four types of mt-tRNA^{Asn}, mt-tRNA^{Gln}, mt-tRNA^{Glu} and mt-tRNA^{Pro} among 22 types of mt-tRNAs, and they were found specific for the four types of mt-tRNAs. Thus, these two consensus sequences were further examined as candidate TDP-43 binding regions.

### (2) Inhibition of binding of mt-tRNA to TDP-43 by TDP-43 binding inhibitor candidate oligonucleotides

### (Object)

Results from (1) above revealed the four consensus regions in the four types of mt-tRNAs that bind to TDP-43. Oligonucleotides containing these consensus sequences are expected to be regions that are involved in binding to TDP-43, and thus may represent candidate TDP-43 binding inhibitors that inhibit binding between the four types of mt-tRNAs and TDP-43. Thus, a competitive assay of endogenous mt-tRNA for TDP-43 was performed using these oligonucleotides.

### (Method)

Based on the base at position 1 on the 5' end of mt-tRNA^{Asn}, a DNA oligonucleotide corresponding to positions 1 to 25 (D-loop; 5'-TAGATTGAAGCCAGTTGATTAGGGT-3': SEQ ID NO: 40) including the D stem & loop, positions 26 to 48 (Anticodon; 5'-GCTTAGCTGTTAACTAAGTGTTT-3': SEQ ID NO: 41) including the anticodon loop, a DNA oligonucleotide at positions 49 to 73 (T-loop; 5'-GTGGGTTTAAGTCCCATTGGTCTAG-3': SEQ ID NO: 42) including the T stem & loop, a DNA oligonucleotide (TG)₁₂ (5'-TGTGTGTGTGTGTGTGTGTGTGTG-3': SEQ ID NO: 43) corresponding to the 12-time repetitive sequence of UG which is a part of the consensus sequence UUG included in the T stem & loop, a control DNA oligonucleotide (TC)₁₂ for (TG)₁₂ (5'-TCTCTCTCTCTCTCTCTCTCTCTC-3': SEQ ID NO: 44), a DNA oligonucleotide (TGG)₈ (5'-TGGTGGTGGTGGTGGTGGTGGTGG-3': SEQ ID NO: 45) consisting of a 8-time repetitive sequence of the consensus sequence UGG included in the T stem & loop and a DNA oligonucleotide TT(GTT)₇G (5'-TTGTTGTTGTTGTTGTTGTTGTTG-3': SEQ ID NO: 46) consisting of a 7-time repetitive sequence of the consensus sequence UGG included in the D stem & loop (in the figure, shown with (TTG)₈ for convenience reasons) were synthesized.

The competitive assay was performed according to the method of Example 1. After first preparing a cell extract according to the method described in "(3) Preparation of a cell extract" of Example 1, each DNA oligonucleotide of a candidate TDP-43 binding inhibitor was added to make 200 nM. A sample without added oligonucleotide served as a negative control (-). Then, the mixture was treated according to the methods described in "(4) Immunoprecipitation and RNA collection" and "(5) Urea denaturation PAGE" of Example 1, and nucleic acids bound to DAP-TDP-43 containing the Flag tag were developed by electrophoresis. In addition, mt-tRNA^{Asn} bound to DAP-TD-43 was detected and determined according to the Northern blotting of Example 2. The oligonucleotide probe used for detection was the oligonucleotide set forth in SEQ ID NO: 10. The signal intensity of the RNA band of mt-tRNA^{Asn} was determined using the LAS4000 image analyzer and MultiGauge software (Fujifilm).

### (Results)

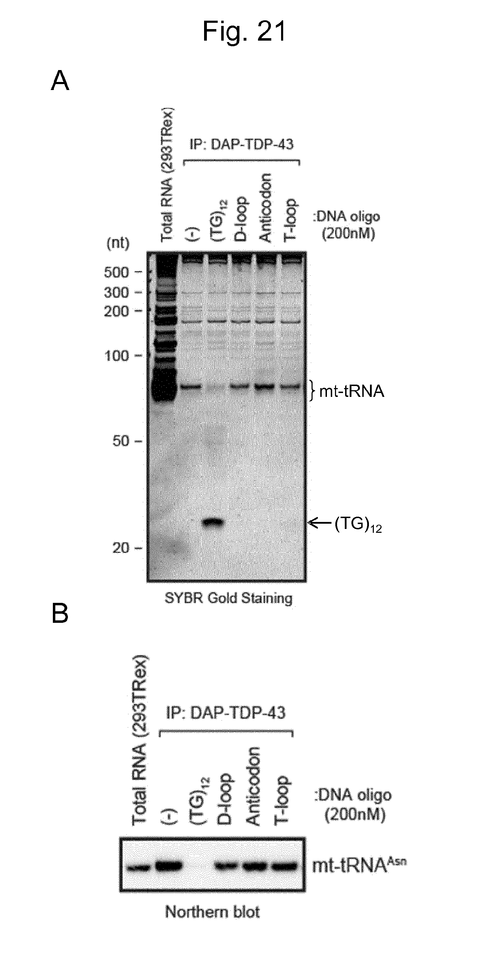

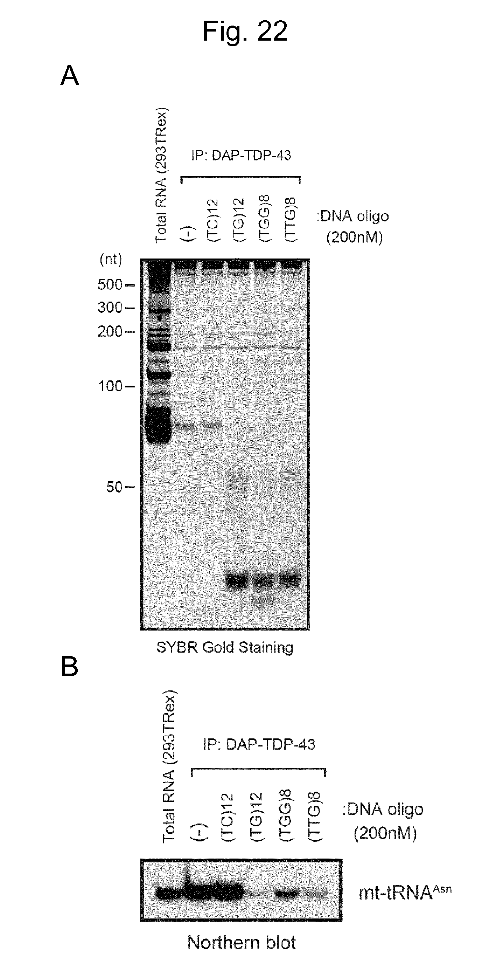

Results are shown in Figures 21 and 22.

Results in Figure 21A have shown that bands from the four types of mt-tRNAs bound to DAP-TD-43 when (TG)₁₂ was added (see Figure 1) were the thinnest, indicating binding to DAP-TD-43 was inhibited. When D-loop and T-loop were added, some inhibition was observed in comparison with the negative control (-), but Anticodon had little inhibitory effect. Results in Figure 21B confirmed similar results in the detection of mt-tRNA^{Asn} by Northern blotting.

Results in Figure 22A have shown that bands from the four types of mt-tRNAs bound to DAP-TD-43 when (TG)₁₂, (TTG)₈ and TT(GTT)₇G were added (see Figure 1) were apparently thinner than the negative control (-), indicating binding to DAP-TD-43 was inhibited. On the other hand, binding between DAP-TD-43 and mt-tRNA was not inhibited when (TC)₁₂, which did not contain a consensus sequence, was added as a control for (TG)₁₂. Similar results were also confirmed in the detection of mt-tRNA^{Asn} by the Northern blotting presented in Figure 22B. (TG)₁₂ had the strongest inhibitory effect on binding, followed by (TTG)₈ and TT(GTT)₇G. Thus, (TG)₁₂, (TTG)₈ and TT(TT)₇G containing the consensus sequence or a part thereof have been demonstrated that they likely represent TDP-43 binding inhibitors that inhibit binding between TDP-43 and the four types of mt-tRNAs in a competitive manner.

### (3) Examination of the inhibitory effect of TDP-43 binding inhibitor (TG)₁₂ on binding

### (Object)

Concentration of the TDP-43 binding inhibitor obtained in (2) above and its inhibitory effect on binding between TDP-43 and the four types of mt-tRNAs were examined.

### (Method)

(TG)₁₂ obtained in (2) above was used as the TDP-43 binding inhibitor. The basic method followed (3) above. However, the concentration of added (TG)₁₂ was 0, 2, 20 and 200 nM.

### (Results)

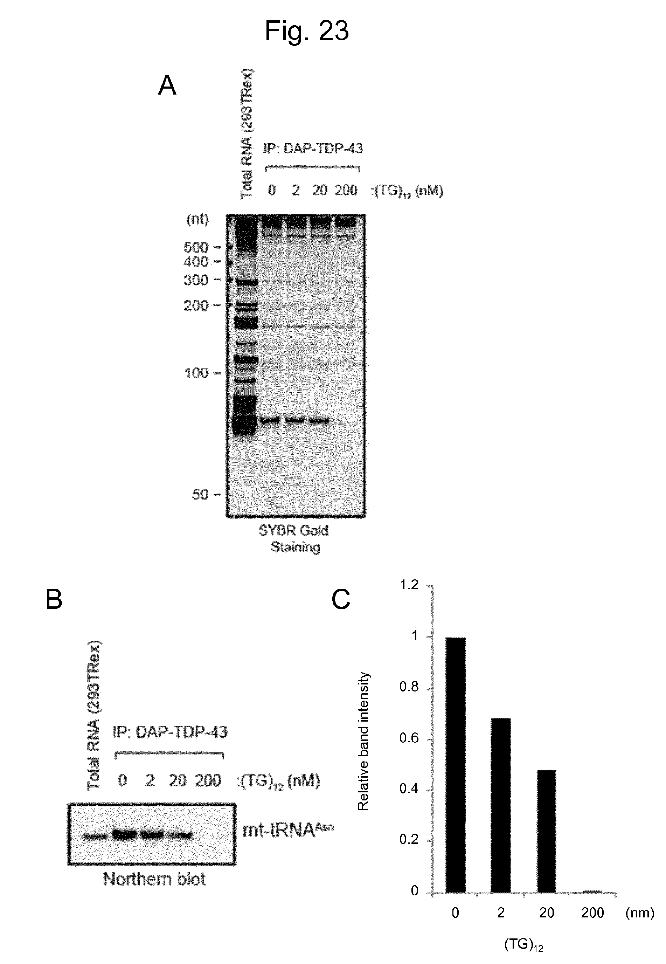

Results are shown in Figure 23A to C. The TDP-43 binding inhibitor (TG)₁₂ was found to increase in its inhibitory effect on binding between TDP-43 and the four types of mt-tRNAs in a concentration-dependent manner.

All publications, patents and patent applications cited herein shall be incorporated herein as is by reference.

### <Example 14: Relationship between TDP-43 and the amount of active oxygen>

### (Object)

Relationship between TDP-43 and the amount of ATP was demonstrated in Example 10. On the other hand, active oxygen is produced at the same time as ATP increases. Active oxygen is believed to be a direct causative agent of cell injury because it is known as a cytotoxin. Thus, the correlation between TDP-43 and the amount of active oxygen was also examined.

### (Method)

DAP-TDP-43 cells prepared in Example 1 were used. The induction of DAP-TDP-43 expression was performed by adding doxycycline to the culture media (+dox), and the intracellular activity of reactive oxygen was measured on cells at 8, 24, and 48 hours after addition. Culture media with no added doxycycline (-dox) was used as a control for expression induction, and syngeneic cell lines (293TRex) cultured under the same conditions for the other.

Cell permeable fluorescent probe 2',7'-Dichlorodihydrofluorescin diacetate (DCFH-DA) is used for the measurement of activity of intracellular hydroxyl, peroxyl or other reactive oxygen species. DCFH-DA is dispersed in cells, deacetylated by intracellular esterase and converted into non-fluorescent 2',7'-Dichlorodihydrofluorescin (DCFH). It is then immediately oxidized by ROS and converted into high fluorescent 2',7'-Dichlorodihydrofluorescein (DCF). Fluorescence intensity is proportional to cytosolic ROS levels. Specifically, the cells were treated with 25 µM of DCFH-DA, incubated at 37°C for 30 minutes and collected. Fluorescence intensity of DCF in the cells collected was detected by flow cytometry. (Miura D et al. (2004) Clinical & Experimental Metastasis 21: 445-451).

### (Results)

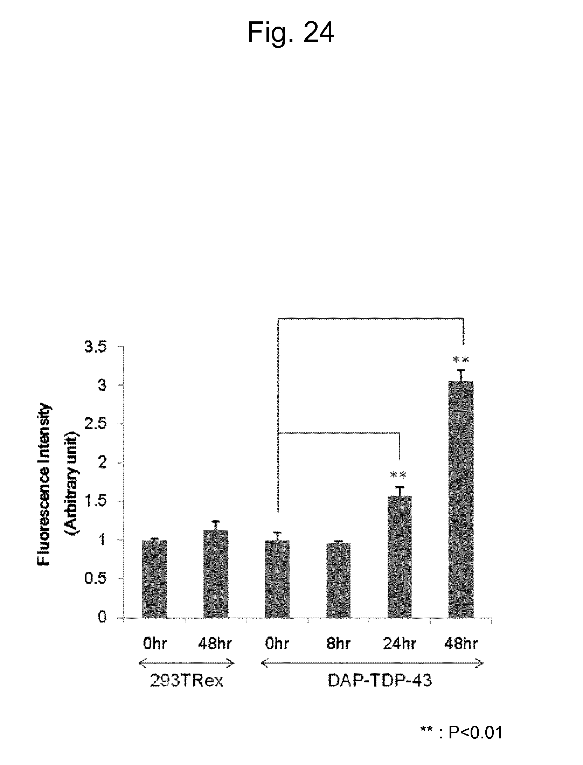

Results are shown in Figure 24. In the cells in which expression of TDP-43 was induced, the intracellular activity of reactive oxygen was found to significantly increase with the progress of induction time, i.e. the increase in the expression level of TDP-43. On the other hand, in the cells (293TRex) without induction of TDP-43 expression, intracellular activity of reactive oxygen exhibited no significant change. These results indicate that the amount of active oxygen in cells increases in proportion to the increase in the expression level of TDP-43, which in turn shows that cytotoxicity increases with the increase in the expression level of TDP-43.

## Claims

1. A method for identifying a disease associated with an abundance of TAR DNA-binding protein-43 in a cell, comprising:
a measurement step of measuring at least one of
(a) an abundance of at least one mt-tRNA selected from the group consisting of mt-tRNA^{Asn}, mt-tRNA^{Gln}, mt-tRNA^{Glu}, and mt-tRNA^{Pro},
(b) an abundance of glutamic acid and/or aspartic acid,
(c) an abundance of ATP or active oxygen,
(d) an amount of binding between at least one polypeptide selected from the group consisting of Aralar 1, Aralar 2, GDH, and Musashi 2 and TAR DNA-binding protein-43, and
(e) an amount of binding between at least one mt-tRNA selected from the group consisting of mt-tRNA^{Asn}, mt-tRNA^{Gln}, mt-tRNA^{Glu}, and mt-tRNA^{Pro} and TAR DNA-binding protein-43
in a cell derived from a subject; and
an identification step comprising comparing a measurement value obtained by the measurement step with a corresponding measurement value in a cell derived from a healthy individual, and when there is a statistically significant difference between these values, identifying the subject as suffering from the disease.

2. The identification method according to claim 1, wherein the disease is a disease in which an abundance of TAR DNA-binding protein-43 in a cell derived from a subject is statistically significantly increased compared to an abundance of TAR DNA-binding protein-43 in a cell derived from a healthy individual.

3. The identification method according to claim 1 or 2, wherein, when an initiation methionine in an amino acid sequence set forth in SEQ ID NO: 1 is designated as position 1, the TAR DNA-binding protein-43 has a mutation in which alanine at position 90 is substituted by valine (A90V), aspartic acid at position 169 is substituted by glycine (D169G), asparagine at position 267 is substituted by serine (N267S), glycine at position 287 is substituted by serine (G287S), glycine at position 290 is substituted by alanine (G290A), serine at position 292 is substituted by asparagine (S292N), glycine at position 294 is substituted by alanine (G294A), glycine at position 294 is substituted by valine (G294V), glycine at position 295 is substituted by arginine (G295R), glycine at position 295 is substituted by serine (G295S), glycine at position 298 is substituted by serine (G298S), methionine at position 311 is substituted by valine (M311V), alanine at position 315 is substituted by threonine (A315T), alanine at position 315 is substituted by glutamic acid (A315E), glutamine at position 331 is substituted by lysine (Q331K), serine at position 332 is substituted by asparagine (S332N), glycine at position 335 is substituted by aspartic acid (G335D), methionine at position 337 is substituted by valine (M337V), glutamine at position 343 is substituted by arginine (Q343R), asparagine at position 345 is substituted by lysine (N345K), glycine at position 348 is substituted by cysteine (G348C), asparagine at position 352 is substituted by serine (N352S), asparagine at position 352 is substituted by threonine (N352T), glycine at position 357 is substituted by serine (G357S), arginine at position 361 is substituted by serine (R361S), proline at position 363 is substituted by alanine (P363A), asparagine at position 378 is substituted by aspartic acid (N378D), serine at position 379 is substituted by cysteine (S379C), serine at position 379 is substituted by proline (S379P), alanine at position 382 is substituted by proline (A382P), alanine at position 382 is substituted by threonine (A382T), isoleucine at position 383 is substituted by valine (I383V), glycine at position 384 is substituted by arginine (G384R), asparagine at position 390 is substituted by aspartic acid (N390D), asparagine at position 390 is substituted by serine (N390S), or serine at position 393 is substituted by leucine (S393L), or
a mutation in which tyrosine at position 374 and subsequent amino acids are deleted.

4. The identification method according to claim 3, wherein the mutation is D169G, G298S, or R361S.

5. The identification method according to any one of claims 1 to 4, wherein the disease is a nervous system disease.

6. The identification method according to claim 5, wherein the disease is selected from the group consisting of amyotrophic lateral sclerosis, Alzheimer's disease, Parkinson's disease, and frontotemporal lobar degeneration.

7. A method for producing a drug which reduces an abundance of aspartic acid, glutamic acid, ATP, or active oxygen in a cell, comprising:
an introduction step of introducing a drug candidate substance into a cell,
a measurement step of measuring an abundance of aspartic acid, glutamic acid, ATP, or active oxygen in a cell comprising the drug candidate substance and in a cell not comprising the drug candidate substance, and
a selection step, comprising comparing measurement values in two cells obtained by the measurement step and, when a measurement value in the cell comprising the drug candidate substance is statistically significantly lower, selecting the drug candidate substance as a drug of interest.

8. A method for producing a TAR DNA-binding protein-43 binding inhibitor, comprising:
a mixing step of mixing
(a) at least one mt-tRNA selected from the group consisting of mt-tRNA^{Asn}, mt-tRNA^{Gln}, mt-tRNA^{Glu}, and mt-tRNA^{Pro}, and/or
(b) at least one protein selected from the group consisting of Aralar1, Aralar2, GDH, and Musashi 2, and
TAR DNA-binding protein-43 with a drug candidate substance,
a detection step of detecting an amount of binding between the mt-tRNA and/or the protein and the TAR DNA-binding protein-43, and
a selection step of selecting, when the binding is not detected or a comparison between the amount of binding detected and an amount of binding between a corresponding mt-tRNA and/or protein and TAR DNA-binding protein-43 in an absence of the drug candidate substance shows a statistically significant difference, the drug candidate substance as a TAR DNA-binding protein-43 binding inhibitor.

9. The production method according to claim 8, wherein the drug candidate substance is a substance competing for binding to TAR DNA-binding protein-43 with the mt-tRNA specified by (a) or the protein specified by (b).

10. The production method according to claim 8, wherein the drug candidate substance is a substance which reduces an abundance of the mt-tRNA specified by (a) or the protein specified by (b) in an active state in a cell.

11. The production method according to claim 9 or 10, wherein the drug candidate substance is a nucleic acid molecule, a peptide, or a low molecular weight compound.

12. The production method according to claim 11, wherein the drug candidate substance is:
(a) a polypeptide of 100 amino acids or less, comprising a polypeptide consisting of an amino acid sequence set forth in SEQ ID NO: 2, 4, or 5,
(b) a polypeptide of 130 amino acids or less, comprising a polypeptide resulting from addition, deletion, or substitution of 1 to 3 amino acids in an amino acid sequence set forth in SEQ ID NO: 2, 4, or 5,
(c) a polypeptide consisting of an amino acid sequence having a 90% or more identity with an amino acid sequence set forth in SEQ ID NO: 2, 4, or 5, or
(d) a moiety of a polypeptide consisting of an amino acid sequence set forth in SEQ ID NO: 2, 4, or 5.

13. The production method according to claim 11, wherein the drug candidate substance is a peptide of 9 to 20 amino acids comprising:
(a) a moiety of a peptide consisting of an amino acid sequence set forth in SEQ ID NO: 2,
(b) a moiety of a peptide comprising addition, deletion, or substitution of 1 to 3 amino acids in an amino acid sequence set forth in SEQ ID NO: 2, or
(c) a moiety of a peptide consisting of an amino acid sequence having a 90% or more identity with an amino acid sequence set forth in SEQ ID NO: 2,
wherein the peptide of 9 to 20 amino acids binds to a nucleic acid molecule of 5 to 50 nucleotides comprising a nucleotide sequence consisting of TGG or UGG and/or GTT or GUU.

14. The production method according to any one of claims 7 to 11, wherein, when an initiation methionine in an amino acid sequence set forth in SEQ ID NO: 1 is designated as position 1, the TAR DNA-binding protein-43 comprises a mutation of A90V, D169G, N267S, G287S, G290A, S292N, G294A, G294V, G295R, G295S, G298S, M311V, A315T, A315E, Q331K, S332N, G335D, M337V, Q343R, N345K, G348C, N352S, N352T, G357S, R361S, P363A, N378D, S379C, S379P, A382P, A382T, I383V, G384R, N390D, N390S, or S393L, or
a mutation in which tyrosine at position 374 and subsequent amino acids are deleted.

15. The production method according to claim 14, wherein the mutation is D169G, G298S, or R361S.

16. The production method according to any one of claims 7 to 15, wherein the drug which reduces an abundance of amino acid, ATP, or active oxygen in a cell according to claim 7 or the TAR DNA-binding protein-43 binding inhibitor according to any one of claims 8 to 15 is an active ingredient of a therapeutic agent for a disease associated with an abundance of TAR DNA-binding protein-43 in a cell.

17. The production method according to claim 16, wherein the disease associated with an abundance of TAR DNA-binding protein-43 in a cell is a disease in which an abundance of TAR DNA-binding protein-43 in a cell derived from a subject is statistically significantly increased compared to an abundance of TAR DNA-binding protein-43 in a cell derived from a healthy individual.

18. The production method according to claim 16 or 17, wherein the disease is a nervous system disease.

19. The production method according to claim 18, wherein the disease is selected from the group consisting of amyotrophic lateral sclerosis, Alzheimer's disease, Parkinson's disease, and frontotemporal lobar degeneration.

20. A TAR DNA-binding protein-43 binding inhibitor comprising a nucleic acid molecule of 5 to 50 nucleotides, wherein the nucleic acid molecule comprises two or more of a nucleotide sequence consisting of TG or UG and/or GT or GU, and inhibits binding between mt-tRNA^{Asn}, mt-tRNA^{Gln}, mt-tRNA^{Glu}, or mt-tRNA^{Pro} and TAR DNA-binding protein-43.

21. The TAR DNA-binding protein-43 binding inhibitor according to claim 20, comprising a nucleic acid molecule of 6 to 50 nucleotides, wherein the nucleic acid molecule comprises two or more of a nucleotide sequence consisting of TGG or UGG and/or GTT or GUU.

22. The TAR DNA-binding protein-43 binding inhibitor according to claim 20 or 21, comprising a sequence comprising a successive repetition of the nucleotide sequence.

23. The TAR DNA-binding protein-43 binding inhibitor according to claim 22, wherein the number of the repetition is 3 to 20.

24. The TAR DNA-binding protein-43 binding inhibitor according to claim 23, comprising a nucleotide sequence set forth in SEQ ID NO: 43, 45, or 46.


SEQUENCE LISTING

<110>  National University Corporation Tokyo University of Agriculture
       and Technology
       Tokyo Metropolitan University

<120>  Method for identifying the disease associated with the amount of
       TDP-43 protein present in a cell

<130>  PH-5519-PCT

<150>  JP 2012-042256
<151>  2012-02-28

<160>  46

<170>  PatentIn version 3.5

<210>  1
<211>  414
<212>  PRT
<213>  Homo sapiens

<220>
<223>  TDP-43

<400>  1

<210>  2
<211>  65
<212>  PRT
<213>  Homo sapiens

<220>
<223>  RRM1 domain of TDP-43

<400>  2

Asp Leu Ile Val Leu Gly Leu Pro Trp Lys Thr Thr Glu Gln Asp Leu
1               5                   10                  15

Lys Glu Tyr Phe Ser Thr Phe Gly Glu Val Leu Met Val Gln Val Lys
            20                  25                  30

Lys Asp Leu Lys Thr Gly His Ser Lys Gly Phe Gly Phe Val Arg Phe
        35                  40                  45

Thr Glu Tyr Glu Thr Gln Val Lys Val Met Ser Gln Arg His Met Ile
    50                  55                  60

Asp
65

<210>  3
<211>  65
<212>  PRT
<213>  Homo sapiens

<220>
<223>  RRM2 domain of TDP-43

<400>  3

Val Phe Val Gly Arg Cys Thr Glu Asp Met Thr Glu Asp Glu Leu Arg
\202P                5                   10                  15

Glu Phe Phe Ser Gln Tyr Gly Asp Val Met Asp Val Phe Ile Pro Lys
            20                   25                  30

Pro Phe Arg Ala Phe Ala Phe Val Thr Phe Ala Asp Asp Gln Ile Ala
        35                   40                  45

Gln Ser Leu Cys Gly Glu Asp Leu Ile Ile Lys Gly Ile Ser Val His
     50                  55                   60

Ile

65

<210>  4
<211>  41
<212>  PRT
<213>  Homo sapiens

<220>
<223>  GR domain of TDP-43

<400>  4

Gly Arg Phe Gly Gly Asn Pro Gly Gly Phe Gly Asn Gln Gly Gly Phe
1               5                   10                  15

Gly Asn Ser Arg Gly Gly Gly Ala Gly Leu Gly Asn Asn Gln Gly Ser
            20                  25                  30

Asn Met Gly Gly Gly Met Asn Phe Gly
        35                  40

<210>  5
<211>  100
<212>  PRT
<213>  Homo sapiens

<220>
<223>  315 domain of TDP-43

<400>  5

Ala Phe Ser Ile Asn Pro Ala Met Met Ala Ala Ala Gln Ala Ala Leu
1               5                   10                  15

Gln Ser Ser Trp Gly Met Met Gly Met Leu Ala Ser Gln Gln Asn Gln
            20                  25                  30

Ser Gly Pro Ser Gly Asn Asn Gln Asn Gln Gly Asn Met Gln Arg Glu
        35                  40                  45

Pro Asn Gln Ala Phe Gly Ser Gly Asn Asn Ser Tyr Ser Gly Ser Asn
    50                  55                  60

Ser Gly Ala Ala Ile Gly Trp Gly Ser Ala Ser Asn Ala Gly Ser Gly
65                  70                  75                  80

Ser Gly Phe Asn Gly Gly Phe Gly Ser Ser Met Asp Ser Lys Ser Ser
                85                  90                  95

Gly Trp Gly Met
            100

<210>  6
<211>  72
<212>  DNA

<213>  Artificial

<220>
<223>  primer

<400>  6
tatataggta ccgccaccat ggactacaag gacgacgacg acaagggatc catgtctgaa    60

tatattcggg ta                                                         72


<210>  7
<211>  33
<212>  DNA
<213>  Artificial

<220>
<223>  primer

<400>  7
tatatactcg agctacattc cccagccaga aga                                  33


<210>  8
<211>  35
<212>  DNA
<213>  Artificial

<220>
<223>  primer

<400>  8
tatataagct tgccaccatg caccaccacc accac                                 35


<210>  9
<211>  31
<212>  DNA
<213>  Artificial

<220>
<223>  primer

<400>  9
tataggtacc cgccagggtc atcagggtgt c                                     31


<210>  10
<211>  28
<212>  DNA
<213>  Artificial

<220>
<223>  probe for mt-tRNA Asn

<400>  10
cagctaagca ccctaatcaa ctggcttc                                         28


<210>  11
<211>  30
<212>  DNA
<213>  Artificial

<220>
<223>  probe for mt-tRNA Gln

<400>  11
tatgagaatc gaacccatcc ctgagaatcc                                        30


<210>  12
<211>  30
<212>  DNA
<213>  Artificial

<220>
<223>  probe for mt-tRNA Pro

<400>  12
gtctttaact ccaccattag cacccaaagc                                        30


<210>  13
<211>  30
<212>  DNA
<213>  Artificial

<220>
<223>  probe for mt-tRNA Glu

<400>  13
gcacggacta caaccacgac caatgatatg                                        30


<210>  14
<211>  30
<212>  DNA
<213>  Artificial

<220>
<223>  probe for mt-tRNA Leu(UUR)

<400>  14
tatgcgatta ccgggctctg ccatcttaac                                        30


<210>  15
<211>  27
<212>  DNA
<213>  Artificial

<220>
<223>  probe for mt-tRNA Met

<400>  15
tagcagagga tggtttcgat ccatcga                                           27


<210>  16
<211>  25
<212>  RNA
<213>  Artificial

<220>
<223>  siRNA for TDP-43

<400>  16
uuaagaucuu ucuugaccug cacca                                              25


<210>  17
<211>  25
<212>  RNA
<213>  Artificial

<220>
<223>  siRNA for TDP-43

<400>  17
uggugcaggu caagaaagau cuuaa                                              25


<210>  18
<211>  25
<212>  RNA
<213>  Artificial

<220>
<223>  negative control siRNA

<400>  18
uuaacaagcu uucuuuagcc gucca                                              25


<210>  19
<211>  25
<212>  RNA
<213>  Artificial

<220>
<223>  negative control siRNA

<400>  19
uggacggcua aagaaagcuu guuaa                                              25


<210>  20
<211>  24
<212>  DNA
<213>  Artificial

<220>
<223>  probe for U1 snRNA

<400>  20
gtatctcccc tgccaggtaa gtat                                               24


<210>  21
<211>  24
<212>  DNA
<213>  Artificial

<220>
<223>  probe for 5.8S rRNA

<400>  21
agacaggcgt agccccggga ggaa                                               24

<210>  22
<211>  30
<212>  DNA
<213>  Artificial

<220>
<223>  primer for dRRM1

<400>  22
cccatcgatg gacgatggtg tgactgcaaa                                        30


<210>  23
<211>  30
<212>  DNA
<213>  Artificial

<220>
<223>  primer for dRRM1

<400>  23
cccatcgatg gatgttttct ggactgctct                                        30


<210>  24
<211>  30
<212>  DNA
<213>  Artificial

<220>
<223>  primer for dRRM2

<400>  24
cccatcgatt ccaatgccga acctaagcac                                        30


<210>  25
<211>  30
<212>  DNA
<213>  Artificial

<220>
<223>  primer for dRRM2

<400>  25
cccatcgatt tttctgcttc tcaaaggctc                                        30


<210>  26
<211>  30
<212>  DNA
<213>  Artificial

<220>
<223>  primer for dGR

<400>  26
tatatcgatg cgttcagcat taatccagcc                                        30


<210>  27

<211>  30
<212>  DNA
<213>  Artificial

<220>
<223>  primer for dGR

<400>  27
cccatcgata cttctttcta actgtctatt                                        30


<210>  28
<211>  33
<212>  DNA
<213>  Artificial

<220>
<223>  primer for d315

<400>  28
tatataggat ccatgtctga atatattcgg gta                                    33


<210>  29
<211>  33
<212>  DNA
<213>  Artificial

<220>
<223>  primer for d315

<400>  29
atactcgagc taaccaaagt tcatcccacc acc                                    33


<210>  30
<211>  30
<212>  DNA
<213>  Artificial

<220>
<223>  primer for D169G

<400>  30
cagcgacata tgataggtgg acgatggtgt                                        30


<210>  31
<211>  30
<212>  DNA
<213>  Artificial

<220>
<223>  primer for D169G

<400>  31
acaccatcgt ccacctatca tatgtcgctg                                        30


<210>  32
<211>  30
<212>  DNA
<213>  Artificial

<220>
<223>  primer for G298S

<400>  32
agagggggtg gagctagttt gggaaacaat                                        30


<210>  33
<211>  30
<212>  DNA
<213>  Artificial

<220>
<223>  primer for G298S

<400>  33
   attgtttccc aaactagctc caccccctct                                        30


<210>  34
<211>  30
<212>  DNA
<213>  Artificial

<220>
<223>  primer for R361S

<400>  34
aaggcaacat gcagagtgag ccaaaccagg                                       30


<210>  35
<211>  30
<212>  DNA
<213>  Artificial

<220>
<223>  primer for R361S

<400>  35
cctggtttgg ctcactctgc atgttgcctt                                       30


<210>  36
<211>  75
<212>  RNA
<213>  Homo sapiens

<400>  36
uaggaugggg ugugauaggu ggcacggaga auuuuggauu cucagggaug gguucgauuc      60

ucauaguccu agcca                                                       75


<210>  37
<211>  76
<212>  RNA
<213>  Homo sapiens

<400>  37
uagauugaag ccaguugauu agggugcuua gcuguuaacu aaguguuugu ggguuuaagu      60

cccauugguc uagcca                                                    76

<210>  38
<211>  71
<212>  RNA
<213>  Homo sapiens

<400>  38
cagagaauag uuuaaauuag aaucuuagcu uugggugcua augguggagu uaaagacuuu      60

uucucugacc a                                                          71

<210>  39
<211>  72
<212>  RNA
<213>  Homo sapiens

<400>  39
guucuuguag uugaaauaca acgaugguuu uucauaucau uggucguggu uguaguccgu      60

gcgagaauac ca                                                         72

<210>  40
<211>  25
<212>  DNA
<213>  Artificial

<220>
<223>  synthetic

<220>
<223>  D-loop

<400>  40
tagattgaag ccagttgatt agggt                                           25

<210>  41
<211>  23
<212>  DNA
<213>  Artificial

<220>
<223>  synthetic

<220>
<223>  Anticodon

<400>  41
gcttagctgt taactaagtg ttt                                             23

<210>  42
<211>  25
<212>  DNA
<213>  Artificial

<220>
<223>  synthetic

<220>
<223>  T-loop

<400>  42
gtgggtttaa gtcccattgg tctag                                              25


<210>  43
<211>  24
<212>  DNA
<213>  Artificial

<220>
<223>  synthetic

<220>
<223>  (TG)12

<400>  43
tgtgtgtgtg tgtgtgtgtg tgtg                                               24


<210>  44
<211>  24
<212>  DNA
<213>  Artificial

<220>
<223>  synthetic

<220>
<223>  (TC)12

<400>  44
tctctctctc tctctctctc tctc                                               24


<210>  45
<211>  24
<212>  DNA
<213>  Artificial

<220>
<223>  synthetic

<220>
<223>  (TGG)8

<400>  45
tggtggtggt ggtggtggtg gtgg                                               24


<210>  46
<211>  24
<212>  DNA
<213>  Artificial

<220>
<223>  synthetic

<220>
<223>  (TTG)8

<400> 46
ttgttgttgt tgttgttgtt gttg 24

SEQUENCE LISTING

<110>  National University Corporation Tokyo University of Agriculture
       and Technology
       Tokyo Metropolitan University

<120> Method for identifying disease associated with abundance of TDP-43
      in cell

<130>  PH-5519-PCT

<150>  JP 2012-042256
<151>  2012-02-28

<160>  46

<170>  PatentIn version 3.5

<210>  1
<211>  414
<212>  PRT
<213>  Homo sapiens

<220>
<223>  TDP-43

<400>  1

<210>  2
<211>  65
<212>  PRT
<213>  Homo sapiens

<220>
<223>  RRM1 domain of TDP-43

<400>  2

Asp Leu Ile Val Leu Gly Leu Pro Trp Lys Thr Thr Glu Gln Asp Leu
1               5                   10                  15

Lys Glu Tyr Phe Ser Thr Phe Gly Glu Val Leu Met Val Gln Val Lys
            20                  25                  30

Lys Asp Leu Lys Thr Gly His Ser Lys Gly Phe Gly Phe Val Arg Phe
        35                  40                  45

Thr Glu Tyr Glu Thr Gln Val Lys Val Met Ser Gln Arg His Met Ile
    50                  55                  60

Asp
65

<210>  3
<211>  65
<212>  PRT
<213>  Homo sapiens

<220>
<223>  RRM2 domain of TDP-43

<400>  3

Val Phe Val Gly Arg Cys Thr Glu Asp Met Thr Glu Asp Glu Leu Arg
\202P                5                   10                  15

Glu Phe Phe Ser Gln Tyr Gly Asp Val Met Asp Val Phe Ile Pro Lys
            20                   25                  30

Pro Phe Arg Ala Phe Ala Phe Val Thr Phe Ala Asp Asp Gln Ile Ala
        35                   40                  45

Gln Ser Leu Cys Gly Glu Asp Leu Ile Ile Lys Gly Ile Ser Val His
     50                  55                   60

Ile

65

<210>  4
<211>  41
<212>  PRT
<213>  Homo sapiens

<220>
<223>  GR domain of TDP-43

<400>  4

Gly Arg Phe Gly Gly Asn Pro Gly Gly Phe Gly Asn Gln Gly Gly Phe
1               5                   10                  15

Gly Asn Ser Arg Gly Gly Gly Ala Gly Leu Gly Asn Asn Gln Gly Ser
            20                  25                  30

Asn Met Gly Gly Gly Met Asn Phe Gly
        35                  40

<210>  5
<211>  100
<212>  PRT
<213>  Homo sapiens

<220>
<223>  315 domain of TDP-43

<400>  5

Ala Phe Ser Ile Asn Pro Ala Met Met Ala Ala Ala Gln Ala Ala Leu
1               5                   10                  15

Gln Ser Ser Trp Gly Met Met Gly Met Leu Ala Ser Gln Gln Asn Gln
            20                  25                  30

Ser Gly Pro Ser Gly Asn Asn Gln Asn Gln Gly Asn Met Gln Arg Glu
        35                  40                  45

Pro Asn Gln Ala Phe Gly Ser Gly Asn Asn Ser Tyr Ser Gly Ser Asn
    50                  55                  60

Ser Gly Ala Ala Ile Gly Trp Gly Ser Ala Ser Asn Ala Gly Ser Gly
65                  70                  75                  80

Ser Gly Phe Asn Gly Gly Phe Gly Ser Ser Met Asp Ser Lys Ser Ser
                85                  90                  95

Gly Trp Gly Met
            100

<210>  6
<211>  72
<212>  DNA

<213>  Artificial

<220>
<223>  primer

<400>  6
tatataggta ccgccaccat ggactacaag gacgacgacg acaagggatc catgtctgaa    60

tatattcggg ta                                                         72


<210>  7
<211>  33
<212>  DNA
<213>  Artificial

<220>
<223>  primer

<400>  7
tatatactcg agctacattc cccagccaga aga                                  33


<210>  8
<211>  35
<212>  DNA
<213>  Artificial

<220>
<223>  primer

<400>  8
tatataagct tgccaccatg caccaccacc accac                                 35


<210>  9
<211>  31
<212>  DNA
<213>  Artificial

<220>
<223>  primer

<400>  9
tataggtacc cgccagggtc atcagggtgt c                                     31


<210>  10
<211>  28
<212>  DNA
<213>  Artificial

<220>
<223>  probe for mt-tRNA Asn

<400>  10
cagctaagca ccctaatcaa ctggcttc                                         28


<210>  11
<211>  30
<212>  DNA
<213>  Artificial

<220>
<223>  probe for mt-tRNA Gln

<400>  11
tatgagaatc gaacccatcc ctgagaatcc                                        30


<210>  12
<211>  30
<212>  DNA
<213>  Artificial

<220>
<223>  probe for mt-tRNA Pro

<400>  12
gtctttaact ccaccattag cacccaaagc                                        30


<210>  13
<211>  30
<212>  DNA
<213>  Artificial

<220>
<223>  probe for mt-tRNA Glu

<400>  13
gcacggacta caaccacgac caatgatatg                                        30


<210>  14
<211>  30
<212>  DNA
<213>  Artificial

<220>
<223>  probe for mt-tRNA Leu(UUR)

<400>  14
tatgcgatta ccgggctctg ccatcttaac                                        30


<210>  15
<211>  27
<212>  DNA
<213>  Artificial

<220>
<223>  probe for mt-tRNA Met

<400>  15
tagcagagga tggtttcgat ccatcga                                           27


<210>  16
<211>  25
<212>  RNA
<213>  Artificial

<220>
<223>  siRNA for TDP-43

<400>  16
uuaagaucuu ucuugaccug cacca                                              25


<210>  17
<211>  25
<212>  RNA
<213>  Artificial

<220>
<223>  siRNA for TDP-43

<400>  17
uggugcaggu caagaaagau cuuaa                                              25


<210>  18
<211>  25
<212>  RNA
<213>  Artificial

<220>
<223>  negative control siRNA

<400>  18
uuaacaagcu uucuuuagcc gucca                                              25


<210>  19
<211>  25
<212>  RNA
<213>  Artificial

<220>
<223>  negative control siRNA

<400>  19
uggacggcua aagaaagcuu guuaa                                              25


<210>  20
<211>  24
<212>  DNA
<213>  Artificial

<220>
<223>  probe for U1 snRNA

<400>  20
gtatctcccc tgccaggtaa gtat                                               24


<210>  21
<211>  24
<212>  DNA
<213>  Artificial

<220>
<223>  probe for 5.8S rRNA

<400>  21
agacaggcgt agccccggga ggaa                                               24

<210>  22
<211>  30
<212>  DNA
<213>  Artificial

<220>
<223>  primer for dRRM1

<400>  22
cccatcgatg gacgatggtg tgactgcaaa                                        30


<210>  23
<211>  30
<212>  DNA
<213>  Artificial

<220>
<223>  primer for dRRM1

<400>  23
cccatcgatg gatgttttct ggactgctct                                        30


<210>  24
<211>  30
<212>  DNA
<213>  Artificial

<220>
<223>  primer for dRRM2

<400>  24
cccatcgatt ccaatgccga acctaagcac                                        30


<210>  25
<211>  30
<212>  DNA
<213>  Artificial

<220>
<223>  primer for dRRM2

<400>  25
cccatcgatt tttctgcttc tcaaaggctc                                        30


<210>  26
<211>  30
<212>  DNA
<213>  Artificial

<220>
<223>  primer for dGR

<400>  26
tatatcgatg cgttcagcat taatccagcc                                        30


<210>  27

<211>  30
<212>  DNA
<213>  Artificial

<220>
<223>  primer for dGR

<400>  27
cccatcgata cttctttcta actgtctatt                                        30


<210>  28
<211>  33
<212>  DNA
<213>  Artificial

<220>
<223>  primer for d315

<400>  28
tatataggat ccatgtctga atatattcgg gta                                    33


<210>  29
<211>  33
<212>  DNA
<213>  Artificial

<220>
<223>  primer for d315

<400>  29
atactcgagc taaccaaagt tcatcccacc acc                                    33


<210>  30
<211>  30
<212>  DNA
<213>  Artificial

<220>
<223>  primer for D169G

<400>  30
cagcgacata tgataggtgg acgatggtgt                                        30


<210>  31
<211>  30
<212>  DNA
<213>  Artificial

<220>
<223>  primer for D169G

<400>  31
acaccatcgt ccacctatca tatgtcgctg                                        30


<210>  32
<211>  30
<212>  DNA
<213>  Artificial

<220>
<223>  primer for G298S

<400>  32
agagggggtg gagctagttt gggaaacaat                                        30


<210>  33
<211>  30
<212>  DNA
<213>  Artificial

<220>
<223>  primer for G298S

<400>  33
   attgtttccc aaactagctc caccccctct                                        30


<210>  34
<211>  30
<212>  DNA
<213>  Artificial

<220>
<223>  primer for R361S

<400>  34
aaggcaacat gcagagtgag ccaaaccagg                                       30


<210>  35
<211>  30
<212>  DNA
<213>  Artificial

<220>
<223>  primer for R361S

<400>  35
cctggtttgg ctcactctgc atgttgcctt                                       30


<210>  36
<211>  75
<212>  RNA
<213>  Homo sapiens

<400>  36
uaggaugggg ugugauaggu ggcacggaga auuuuggauu cucagggaug gguucgauuc      60

ucauaguccu agcca                                                       75


<210>  37
<211>  76
<212>  RNA
<213>  Homo sapiens

<400>  37
uagauugaag ccaguugauu agggugcuua gcuguuaacu aaguguuugu ggguuuaagu      60

cccauugguc uagcca                                                    76

<210>  38
<211>  71
<212>  RNA
<213>  Homo sapiens

<400>  38
cagagaauag uuuaaauuag aaucuuagcu uugggugcua augguggagu uaaagacuuu      60

uucucugacc a                                                          71

<210>  39
<211>  72
<212>  RNA
<213>  Homo sapiens

<400>  39
guucuuguag uugaaauaca acgaugguuu uucauaucau uggucguggu uguaguccgu      60

gcgagaauac ca                                                         72

<210>  40
<211>  25
<212>  DNA
<213>  Artificial

<220>
<223>  synthetic

<220>
<223>  D-loop

<400>  40
tagattgaag ccagttgatt agggt                                           25

<210>  41
<211>  23
<212>  DNA
<213>  Artificial

<220>
<223>  synthetic

<220>
<223>  Anticodon

<400>  41
gcttagctgt taactaagtg ttt                                             23

<210>  42
<211>  25
<212>  DNA
<213>  Artificial

<220>
<223>  synthetic

<220>
<223>  T-loop

<400>  42
gtgggtttaa gtcccattgg tctag                                              25


<210>  43
<211>  24
<212>  DNA
<213>  Artificial

<220>
<223>  synthetic

<220>
<223>  (TG)12

<400>  43
tgtgtgtgtg tgtgtgtgtg tgtg                                               24


<210>  44
<211>  24
<212>  DNA
<213>  Artificial

<220>
<223>  synthetic

<220>
<223>  (TC)12

<400>  44
tctctctctc tctctctctc tctc                                               24


<210>  45
<211>  24
<212>  DNA
<213>  Artificial

<220>
<223>  synthetic

<220>
<223>  (TGG)8

<400>  45
tggtggtggt ggtggtggtg gtgg                                               24


<210>  46
<211>  24
<212>  DNA
<213>  Artificial

<220>
<223>  synthetic

<220>
<223>  (TTG)8

<400> 46
ttgttgttgt tgttgttgtt gttg 24